(19) Europäisches Patentamt
European Patent Office
Office européen des brevets

(11) EP 1 889 622 A1

(12) EUROPEAN PATENT APPLICATION
published in accordance with Art. 153(4) EPC

(43) Date of publication:
**20.02.2008 Bulletin 2008/08**

(21) Application number: **06756762.8**

(22) Date of filing: **30.05.2006**

(51) Int Cl.:
*A61K 31/499* (2006.01) *A61K 31/527* (2006.01)
*A61P 1/04* (2006.01) *A61P 1/16* (2006.01)
*A61P 3/10* (2006.01) *A61P 9/10* (2006.01)
*A61P 11/00* (2006.01) *A61P 11/06* (2006.01)
*A61P 13/12* (2006.01) *A61P 17/00* (2006.01)
*A61P 17/06* (2006.01) *A61P 19/02* (2006.01)
*A61P 25/00* (2006.01) *A61P 27/02* (2006.01)
*A61P 27/16* (2006.01) *A61P 29/00* (2006.01)
*A61P 31/18* (2006.01) *A61P 35/00* (2006.01)
*A61P 37/06* (2006.01) *A61P 37/08* (2006.01)
*A61P 43/00* (2006.01)

(86) International application number:
**PCT/JP2006/310814**

(87) International publication number:
**WO 2006/129679 (07.12.2006 Gazette 2006/49)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.05.2005 JP 2005160566**

(71) Applicant: **ONO PHARMACEUTICAL CO., LTD.**
**Osaka-shi, Osaka 541-8526 (JP)**

(72) Inventors:
- **HABASHITA, Hiromu,**
  **c/o ONO PHARMACEUTICAL CO., LTD**
  **Mishima-gu,**
  **Osaka 618-8585 (JP)**
- **SHIBAYAMA, Shiro,**
  **c/o ONO PHARMACEUTICAL CO., LTD**
  **Tsukuba-shi,**
  **Ibaraki 300-4247 (JP)**

(74) Representative: **Henkel, Feiler & Hänzel**
**Patentanwälte**
**Maximiliansplatz 21**
**80333 München (DE)**

# (54) SPIROPIPERIDINE COMPOUND AND MEDICINAL USE THEREOF

(57)

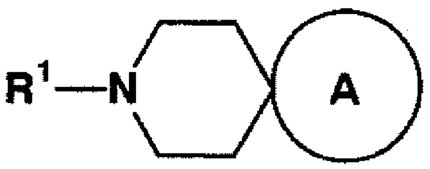

(I)

The present invention relates to a CXCR3 antagonist comprising a compound represented by formula (I) (wherein all the symbols have the same meaning as defined in the description),
a salt thereof, a quaternary ammonium salt thereof, an N-oxide form thereof or a solvate thereof, or a prodrug thereof.

This antagonist is useful as a preventive, therapeutic and/or progression-suppressing agent for a CXCR3-mediated disease such as an immune or an allergic disease [e.g., an atopic disease, an autoimmune disease (e.g., multiple sclerosis, rheumatoid arthritis, systemic lupus erythematosus, type I diabetes, glomerulonephritis, Sjogren's syndrome and the like), systemic inflammatory response syndrome (SIRS), a rejection response to transplanted organ, tissue and/or cell or the like], a gastrointestinal disease [e.g., an inflammatory bowel disease or the like], or a respiratory disease [e.g., asthma, a chronic obstructive pulmonary disease or the like].

EP 1 889 622 A1

**Description**

TECHNICAL FIELD

A present invention relates to a spiropiperidine compound which is useful as medicament and a CXCR3 antagonist containing the same as an active ingredient.

BACKGROUND OF THE INVENTION

Chemokine is known as an endogeneous basic protein having leukocyte chemotactic, activating abilities and strong heparin-binding abilities. At present, it is considered that the chemokine is related to not only a control of an infiltration of specific leukocytes at the time of inflammations and immune responses but also a development, a homing of lymphocyte under physiological conditions, a migration of hemocyte precursor cells and somatic cells.

A Differentiation, a proliferation and a cell death of hemocytes are controlled by various types of cytokine. In living bodies, inflammations are happened locally, and such as differentiation, maturation of lymphocytes are carried out at certain specified sites. That is, various necessary cells migrate into certain specified sites and accumulate therein to cause a series of inflammations and immune responses. Accordingly, a migration of cells is also an indispensable phenomenon for immune system in addition to the differentiation, the proliferation and the death of cells.

Chemokine binds with a receptor of a specific cell-surface belonging to G protein coupled seven transmembrane receptors. These are named chemokine receptors. Chemokine binds with the chemokine receptor, and intracellular information transmission system is activated through its binding G protein. As a result, it is provided that a variation in a cell shape, a transient rise of a concentration of intracellular disengagement calcium ion, a granular leukocyte emiocytosis, a manifestation rise of integrin, a production of bioactive fat (e.g., leukotriene), and a mechanism alteration of cell, organizations or organs such as a disorder of respiratory system with relation to a leukotriene activation. The chemokine receptor (e.g, CCR 1, CCR 2, CCR 2A, CCR 2B, CCR 3, CCR 4, CCR 5, CCR 6, CCR 7, CCR 8, CCR 9, CXCR 1, CXCR 2, CXCR 3, CXCR 4, CXCR 5, $CX_3CR$ 1 and XCR 1) is connected as an important mediator of an inflammatory disease and a disease due to a disorder of immunoregulation system (e.g, an asthma and an allergic disease, an autoimmune disease (e.g, an rheumatoid arthritis and an atherosclerosis), and a rejection response to a transplanted organ).

A CXCR3 which is a kind of chemokine receptor is activated in three kinds of chemokines (IP-10, Mig and I-TAC) induced by IFN-γ. Of these, IP - 10 is expressed in a field of various kinds of inflammation that T cell interfuses in large quantities. In addition, the CXCR3 is expressed in T cell (particularly, Th1 cell), B cell, NK cell and the like. T cells participate in an autoimmune disease such as a multiple sclerosis, a rheumatoid arthritis, an atherosclerosis or a type I diabetes. In addition, an interfusion of T cell is generated in an immunologic inflammatory disease such as a psoriasis. In addition, T cell participates in an allergic disease such as a bronchial asthma and a rejection response to a transplanted organ. In other words, a CXCR3 antagonist is effective for the diseases because it is able to inhibit a migration of T cell through CXCR3 and an accumulation of T cell. In addition, it is reported that CXCR3 is expressed in a neoplasm, in particular a malignant of B cell system. Therefore the CXCR3 antagonist is effective for a carcinomatous immunotherapy, a metastasis control in particular.

Based on the above, the CXCR3 antagonist is useful as a preventive, therapeutic and/or progression-suppressing agent for a disease such as an immune or an allergic disease [e.g, an atopic disease, anaphylaxis or anaphylactoid reaction, an autoimmune disease (e.g, multiple sclerosis, rheumatoid arthritis, systemic lupus erythematosus, type I diabetes, glomerulonephritis, Sjogren's syndrome and the like), systemic inflammatory response syndrome (SIRS), a rejection response to a transplanted organ, tissue and/or cell, allergia angiitis, rhinitis, arthritis, an inflammatory oculopathy (e.g, a conjunctivitis) and the like], a gastrointestinal disease [e.g, an inflammatory bowel disease (e.g, colitis ulcerosa, Crohn's disease, eosinophil stomach and intestines symptoms and the like), hepatitis, nephritis, nephropathy, pancreatitis and the like], a respiratory disease [e.g, asthma, a chronic obstructive pulmonary disease, a respiratory insufficiency, lungs irritation, acute respiratory distress syndrome (ARDS), an allergic bronchopulmonary aspergillosis], a brain / neurologic disease [e.g, a cerebrovascular disease (e.g, an arterial sclerosis, a thrombosis, ischemia / a reperfusion disorder, re-constriction, infarct and the like) and the like], a dermatosis [e.g, a dermatitis (e.g, an atopic dermatitis, a psoriasis, contact dermatitis, eczema, urticaria, itch and the like) and the like], a metabolism / an endocrine disease (e.g, a diabetes and the like), a carcinoma disease [e.g, a malignant neoplasm (e.g, a leucaemia, a solid cancer and a cancer metastasis and the like) or the like], an infection or a disease with infection [e.g, a viral disease (e.g, an acquired immunodeficiency syndrome (AIDS), SARS and the like), an affiliation symptom (a dementia) by AIDS and the like].

Some compounds are reported as a low molecular weight compound having a CXCR3 antagonism by the present, but there is not report that spiropiperidine derivatives having spiro bond provide the CXCR3 antagonism (see patent documents1-6).

On the other hand, it is described that a compound represented by formula (Z):

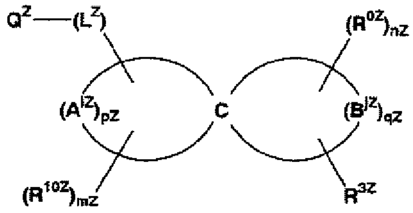

(Z)

[wherein, $A^{iZ}$ and $B^{jZ}$ are each independently selected from carbon, nitrogen, oxygen or sulfur (provided that at least one atom of $A^{iZ}$ is carbon, and at least one atom of $B^{jZ}$ is carbon); Spiro bicyclic ring formed by $A^{iZ}$ and $B^{jZ}$ may be unsaturated by each case partially; pZ and qZ are each independently a number from 2 to 6; mZ is a number from 0 to pZ; $R^{10Z}$ is same or different, and is each independently an incoherent substituent which is selected from hydrogen atom, alkyl group, alkyl group which is substituted for halogen, alkenyl group, alkynyl group, cycloalkyl group, =O, =S or the like; nZ is a number from 0 to qZ; $R^{0Z}$ is same or different and is each independently an incoherent substituent which is selected from hydrogen atom, alkyl group, alkyl group which is substituted for halogen, alkenyl group, alkynyl group, cycloalkyl group, =O, =S or the like; - $(L^Z)$ - is a bond, or a bivalent substituted or non- substituted chain comprising 1 to 10 atom(s) selected from carbon atom, nitrogen atom, sulfur atom and oxygen atom; $Q^Z$ is a basic group including one or more a basicity radical(s); and $R^{3Z}$ is a acidic group including one or more acid radical(s) (only necessary site extracts)], and a pharmaceutically acceptable salt thereof, a solvate thereof or a prodrug thereof is useful for a platelet aggregation depression (for example, see patent document 7).

In addition, it is described that a compound represented by formula (Y):

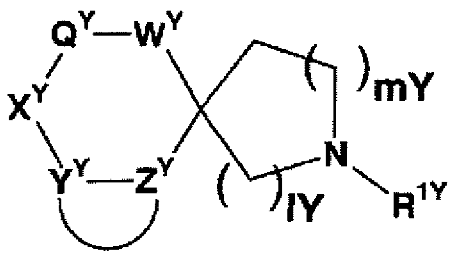

(Y)

[wherein, 1Y and mY represent each independently 0, 1, 2, 3, 4 or 5; $R^{1Y}$ is selected from hydrogen atom, C1-8 alkyl group, C2-8 alkenyl group, C2-8 alkynyl group and the like; $W^Y$ is selected from a bond, C1-3 alkyl group, C1-3 alkyl group substituted by oxo group and the like, and the like; $Q^Y$ is selected from $-NR^2-$, -O-, -S-, -S(O)- or $-SO_2-$; $X^Y$ is selected from a bond, C1-3 alkyl group, C1-3 alkyl group substituted by oxo group and the like, and the like; ring $Y^Y$-$Z^Y$ represents phenyl, naphthyl, heteroaryl (only necessary site extracts)], and a pharmaceutically acceptable salt thereof are useful for a chemokine receptor modulator (for example, see patent document 9).

Furthermore, it is described that triazaspiro[5.5]undecane derivative compounds represented by formula (X):

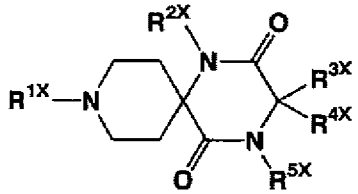

(X)

[wherein, $R^{1X}$ represents formula (X-2):

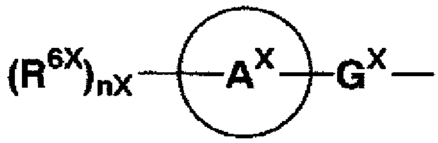 (X-2)

or formula (X-3):

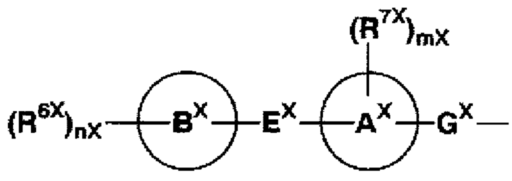 (X-3)

$R^{2X}$ represents alkyl group, alkynyl group and the like; $R^{3X}$ and $R^{4X}$ represent hydrogen atom, alkyl group, alkyl group which have substituents and the like, or $R^{3X}$ form formula (X-4)

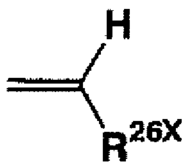 (X-4)

together with $R^{4X}$;
$R^{5X}$ represents hydrogen atom or alkyl group (only necessary site extracts)] are useful as a preventive and/or therapeutic agent for an asthma, an atopic dermatitis, an urticaria, an allergic bronchopulmonary aspergillosis, an allergic eosinophil gastric enteropathy, a nephritis, a nephropathy, a hepatitis, an arithritis, a rheumatoid an arthritis, a psoriasis, a rhinitis, a conjunctivitis, an ischemia/a depression of a reperfusion damage, a multiple sclerosis, a colitis ulcerosa, an acute respiratory distress syndrome, a shock with bacterial infection, a diabetes, a therapy of an autoimmune disease, a rejection response of a transplant, an immunologic inhibition, a prevention of cancer metastasis, an acquired immunodeficiency syndrome since they control an interaction of chemokine/chemokine receptor, however neither a concrete description nor suggestion is done about an action as opposed to CXCR3 of the compound at all (for example, see patent document 9).

In addition, it is described that a spiropiperidine compound represented by formula (W):

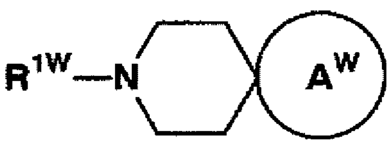 (W)

[wherein, $R^{1W}$ represents hydrogen atom, aliphatic hydrocarbon group which may have substituents or cyclic group which may have substituents, a ring $A^W$ represents 5- to 8-membered cyclic group which may have substituents (provided that 2,5- diketopiperazine ring which spiro-binded at the 3-position is excluded), the ring $A^W$ may further condense with a ring $B^W$, the ring $B^W$ represents 3- to 8-membered mono-carbocyclic or heterocyclic ring which may have a substituent(s) (only necessary site extracts)],
a salt thereof, an N-oxide form thereof, a quaternary ammonium salt thereof, a solvate thereof, or a prodrug thereof, have a chemokine receptor antagonism, and is useful for a prevention and/or treatment of a various inflammation, an autoimmune disease, an immune disorders such as an allergosis or HIV infection, however neither a concrete description nor suggestion is done about an action as opposed to CXCR3 of the compound at all (see patent document 10).

Furthermore, 9-benzyl-1-methyl-1,3,9-triazaspiro[5.5]undecan-2-one and 9-benzyl-1,3-dimethyl-1,3,9-triazaspiro[5.5]undecan-2-one are known as a manufacturing intermediate of adrenaline $\alpha_{2C}$ antagonist, however neither a

concrete description nor suggestion is done about an action as opposed to CXCR3 of these compounds at all (see patent document 11).

Patent Document 1 : WO02/85862
Patent Document 2 : WO02/83143
Patent Document 3 : WO01/16114
Patent Document 4 : WO03/70242
Patent Document 5 : WO2004/94381
Patent Document 6 : WO2005/3127
Patent Document 7 : WO97/11940
Patent Document 8 : WO98/25605
Patent Document 9 : WO02/74770
Patent Document 10 : WO2004/92169
Patent Document 11 : WO2003/28

DISCLOSURE OF THE INVENTION

PROBLEMS TO BE SOLVED BY THE INVENTION

A preventive, therapeutic and/or progression-suppressing agent of an immunology an allergic disease such as a systemic lupus erythematosus, a rheumatoid arthritis, a multiple sclerosis, a type I diabetes, a psoriasis, an atopic dermatitis, a respiratory disease such as an asthma, a chronic obstructive pulmonary disease and a gastrointestinal disease such as an inflammatory bowel disease, a carcinoma disease and a rejection response of a graft in an organic transplantation and the like is useful as a medicament. A development of a safe CXCR3 antagonist is desired earnestly.

MEANS FOR SOLVING THE PROBLEMS

The present inventors, as a result of having studied zealously in order to find the compounds having a CXCR3 antagonism, found that the compounds represented by formula (I), especially the compounds represented by formula (II) have a superior CXCR3 antagonism, and accomplished the present invention.
The present invention relates to

[1] A CXCR3 antagonist comprising a compound represented by formula (I):

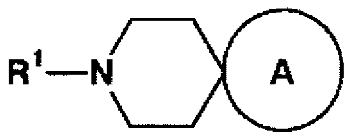
(I)

wherein ring A is 5- to 8-membered cyclic group which may be condensed with C3-8 mono-carbocyclic group which may have a substituent(s) or 3- to 8-membered mono-heterocyclic group which may have a substituent(s), which may have a further substituent(s);
$R^1$ is a hydrogen atom, aliphatic hydrocarbon which may have a substituent(s) or a cyclic group which may have a substituent(s)),
a salt thereof, a quaternary ammonium salt thereof, an N-oxide form thereof or a solvate thereof, or a prodrug thereof;
[2] The CXCR3 antagonist according to the above item [1], wherein the compound represented by formula (I):

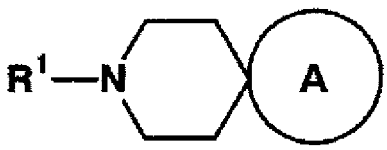
(I)

wherein all symbols have the same meanings as those described in the above item [1] is a compound represented by formula (II):

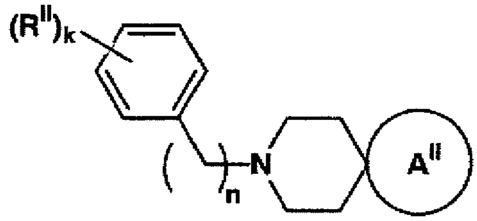

(II)

wherein ring $A^{II}$ is 6-membered mono-carbocyclic group which may have a substituent(s) or 6-membered mono-heterocyclic group which may have a substituent(s), and n is an integer of 1 to 4, and $R^{II}$ is substituent, and k is 0 or an integer of 1 to 5, and plural of $R^{II}$ may be same or different when k is 2 or more);

[3] The CXCR3 antagonist according to the above item [2], wherein n is 1;

[4] The CXCR3 antagonist according to the above item [2], wherein ring $A^{II}$ is cyclohexane ring which may have a substituent(s), piperazine ring which may have a substituent(s), tetrahydropyrimidine ring which may have a substituent(s), perhydropyrimidine ring which may have a substituent(s), tetrahydropyridine ring which may have a substituent(s) or piperidine ring which may have a substituent(s);

[5] The CXCR3 antagonist according to the above item [2], wherein $R^{II}$ is a chlorine atom, benzene ring which may have a substituent(s), methyl, methoxy, allyloxy, propargyloxy or cyano;

[6] The CXCR3 antagonist according to the above item [2], wherein the compound represented by formula (II):

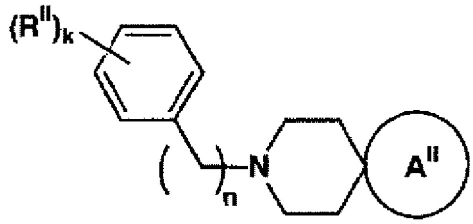

(II)

wherein all symbols have the same meanings as those described in the above item [2] is a compound represented by formula (II-A):

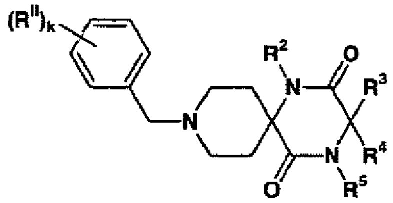

(II-A)

wherein, $R^2$, $R^3$, $R^4$, and $R^5$ are each independently a hydrogen atom, aliphatic hydrocarbon which may have a substituent(s), hydroxy which may be protected, carboxy which may be protected, carbamoyl which may have a substituent(s) or cyclic group which may have a substituent(s), and the other symbols have the same meanings as those described in the above item [2];

[7] The CXCR3 antagonist according to the above item [6],

wherein $R^{II}$ is a chlorine atom, benzene ring which may have a substituent(s), methyl, methoxy, allyloxy, propargyloxy, or cyano;

$R^2$ is propyl, cyclopropylmethyl, cyclobutyl or cyclopentyl;

$R^3$ is benzyl which may have a substituent(s), pyridylmethyl which may have a substituent(s) or indolylmethyl which may have a substituent(s);

$R^4$ is a hydrogen atom and

$R^5$ is a hydrogen atom;

[8] The CXCR3 antagonist according to the above item [2], wherein the compound represented by formula (II):

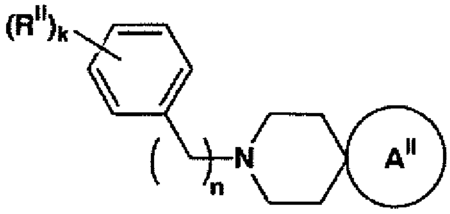

(II)

wherein all symbols have the same meanings as those described in the above item [2] is a compound represented by formula (II-B):

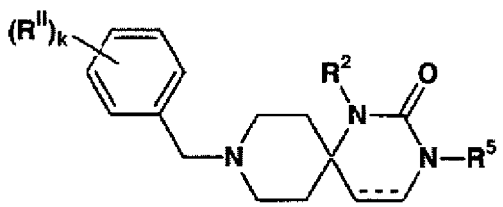

(II-B)

wherein

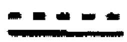

is a single bond or a double bond, the other symbols have the same meanings as those described in the abobe items [2] and [6];
[9] The CXCR3 antagonist according to the abobe item [8],
wherein $R^{II}$ is a chlorine atom, methyl, methoxy, allyloxy, propargyloxy or cyano;
$R^2$ is propyl, cyclopropylmethyl, cyclobutyl or cyclopentyl;
$R^5$ is benzyl which may have a substituent(s), phenylethyl which may have a substituent(s) or butyl;
[10] The CXCR3 antagonist according to the above item [2], wherein the compound represented by formula (II):

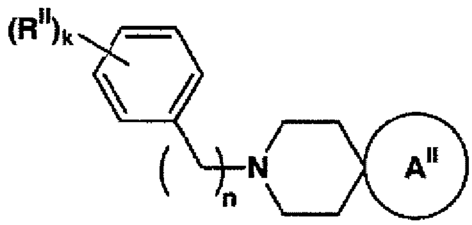

(II)

wherein all symbols have the same meanings as those described in the above item [2] is a compound represented by formula (II-C):

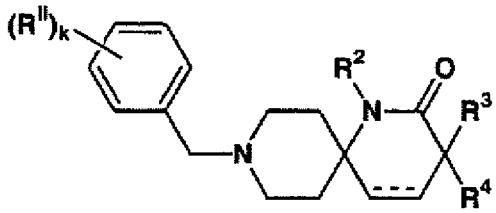

(II-C)

wherein all symbols have the same meanings as those described in the above items [2], [6] and [8], or a compound represented by formula (II-D):

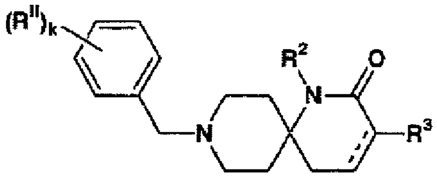

(II-D)

wherein all symbols have the same meanings as those described in the above items [2], [6] and [8];

[11] The CXCR3 antagonist according to the above item [10],

wherein $R^{II}$ is a chlorine atom, methyl, methoxy, allyloxy, propargyloxy or cyano;

$R^2$ is propyl, cyclopropylmethyl, cyclobutyl or cyclopentyl;

$R^3$ is benzyl which may have a substituent(s), pyridylmethyl which may have a substituent(s) or indolylmethyl which may have a substituent(s); and

$R^4$ is a hydrogen atom;

[12] The CXCR3 antagonist according to the above item [1], which is a preventive, therapeutic and/or progression-suppressing agent of CXCR3-related disease;

[13] The CXCR3 antagonist according to the above item [12], wherein the CXCR3-related disease is a rejection response to transplanted organ, tissue and/or cell, autoimmune disease, allergic disease, inflammatory bowel disease and/or respiratory disease;

[14] The CXCR3 antagonist according to the above item [13], wherein the autoimmune disease is systemic lupus erythematosus, rheumatoid arthritis, multiple sclerosis, type I diabetes and/or psoriasis, and the allergic disease is atopic dermatitis, and the respiratory disease is a chronic obstructive pulmonary disease;

[15] A compound represented by formula (II-B):

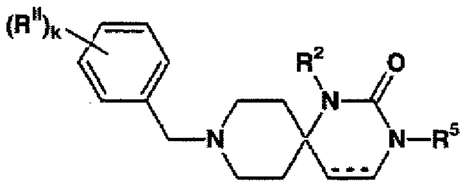

(II-B)

wherein all symbols have the same meanings as those described in the above items [2], [6] and [8],

a salt thereof, a quaternary ammonium salt thereof, an N-oxide form thereof, the solvate thereof, or a prodrug thereof;

[16] The compound according to the above item [15],

wherein $R^{II}$ is a chlorine atom, methyl, methoxy, allyloxy, propargyloxy or cyano;

$R^2$ is propyl, cyclopropylmethyl, cyclobutyl or cyclopentyl;

$R^5$ is benzyl which may have a substituent(s), phenylethyl which may have a substituent(s) or butyl;

k is an integer of 1 to 5;

[17] A compound represented by formula (II-C):

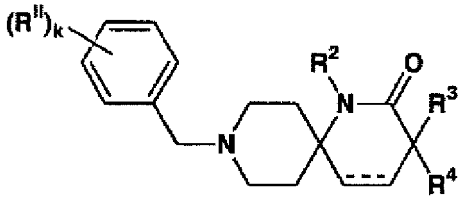

(II-C)

wherein all symbols have the same meanings as those described in the above items [2], [6] and [8] or a compound represented by formula (II-D):

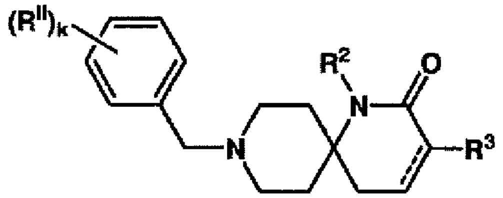

(II-D)

wherein all symbols have the same meanings as those described in the above items [2], [6] and [8], a salt thereof, a quaternary ammonium salt thereof, an N-oxide form thereof, a solvate thereof, or a prodrug thereof;
[18] The compound according to the above item [17],
wherein $R^{II}$ is a chlorine atom, methyl, methoxy, allyloxy, propargyloxy or cyano;
$R^2$ is propyl, cyclopropylmethyl, cyclobutyl or cyclopentyl;
$R^3$ is benzyl which may have a substituent(s), pyridylmethyl which may have a substituent(s), or indolylmethyl which may have a substituent(s);
$R^4$ is a hydrogen atom; and
k is an integer of 1 to 5;
[19] (3R)-3-benzyl-9-(4-chlorobenzyl)-1-propyl-1,4,9-triazaspiro[5.5]undecane-2,5-dione,
(3R)-3-benzyl-9-(4-chlorobenzyl)-1-(cyclopropylmethyl)-1,4,9-triazaspiro[5.5]undecane-2,5-dione,
(3R)-3-benzyl-9-(4-chlorobenzyl)-1-cyclobutyl-1,4,9-triazaspiro[5.5]undecane-2,5-dione,
(3R)-3-benzyl-9-(4-chlorobenzyl)-1-cyclopentyl-1,4,9-triazaspiro[5.5]undecane-2,5-dione,
(35)-3-benzyl-9-(4-chlorobenzyl)-1-cyclobutyl-1,4,9-triazaspiro[5.5]undecane-2,5-dione,
(3R)-9-(4-chlorobenzyl)-1-propyl-3-(3-pyridinylmethyl)-1,4,9-triazaspiro[5.5]undecane-2,5-dione,
(3R)-9-(4-chlorobenzyl)-1-(cyclopropylmethyl)-3-(3-pyridinylmethyl)-1,4,9-triazaspiro[5.5]undecane-2,5-diorte,
(3R)-9-(4-chlorobenzy)-1-cyclobutyl-5-(3-pyridinylmethyl)-1,4,9-triazaspiro[5.5]undecane-2,5-dione,
(3R)-9-(4-chiorobenzyl)-1-cyclopentyl-3-(3-pyridinylmethyl)-1,4,9-triazaspiro[5.5]undecane-2,5-dione,
(3R)-9-(4-chlorobenzyl)-3-(1H-indol-3-ylmethyl)-1-propyl-1,4,9-triazaspiro[5.5]undecane-2,5-dione,
(3R)-9-(4-chlorobenzyl)-1-(cyclopropylmethyl)-3-(1 H-indol-3-ylmethyl)-1,4,9-triazaspiro[5.5]undecane-2,5-dione,
(3R)-9-(4-chlorobenzyl)-1-cyclobutyl-3-(1H-indol-3-ylmethyl)-1,4,9-triazaspiro[5.5]undecane-2,5-dione,
(3R)-9-(4-chlorobenzyl)-1-cyclopentyl-3-(1H-indol-3-ylmethyl)-1,4,9-triazaspiro[5.5]undecane-2,5-dione,
3-benzyl-9-(biphenyl-4-ylmethyl)-1-propyl-1,4,9-triazaspiro[5.5]undecane-2,5-dione,
9-[4-(allyloxy)benzyl]-3-benzyl-1-propyl-1,4,9-triazaspiro[5.5]undecane-2,5-dione,
3-[4-(prop-2-yn-1-yloxy)benzyl]-3-azaspiro[5.5]undecane,
9-(4-chlorobenzyl)-3-(2-phenylethyl)-1-propyl-1,3,9-triazaspiro[5.5]undec-4-en-2-one,
3-benzyl-9-(4-chlorobenzyl)-1-(cyclopropylmethyl)-1,3,9-triazaspiro[5.5]undec-4-en-2-one,
3-benzyl-9-(4-chlorobenzyl)-1-propyl-1,3,9-triazaspiro[5.5]undec-4-en-2-one,
9-(4-chlorobenzyl)-1-(cyclopropylmethyl)-3-(2-phenylethyl)-1 ,3,9-triazaspiro[5.5]undec-4-en-2-one,
9-(4-chlorobenzyl)-1-cyclobutyl-3-(2-phenylethyl)-1,3,9-triazaspiro[5.5]undec-4-en-2-one,
3-butyl-9-(4-chlorobenzyl)-1-propyl-1,3,9-triazaspiro[5.5]undec-4-en-2-one,
3-butyl-9-(4-chlorobenzyl)-1-(cyclopropylmethyl)-1,3,9-triazaspiro[5.5]undec-4-en-2-one,
3-butyl-9-(4-chlorobenzyl)-1-cyclopentyl-1,3,9-triazaspiro[5.5]undecan-2-one,
9-(4-chlorobenzyl)-3-(2-phenylethyl)-1-propyl-1,3,9-triazaspiro[5.5]undecan-2-one,
9-(4-chlorobenzyl)-1-cyclobutyl-3-(2-phenylethyl)-1,3,9-triazaspiro[5.5]undecan-2-one,
9-(4-chlorobenzyl)-1-cyclopentyl-3-(2-phenylethyl)-1,3,9-triazaspiro[5.5]undecan-2-one,
3-butyl-9-(4-chlorobenzyl)-1-(cyclopropylmethyl)-1,3,9-triazaspiro[5.5]undecan-2-one,
3-butyl-9-(4-chlorobenzyl)-1-cyclobutyl-1,3,9-triazaspiro[5.5]undecan-2-one,
3-benzyl-9-(4-chlorobenzyl)-1-(cyclopropylmethyl)-1,9-diazaspiro[5.5]undecan-2-one, or
3-benzyl-9-(4-chlorobenzyl)-1-propyl-1,9-diazaspiro[5.5]undecan-2-one,
a salt thereof, a quaternary ammonium salt thereof, an N-oxide form thereof, or a solvate thereof, or a prodrug thereof;
[20] A pharmaceutical composition which comprises the compound represented by formula (II-B) described in the above item [15], formula (II-C) described in the above item [17], formula (II-D) described in the above [17], or the compound according to the above item [19], a salt thereof, a quaternary ammonium salt thereof, an N-oxide form thereof, a solvate thereof, or a prodrug thereof;
[21] A medicament comprising the compound represented by formula (I) described in the above item [1], a salt thereof, a quaternary ammonium salt thereof, an N-oxide form thereof, a solvate thereof, or a prodrug thereof, and one or more agent(s) selected from a nonsteroidal antiinflammatory drug, a disease modifying anti-rheumatic drug, steroids, an immunosuppressant agent, an antiinflammatory enzyme preparations, a chondroprotective agents, a T-cell inhibitor, a TNFα inhibitor, a prostaglandin synthase inhibitor, an IL-1 inhibitor, an IL-6 inhibitor, an interferon

gamma agonist, prostaglandins, a phosphodiesterase inhibitor, a metalloproteinase inhibitor, and a chemokine receptor antagonist in combination;

[22] A method for antagonizing against CXCR3 in a mammal, which comprises administering to a mammal an effective amount of a compound represented by formula (I) described in the above item [1], a salt thereof, a quaternary ammonium salt thereof, an N-oxide form thereof, a solvate thereof, or a prodrug thereof;

[23] A method for preventing, treating or progression-suppressing for CXCR3-related disease in a mammal, which comprises administering to a mammal an effective amount of the compound represented by formula (I) described in the above item [1], a salt thereof, a quaternary ammonium salt thereof, an N-oxide form thereof, a solvate thereof, or a prodrug thereof;

[24] A use of the compound represented by formula (I) described in the above item [1], a salt thereof, a quaternary ammonium salt thereof, an N-oxide form thereof, a solvate thereof, or a prodrug thereof, for the manufacture of a CXCR3 antagonist;

[25] A method for producing a compound represented by formula (I) described in the above item [1], a salt thereof, a quaternary ammonium salt thereof, an N-oxide form thereof, a solvate thereof, or a prodrug thereof, or the like.

In the present description, for an autoimmune disease, for example, a systemic lupus erythematosus, a rheumatoid arthritis, a multiple sclerosis, a type I diabetes, a psoriasis, a psoriatic arthritis, a Hashimoto's thyroiditis, a Goodpasture's syndrome, a pemphigus, an autoimmunity of a receptor (a Graves' disease, a myasthenia gravis, an insulin resistance), an autoimmune hemolytic anemia, an autoImmune thrombopenic purpura, a hidebound disease with an anticollagen immune body, a mixing connective tissue disease, a polymyositis, a pernicious anemia, an idiopathic Addison's disease, a glomerulonephritis, a bullous pemphigoid, a Sjogren's syndrome, an atherosclerosis, an adrenergic agent fastness (One part is accompanied with an asthma or a cystic fibrosis), an active chronic hepatitis, a primary biliary liver cirrhosis, a vitiligo, an angiitis, an urticaria, an atopic dermatitis and an asthma and the like are given. For an autoimmune disease, a systemic lupus erythematosus, a rheumatoid arthritis, a multiple sclerosis, a type I diabetes and a psoriasis are given preferably.

In the present description, for an allergic disease, for example, an atopic dermatitis, an allergic rhinitis, a bronchial asthma, an allergic angiitis, an allergic conjunctivitis, an allergic eye disease, an alimentary allergy and an intolerance, an allergic pulmonary disease, an anaphylaxis or an anaphylactoid reaction and the like are given. For an allergic disease, an atopic dermatitis is given preferably.

In a rejection response which is described in the present description, an acute rejection response happened within 3 months and a chronic rejection response happened after it, and a graft-versus-host disease are included.

In the present description, for a graft, a transplant (for example, a kidney, a liver, a heart, a lung, an intestinum tenue and the like), an implant (for example, a skin (for example, a full thickness skin graft, an epidermis graft, a dermal graft, a Davis graft and the like), a cornea, a vessel, a chorda, a bone, a foetus organization and the like) or a graft cell (for example, a bone marrow cell, a hematopoietic stem cell, a peripheral blood stem cell, a cord blood stem cell, an islet cell, an islet cell of Langerhans which is the one part thereof, a hepatic cell, a nerve cell, a bowel epidermic cell and the like) is called. For an organ, a kidney, a liver, a heart and a lungs are given preferably. For a tissue, a skin, a cornea, a vessel, a chorda and a bone are given preferably. For a cell, a bone marrow cell, a nerve cell, an islet cell are given preferably.

In the present description, for an inflammatory bowel disease, for example, a Crohn's disease and a colitis ulcerosa and the like are given.

In the present description, for a respiratory disease, for example, an asthma, a chronic obstructive pulmonary disease, a respiratory insufficiency, a lungs irritation, an acute respiratory distress syndrome (ARDS), an allergic bronchopulmonary aspergillosis and the like are given. For a respiratory disease, a chronic obstructive pulmonary disease is given preferably.

In the present description, for a carcinoma disease, for example, a leucaemia, a solid cancer and a cancer metastasis or the like are given.

In the present description, for "aliphatic hydrocarbon group" in "aliphatic hydrocarbon group which may have a substituent(s)" which is represented by $R^1$, for example, "linear or branched C1-18 hydrocarbon group" and the like is given. For "linear or branched C1-18 hydrocarbon group", for example, C1-18 alkyl group, C2-18 alkenyl group and C2-18 alkynyl group and the like are given. Here, for C1-18 alkyl group, for example, methyl, ethyl, propyl, isopropyl, butyl, isobutyl, sec- butyl, tert- butyl, pentyl, hexyl, heptyl, octyl, nonyl, decyl, undecyl, dodecyl, tridecyl, tetradecyl, pentadecyl, hexadecyl, heptadecyl, octadecyl group and isomer group thereof or the like are given. For C2-18 alkeny group, for example, vinyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, butadienyl, pentadienyl, hexadienyl, heptadienyl, octadienyl, nonadienyl, decadienyl, undecadienyl, dodecadienyl, tridecadienyl, tetradecadienyl, pentadecadienyl, hexadecadienyl, heptadecadienyl, octadecadienyl, hexatrienyl, heptatrienyl, octatrienyl, nonatrienyl, decatrienyl, undecatrienyl, dodecatrienyl, tridecatrienyl, tetradecatrienyl, pentadecatrienyl, hexadecatrienyl, heptadecatrienyl, octadecatrienyl group and isomer group thereof or the like are given. For C2-18 alkynyl group, for example, ethynyl, propynyl, butynyl, pentynyl, hexynyl, heptynyl, octynyl, nonynyl, decynyl, undecynyl, dodecynyl, tridecynyl, tetradecynyl, pentadecynyl, hexadecynyl, heptadecynyl, octadecynyl, butadiynyl, pentadiynyl, hexadiynyl, heptadiynyl, octadiynyl, nonadiynyl, decadiynyl, undecadiynyl, dodecadiynyl, tridecadiynyl, tetradecadiynyl, pentadecadiynyl, hexadecadiynyl, heptadecadiynyl, octadecadiynyl, hexatriynyl, heptatriynyl, octatriynyl, nonatriynyl, decatriynyl, undecatriynyl, dodecatriynyl, tridecatriynyl, tetradecatriynyl, pentadecatriynyl, hexadecatriynyl, heptadecatriynyl, octadecatriynyl group and isomer group thereof and the like are given.

In the present description, "substituent" in "aliphatic hydrocarbon group which may have a substituent(s)" which is represented by $R^1$ is not limited in particular if it is substituent. For this "substituent", for example, (a) the substituent selected from the first group described afterward, (b) the substituent selected from the second group described afterward and (c) cyclic group which may have a substituent(s) and the like are given. These optional substituents may substitute for 1-5 at displaceable position.

<The first group>

(1) halogen atom (chlorine, bromine, fluorine or iodine atom), (2) nitro group, (3) trifluoromethyl group, (4) trifluoromethoxy group, (5) cyano group, (6) oxo group.

<The second group>

(1) $-SR^{a1}$, (2) $-SO_2R^{a1}$, (3) $-SO_2NR^{b1}R^{b2}$, (4) $-S(O)R^{a1}$, (5) $-OR^{a1}$, (6) $-OCOR^{a1}$, (7) $-NR^{a1}SO_2R^{a2}$, (8) $-NR^bR^{b2}$, (9) $-NR^{a1}COR^{a2}$, (10) $-NR^{a1}COOR^{a2}$, (11) $-NR^{a1}CONR^{b1}R^{b2}$, (12) $-N(SO_2R^{a1})_2$, (13) $-COR^{a1}$, (14) $-COOR^{a1}$, (15) $-CONR^{b1}R^{b2}$, (16) $-CONR^{a1}COR^{a2}$, (17) $-COCOOR^{a1}$, (18) $-B(OR^{a1})_2$

[wherein, $R^{a1}$, $R^{a2}$, $R^{b1}$ and $R^{b2}$ represent each independently hydrogen atom, cyclic group (ring1) which may have a substituent(s) or aliphatic hydrocarbon group which may have a substituent(s), or $R^{b1}$ and $R^{b2}$ take together to represent (1) -C2-6 alkylene-, (2) -(C2-6 alkylene)-O-(C2-6 alkylene)-, (3) -(C2-6 alkylene)-S-(C2-6 alkylene)-, (4) -(C2-6 alkylene)-$NR^{N1}$-(C2-6 alkylene)-(wherein, for C2-6 alkylene, for example, methylene, ethylene, trimethylene, tetramethylene, pentamethylene, hexamethylene group and isomer group thereof or the like are represented, $R^{N1}$ represents hydrogen atom, cyclic group (ring1) which may have a substituent(s) or C1-8 alkyl group (wherein, C1-8 alkyl group is as defined afterward))].

Here, for "aliphatic hydrocarbon group" in "aliphatic hydrocarbon group which may have a substituent(s)" which is represented by $R^{a1}$, $R^{a2}$, $R^{b1}$ and $R^{b2}$, for example, "linear or branched C1-8 hydrocarbon group" and the like is given. For "linear or branched C1-8 hydrocarbon group", for example, C1-8 alkyl group, C2-8 alkenyl group and C2-8 alkynyl group and the like are given. Here, for C1-8 alkyl group, for example, methyl, ethyl, propyl, isopropyl, butyl, sec- butyl, tert- butyl, pentyl, hexyl, heptyl, octyl group and isomer group hereof and the like are given. For C2-8 alkeny group, for example, vinyl, propenyl, butenyl, pentenyl, hexenyl, heptenyl, octenyl, butadienyl, pentadienyl, hexadienyl, heptadienyl, octadienyl, hexatrienyl, heptatrienyl, octatrienyl group and isomer group hereof and the like are given. For C2-8 alkynyl group, for example, ethynyl, propynyl, butynyl, pentynyl, hexynyl, heptynyl, octynyl, butadiynyl, pentadiynyl, hexadiynyl, heptadiynyl, octadiynyl, hexatriynyl, heptatriynyl, octatriynyl group and isomer group hereof and the like are given. In addition, "substituent" in "aliphatic hydrocarbon group which may have a substituent(s)" which is represented by $R^{a1}$, $R^{a2}$, $R^{b1}$ and $R^{b2}$ is not limited in particular if it is substituent. For this "substituent", for example, (a) cyclic group (ring 1) which may have a substituent(s) and (b) the substituent selected from the third group described afterward and the like are given. These optional substituents may substitute for 1-5 at displaceable position.

<The third group>

(1) halogen atom (it is as defined above), (2) $-OR^{c1}$, (3) $-SR^{c1}$, (4) $-NR^{d1}R^{d2}$, (5) $-COOR^{c1}$, (6) $-CONR^{d1}R^{d2}$, (7) $-NR^{c1}COR^{c2}$, (8) $-NR^{c1}SO_2R^{c2}$, (9) $-N(SO_2R^{c1})_2$

[wherein, $R^{c1}$, $R^{c2}$, $R^{d1}$ and $R^{d2}$ are same as $R^{a1}$, $R^{a2}$, $R^{b1}$ and $R^{b2}$ described above respectively. However, $R^{c1}$, $R^{c2}$, $R^{d1}$ and $R^{d2}$ are not represented by aliphatic hydrocarbon group which was substituted by the substituent selected from the present group (the third group) all].

In the present description, for example, "cyclic group" in "cyclic group (ring 1) which may have a substituent (s)", carbocyclic ring and heterocyclic ring and the like are given.

For carbocyclic ring, for example, C3-15 monocyclic ring or polycyclic carbocyclic aryl ring which may be saturated with a part or all and the like are given. Here, for "C3-15 monocyclic ring or polycyclic carbocyclic aryl ring which may be saturated with a part or all", for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene and the like are given. In addition, polycyclic carbocyclic ring spiro-bonded or polycyclic carbocyclic ring bridged in "C3-15 monocyclic ring or polycyclic carbocyclic aryl ring which may be saturated with a part or all" is included, for example, spiro[4.4]nonane, spiro[4.5]decane, spiro[5.5]undecane, bicyclo[2.2.1]heptane, bicyclo[2.2.1]hepta-2-ene, bicyclo[3.1.1]heptane, bicyclo[3.1.1]hepta-2-ene, bicyclo[2.2.2]octane, bicyclo[2.2.2]octa-2-ene, adamantane, noradamantane and the like are given.

On the other hand, for heterocyclic ring, for example, 3- to 15 membered monocyclic ring or polycyclic heterocyclic aryl ring including 1-4 nitrogen atom(s), 1-3 oxygen atom(s) and/or 1-3 sulfur atom(s), the heterocyclic ring saturated with a part or all, polycyclic heterocyclic ring spiro-bonded and polycyclic heterocyclic ring bridged and the like are given. Here, for "3- to 15 membered monocyclic ring or polycyclic heterocyclic aryl ring including 1-4 nitrogen atom(s), 1-3 oxygen atom(s) and/or 1-3 sulfur atom(s)", for example, pyrrole, imidazole, triazole, tetrazole, pyrazole, pyridine, pyrazine, pyrimidine, pyridazine, azepine, diazepine, furan, pyran, oxepin, thiophene, thiaine (thiopyran), thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, benzofuran, iso benzofuran, benzothiophene, isobenzothiophene, indazole, quinoline, isoquinoline, phthalazine, naphthyridine, quinoxaline, quinazoline, cinnoline, benzoxazole, benzothiazole, benzimidazole, benzoxepin, benzoxaazepine, benz oxadiazepine, benzothiepine, benzothiaazepine, benzothiadiazepine, benzoazepine, benzodiazepine, benzofurazan, benzothiadiazole, benzotriazole, carbazole, acridine, dibenzofuran, dibenzothiophene and the like are given. In addition, among "3- to 15 membered monocyclic ring or polycyclic heterocyclic ring (condensation or spiro) including 1-4 nitrogen atom(s), 1-3 oxygen atom(s) and/or 1-3 sulfur atom(s)", for the ring which is saturated with a part or all, for example, pyrroline, pyrrolidine, imidazoline, imidazolidine, pyrazoline, pyrazolidine, triazoline, triazolidine, tetrazolidine, tetrazolidine, dihydropyridine, tetrahydropyridine, piperidine, dihydropyrazine, tetrohydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrothiophene, tetrahydrothiophene, dihydrothiaine (dihydrothiopyran), tetrahydrothiaine (tetrahydrothiopyran), dihydrooxazole, tetrahydrooxazole, dihydroisoxazole, tetrahydroisoxazole, dihydrothiazole, tetrahydrothiazole, dihydroisothiazole, tetrahydroisothiazole, dihydrooxadiazole, tetrahydrooxadiazole, dihydrothiodiazole, tetrahydrothiodiazole, tetrahydrooxadiazine, tetrahydro thiadiazine, tetrahydrooxaazepine, tetrahydrooxadiazepine, perhydrooxaazepine, perhydrooxadiazepine, tetrahydrothiaazepine, tetrahydro thiadiazepine, perhydrothiaazepine, perhydrothiadiazepine, morpholine, thiomorpholine, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydro phthalazine, perhydrophthalazine, dihydronaphthyridine, tetrahydronaphthyridine, perhydronaphthyridine, dihydroquinaxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzooxazole, perhydrobenzo oxazole, dihydrobenzothiazole, perhydrobenzothiazole, dihydrobenzimidazole, perhydrobenzimidazole, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, benzodioxolane, benzodioxane, benzodithiolane, benzodithiane, 2,4,6-trioxaspiro[bicyclo[3.3.0]octane-3,1'-cyclohexane], 1,3-dioxolano[4,5-g]chromene, 2-oxabicyclo[2.2.1]heptane and the like are given.

In the present description, "substituent" in "cyclic group (ring 1) which may have a substituent(s)" is not limited in particular if it is substituent. For this "substituent", for example, (a) aliphatic hydrocarbon group which may have a substituent(s), (b) cyclic group (ring 2) which may have a substituent(s), (c) the substituent selected from the first group described above and (d) the substituent selected from the fourth group described afterward and the like are given. These optional substituents may substitute for 1-5 at displaceable position. Here, "aliphatic hydrocarbon group" in "aliphatic hydrocarbon group which may have a substituent(s)" as "substituent" is as "linear or branched C1-8 hydrocarbon group" described above. "substituent" in "aliphatic hydrocarbon group which may have a substituent(s)" is not limited in particular if it is substituent. For this "substituent", for example, (a) cyclic group which may have a substituent(s) (ring 2), (b) the substituent selected from the first group described above and (c) the substituent selected from the fourth group described afterward and the like are given. These optional substituents may substitute for 1-5 at displaceable position.

<The fourth group>

(1) $-SR^{e1}$, (2) $-SO_2R^{e1}$, (3) $-SO_2NR^{f1}R^{f2}$, (4) $-S(O)R^{e1}$, (5) $-OR^{e1}$, (6) $-OCOR^{e1}$, (7) $-NR^{e1}SO_2R^{e2}$, (8) $-NR^{f1}R^{f2}$, (9) $-NR^{e1}COR^{e2}$, (10) $-NR^{e1}COOR^{e2}$, (11) $-NR^{e1}CONR^{f1}R^{f2}$, (12) $-N(SO_2R^{e1})_2$, (13) $-COR^{e1}$, (14) $-COOR^{e1}$, (15) -

$CONNR^{f1}R^{f2}$, (16) $-CONR^{e1}COR^{e2}$, (17) $-COCOOR^{e1}$, (18) $-B(OR^{e1})_2$ [wherin, $R^{e1}$, $R^{e2}$, $R^{f1}$ and $R^{f2}$ represent each independently hydrogen atom, cyclic group (ring2) which may have a substituent(s) or aliphatic hydrocarbon group which may have a substituent(s), or $R^{f1}$ and $R^{f2}$ take together to represent (1) -C2-6 alkylene-, (2) -(C2-6 alkylene)-O-(C2-6 alkylene)-, (3) -(C2-6 alkylene)-S-(C2-6 alkylene)-, (4) -(C2-6 alkylene)-$NR^{N2}$-(C2-6 alkylene)-(wherein, C2-6 alkylene is as defined above, and $R^{N2}$ represents hydrogen atom, phenyl group or C1-8 alkyl group which may have phenyl group (wherein, C1-8 alkyl group is as defined above))].

Here, "aliphatic hydrocarbon group" in "aliphatic hydrocarbon group which may have a substituent(s)" which is represented by $R^{e1}$, $R^{e2}$, $R^{f1}$ and $R^{f2}$ is as "linear or branched C1-8 hydrocarbon group" described above. In addition, "substituent" in "aliphatic hydrocarbon group which may have a substituent(s)" which is represented by $R^{e1}$, $R^{e2}$, $R^{f1}$ and $R^{f2}$ is not limited in particular if it is substituent. For this "substituent", for example, (a) cyclic group (ring 2) which may have a substituent(s) and (b) the substituent selected from the fifth group described afterward is given. These optional substituents may substitute for 1-5 at displaceable position.

<The fifth group>

(1) halogen atom (it is as defined above.), (2) $-OR^{g1}$, (3) $-SR^{g1}$, (4) $-NR^{h1}R^{h2}$, (5) $-COOR^{g1}$, (6) $-CONR^{h1}R^{h2}$, (7) $-NR^{g1}COR^{g2}$, (8) $-NR^{g1}SO_2R^{g2}$, (9) - $N(SO_2R^{g1})_2$, (10) $-SO_2NR^{h1}R^{h2}$

[In the group, $R^{g1}$, $R^{g2}$, $R^{h1}$ and $R^{h2}$ are same as $R^{e1}$, $R^{e2}$, $R^{f1}$ and $R^{f2}$ described above respectively. However, $R^{g1}$, $R^{g2}$, $R^{h1}$ and $R^{h2}$ are not represented by aliphatic hydrocarbon group which was substituted by the substituent selected from the present group (the fifth group) all].

In the present description, "cyclic group" in "cyclic group (ring 2) which may have a substituent(s)" is as "cyclic group" in "cyclic group (ring 1) which may have a substituent(s)" described above.

In the present description, "substituent" in "cyclic group (ring 2) which may have substituents" is not limited in particular if it is substituent. For this "substituent", for example, (a) aliphatic hydrocarbon group which may have a substituent(s), (b) C3-8 mono-carbocyclic ring which may have a substituent(s) or 3- to 8-membered mono-heterocyclic ring which may have a substituent(s), (c) the substituent selected from the first group described above and (d) the substituent selected from the sixth group described afterward and the like are given. These optional substituents may substitute for 1-5 at displaceable position. Here, "aliphatic hydrocarbon group" in "aliphatic hydrocarbon group which may have a substituent(s)" as "substituent" is as "linear or branched C1-8 hydrocarbon group" described above. "Substituent" in "aliphatic hydrocarbon group which may have a substituent(s)" is not limited in particular if it is substituent. For this "substituent", for example, (a) C3-8 mono-carbocyclic ring which may have a substituent(s) or 3- to 8-membered mono-heterocyclic ring which may have a substituent(s), (b) the substituent selected from the first group described above and (c) the substituent selected from the sixth group described afterward and the like are given. These optional substituents may substitute for 1-5 at displaceable position.

<The sixth group>

(1) $-SR^{i1}$, (2) $-SO_2R^{i1}$, (3) $-SO_2NR^{j1}R^{j2}$, (4) $-S(O)R^{i1}$ (5) $-OR^{i1}$, (6) $-OCOR^{i1}$, (7) $-NR^{i1}SO_2R^{i2}$, (8) $-NR^{j1}R^{j2}$, (9) $-NR^{i1}COR^{i2}$, (10) $-NR^{i1}COOR^{i2}$, (11) - $NR^{i1}CONR^{j1}R^{j2}$, (12) $-N(SO_2R^{i1})_2$, (13) $-COR^{i1}$, (14) $-COOR^{i1}$, (15) - $CONR^{j1}R^{j2}$, (16) $-CONR^{i1}COR^{i2}$, (17) $-COCOOR^{i1}$ (18) $-B(OR^{i1})_2$

[In the group, $R^{i1}$, $R^{i2}$, $R^{j1}$ and $R^{j2}$ represent each independently hydrogen atom, C3-8 mono-carbocyclic ring which may have a substituent(s), 3- to 8-membered mono-heterocyclic ring which may have a substituent(s) or aliphatic hydrocarbon group which may have a substituent(s), or $R^{j1}$ and $R^{j2}$ take together to represent (1) -C2-6 alkylene-, (2) -(C2-6 alkylene)-O-(C2-6 alkylene)-, (3) -(C2-6 alkylene)-S-(C2-6 alkylene)-, (4) -(C2-6 alkylene)-$NR^{N2}$-(C2-6 alkylene)-(wherein, C2-6 alkylene is as defined above, and $R^{N2}$ is as defined above)].

Here, "aliphatic hydrocarbon group" in "aliphatic hydrocarbon group which may have a substituent(s)" which is represented by $R^{i1}$, $R^{i2}$, $R^{j1}$ and $R^{j2}$ is as "linear or branched C1-8 hydrocarbon group" described above.

"Substituent" in "aliphatic hydrocarbon group which may have a substituent(s)" which is represented by $R^{i1}$, $R^{i2}$, $R^{j1}$ and $R^{j2}$ is not limited in particular if it is substituent. For this "substituent", for example, (a) C3-8 mono-carbocyclic ring or 3- to 8-membered mono-heterocyclic ring and (b) the substituent selected from the seventh group described afterward is given. These optional substituents may substitute for 1-5 at displaceable position.

<The seventh group>

(1) halogen atom (it is as defined above.), (2) $-OR^{k1}$, (3) $-SR^{k1}$, (4) $-NR^{m1} R^{m2}$, (5) $-COOR^{k1}$, (6) $-CONR^{m1}R^{m2}$, (7) $-NR^{k1}COR^{k2}$, (8) $-NR^{k1}SO_2R^{k2}$, (9) - $N(SO_2R^{k1})_2$

[In the group, $R^{k1}$, $R^{k2}$, $R^{m1}$ and $R^{m2}$ are same as $R^{i1}$, $R^{i2}$, $R^{j1}$ and $R^{j2}$ described above respectively. However, $R^{k1}$, $R^{k2}$, $R^{m1}$ and $R^{m2}$ are not represented by aliphatic hydrocarbon group which was substituted by the substituent selected

from the present group (the seventh group) all].

In the present description, for "C3-8 mono-carbocyclic ring or 3- to 8-membered mono-heterocyclic ring" in "C3-8 mono-carbocyclic ring which may have a substituent(s) or 3- to 8-membered mono-heterocyclic ring which may have a substituent(s)", for example, C3-8 mono-carbocyclic aryl ring which may be saturated with a part or all or 3- to-8-membered mono-heterocyclic aryl ring including 1-4 nitrogen atom(s), 1-2 oxygen atom(s) and/or 1-2 sulfur atom(s), and heterocyclic ring saturated with a part or all and the like are given.

For "C3-8 mono-carbocyclic aryl ring which may be saturated with a part or all", for example, cyclopropane, cyclobutane, cyclopentane, cyclohexane, cycloheptane, cyclooctane, cyclopentene, cyclohexene, cycloheptene, cyclooctene, cyclopentadiene, cyclohexadiene, cycloheptadiene, cyclooctadiene, benzene and the like are given. In addition, for "3- to 8-membered mono-heterocyclic aryl ring including 1-4 nitrogen atom(s), 1-2 oxygen atom(s) and/or 1-2 sulfur atom(s)", for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine and the like are given. For heterocyclic ring saturated with a part or all, among "3- to 8-membered mono-heterocyclic aryl ring including 1-4 nitrogen atom(s), 1-2 oxygen atom(s) and/or 1-2 sulfur atom(s)", for example, aziridine, azethidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thiethane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (iso oxazolidine), dihydrothiazole, tetrahydrothiazole, (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydro oxadiazepine, tetrahydrooxadiazepine, perhydrooxadiazepine, dihydro thiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydro thiadiazepine, perhydrothiadiazepine, morpholine, thiomorpholine, oxathiane, dioxolane, dioxane, dithiolane, dithiane and the like are given.

In the present description, "substituent" in "C3-8 mono-carbocyclic ring which may have a substituent(s) or 3- to 8-membered mono-heterocyclic ring which may have a substituent(s)" is not limited in particular if it is substituent. For this "substituent", for example, (a) C1-8 alkyl group (it is as defined above), (b) the substituent selected from the first group described above and (c) the substituent selected from the eighth group described afterward and the like are given.

<The eighth group>

(1) $-OR^{n1}$, (2) $-NR^{o1}R^{o2}$, (3) $-COOR^{n1}$, (4) $-SR^{n1}$, (5) $-CONR^{o1}R^{o2}$
[wherein, $R^{n1}$, $R^{o1}$ and $R^{o2}$ represent each independently hydrogen atom, phenyl group or C1-8 alkyl group which may have phenyl group, or $R^{o1}$ and $R^{o2}$ take together to represent (1) -C2-6 alkylene-, (2) -(C2-6 alkylene)-O-(C2-6 alkylene)-, (3) -(C2-6 alkylene)-S-(C2-6 alkylene)-, (4) -(C2-6 alkylene)-$NR^{N2}$-(C2-6 alkylene)-(wherein, C1-8 alkyl group, C2-6 alkylene and $R^{N2}$ are as defined above)].

In the present description, "cyclic group which may have a substituent(s)" is same as "cyclic group (ring1) which may have a substituent(s)" described above.

In the present description, "substituent" which is represented by $R^{II}$ is same as "substituent" in "cyclic group (ring1) which may have a substituent(s)" described above.

In the present description, ring A represents 5- to 8-membered cyclic group which may have a substituent(s), and this "substituent" is not limited in particular if it is substituent. Specifically, the group which is represented by $R^2$, $R^3$, $R^4$ and $R^5$ described afterward and the oxo group and the like are given. These may substitute for 1-8 at displaceable position. In addition, ring A may further be condensed to C3-8 mono-carbocyclic ring which may have a substituent(s) or 3- to 8-membered mono-heterocyclic ring which may have a substituent(s) described afterward in a position which can be condensed.

In the present description, for "5- to 8-membered cyclic group" in "5- to 8-membered cyclic group which may have a substituent(s)" which is represented by ring A, for example, C5-8 carbocyclic ring and 5- to 8-membered heterocyclic ring and the like are given.

For C5-8 carbocyclic ring, for example, C5-8 mono-carbocyclic aryl ring saturated with a part or all and the like are given. For "C5-8 mono-carbocyclic aryl ring saturated with a part or all", for example, cyclopentane, cyclohexane,

cycloheptane, cyclooctane, cyclopentene, cyclohexene, cycloheptene, cyclooctene, cyclopentadiene, cyclohexadiene, cycloheptadiene, cycloocta diene or the like are given. In addition, carbocyclic ring bridged is included in "C5-8 mono-carbocyclic aryl ring saturated with a part or all", for example, bicyclo[3.1.1]heptane, bicyclo[2.2.1]heptane, adamantine and the like are given.

On the other hand, for 5- to 8-membered heterocyclic ring, for example, "5- to 8-membered nitrogen-containing hetrocyclic ring" including one nitrogen atom at least and "5- to 8-membered no nitrogen-containing hetrocyclic ring" which does not include nitrogen atom and the like are given. For "5- to 8-membered nitrogen-containing hetrocyclic ring", for example, heterocyclic ring saturated with a part or all, among 5- to 8-membered mono-heterocyclic aryl ring which includes one nitrogen atom at least, which may be included 1-5 heteroatom(s) selected from nitrogen atom, oxygen atom and/or sulfur atom more by a wish, and the like are given. For "heterocyclic ring saturated with a part or all among 5- to 8-membered mono-heterocyclic aryl ring which includes one nitrogen atom at least, which may be included 1-5 heteroatom selected from nitrogen atom, oxygen atom and/or sulfur atom more by a wish", for example, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrooxazole, tetrahydrooxazole (oxazolidine), dihydroisoxazole, tetrahydroisoxazole (isooxazolidine), dihydrothiazole, tetrahydrothiazole (thiazolidine), dihydroisothiazole, tetrahydroisothiazole (isothiazolidine), dihydrofurazane, tetrahydrofurazane, dihydrooxadiazole, tetrahydrooxadiazole (oxadiazolidine), dihydrooxazine, tetrahydrooxazine, dihydrooxadiazine, tetra hydrooxadiazine, dihydrooxazepine, tetrahydrooxazepine, perhydrooxazepine, dihydrooxadiazepine, tetrahydrooxadiazepine, perhydrooxadiazepine, dihydro thiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothidiazepine, tetrahydrothidiazepine, perhydrothidiazepine, morpholine, thiomorpholine, oxadinane, thiazinane, dioxazine, oxazepane, tetrahydrooxazepine and the like are given. For "5- to 8-membered no nitrogen-containing hetrocyclic ring", for example, heterocyclic ring saturated with a part or all, among 5- to 8-membered mono-heterocyclic aryl ring which includes 1-6 heteroatom(s) selected from oxygen atom and/or sulfur atom, and the like are given. For "heterocyclic ring saturated with a part or all, among 5- to 8-membered mono-heterocyclic aryl ring which includes 1-6 heteroatom(s) selected from oxygen atom and/or sulfur atom", for example, dihydrofuran, tetrahydrofuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepine, tetrahydrothiepine, perhydrothiepine, oxathiane, dioxolane, dioxane, dithiolane, dithiane, oxathiolane and the like are given.

In the present description, "substituent" in "6-membered mono-carbocyclic ring which may have a substituent (s)" or "6-membered mono-heterocyclic ring which may have a substituent(s)" which is represented by ring $A^{II}$ is not limited in particular if it is substituent. Specifically, the group which is represented by $R^2$, $R^3$, $R^4$ and $R^5$ described afterward and oxo group and the like are given. These may substitute for 1-8 at displaceable position.

In the present description, for "6-membered mono-carbocyclic ring" in "6-membered mono-carbocyclic ring which may have a substituent(s)" which is represented by ring $A^{II}$, for example, cyclohexane, cyclohexene, cyclohexadiene and the like are given.

In the present description, for "6-membered mono-heterocyclic ring" in "6-membered mono-heterocyclic ring which may have a substituent(s)" which is represented by ring $A^{II}$, for example, "6-membered nitrogen-containing hetrocyclic ring" including at least one nitrogen atom and "6-membered no nitrogen-containing hetrocyclic ring" which does not include nitrogen atom, etc., are given. For "6-membered nitrogen-containing hetrocyclic ring", for example, 6-membered mono-heterocyclic ring which includes at least one nitrogen atom, which may be optionally included 1-5 heteroatom (s) selected from nitrogen atom, oxygen atom and/or sulfur atom, and the like are given. For "6-membered mono-heterocyclic ring which includes at least one nitrogen atom, which may be optionally included 1-5 heteroatom(s) selected from nitrogen atom, oxygen atom and/or sulfur atom", for example, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrooxazine, tetrahydrooxazine, dihydrooxadiazine, tetrahydrooxadiazine, dihydrothiazine, tetrahydrothiazine, dihydrothiadiazine, tetrahydrothiadiazine, morpholine, thiomorpholine or the like are given. For "6-membered no nitrogen-containing hetrocyclic ring", for example, 6-membered mono-heterocyclic ring which includes 1-6 heteroatom(s) selected from oxygen atom and/or sulfur atom and the like are given. For "6-membered mono-heterocyclic ring which includes 1-6 heteroatom(s) selected from oxygen atom and/or sulfur atom", for example, dihydropyran, tetrahydropyran, dihydrothiopyran, tetrahydrothiopyran, oxathiane, dioxane, dithiane and the like are given.

In the present description, "aliphatic hydrocarbon group" in "aliphatic hydrocarbon group which may have a substituent(s)" which is represented by $R^2$, $R^3$, $R^4$ and $R^5$ is as "linear or branched C1-8 hydrocarbon group" described above. "Substituent" in "aliphatic hydrocarbon group which may have a substituent(s)" is not limited in particular if it is substituent. This "substituent" is given, for example, (a) the substituent selected from the first group described above,

(b) the substituent selected from the second group described above or (c) cyclic group which may have a substituent (s) and the like, and these optional substituents may substitute for 1-5 at displaceable position. Here, "cyclic group which may have a substituent(s)" as substituent is as "cyclic group (ring1) which may have a substituent(s)" described above.

In the present description, hydroxy group which may be protected, carboxy group which may be protected, carbamoyl group which may be substituted, which is represented by $R^2$, $R^3$, $R^4$ and $R^5$ is as $-OR^{a1}$, $-COOR^{a1}$, $-CONR^{b1}R^{b2}$ which was given in the second group respectively [wherein, symbol is as defined above].

In the present description, "C3-8 mono-carbocyclic ring or 3- to 8-membered mono-heterocyclic ring" in "C3-8 mono-carbocyclic ring which may have a substituent(s) or 3- to 8- membered mono-heterocyclic ring which may have a substituent(s)" which may be condensed with ring A is as "C3-8 mono-carbocyclic ring or 3- to 8- membered mono-heterocyclic ring" in "C3-8 mono-carbocyclic ring which may have a substituent(s) or 3- to 8- membered mono-heterocyclic ring which may have a substituent(s)" which is represented by $R^{i1}$, $R^{i2}$, $R^{j1}$ and $R^{j2}$ described above. In addition, "substituent" in "C3-8 mono-carbocyclic ring which may have a substituent(s) or 3- to 8- membered mono-heterocyclic ring which may have a substituent(s)" which may be condensed with ring A is as "substituent" in "cyclic group which may have substituents" which is represented by $R^1$ described above.

In the present invention, each group which $R^1$, $R^2$, $R^3$, $R^4$, $R^5$, $R^{II}$, ring A and ring $A^{II}$ represents are preferable all. The group which is described in a example is preferable in particular.

In the present invention, $R^1$ is preferably aliphatic hydrocarbon group which may have a substituent(s), more preferably, aliphatic hydrocarbon group which may have "cyclic group (ring1) which may have a substituent(s)" as substituent. Here, "aliphatic hydrocarbon group" is preferably such as C1-10 aliphatic hydrocarbon group, more preferably such as C1-6 alkyl group and C2-6 alkenyl group, particularly preferably such as C1-6 alkyl group. In particular, C1-5 alkyl group is preferable, methyl group and pentyl group are more preferable. In addition, "cyclic group" in "cyclic group (ring1) which may have a substituent(s)" is preferably such as 3- to 10-membered monocyclic ring or polycyclic cyclic group, more preferably such as C3-6 cycloalkyl, C4-6 cycloalkenyl, benzene, pyrazole, thiazole, furan, thiophene, quinoline, benzodioxane, dioxaindane, benzofuran, imidazole, isothiazole, dihydropyrazole, pyridine, tetrahydropyran, triazole, pyrrole, oxazole, isoxazole, oxadiazole, particularly preferably such as cyclohexene, benzene, furan. In particular, benzene is preferable. "Substituent" in "cyclic group which may have a substituent(s)" is preferably such as C1-6 alkyl which may have a substituent(s), cyano group, halogen atom, benzene which may have a substituent(s), alkoxy group, alkenyloxy group, alkynyloxy group, more preferably such as phenyl group, cyano group, fluorine atom, chlorine atom, methoxy group, allyloxy group, propargyloxy group, most preferably, chlorine atom.

In the present invention, $R^1$ is preferably, as more concrete group, such as -(C1-6 alkyl)-(benzene which may have a substituent(s)), -(C1-6 alkyl)-(C4-6 cycloalkenyl which may have a substituent(s)), -(C1-6 alkyl)-(furan which may have a substituent(s)), more preferably such as -(C1-4 alkyl)-(benzene which may have a substituent(s)), particularly preferably such as benzyl group, chlorobenzyl group, fluorobenzyl group, cyanobenzyl group, methoxybenzyl group, allyloxybenzyl group, propargyloxybenzyl group.

In the present invention, $R^{II}$ is preferably halogen atom, aliphatic hydrocarbon group which may have a substituent(s), cyclic group which may have a substituent(s), alkoxy group, alkenyloxy group, alkynyloxy or cyano group, more preferably, chlorine atom, benzene ring, methyl group, methoxy group, allyloxy group, propargyloxy group or cyano group.

In the present invention, "cyclic group" in "cyclic group (ring1) which may have a substituent(s)" is preferably such as 3- to 10-membered monocyclic ring or polycyclic cyclic group, more preferably such as cyclopropane, benzene, cyclohexane, cyclohexene, thiophene, pyrazole, isothiazole, thiazole, imidazole, furan, dihydropyrazole, quinoline, benzodioxan, dioxaindane, benzofuran, pyridine, tetrahydropyran, triazole, pyrrole, oxazole, isoxazole, oxadiazole, particularly preferably such as cyclopropane, benzene, pyridine. In particular, benzene is preferable.

In the present invention, "C3-8 mono-carbocyclic ring or 3- to 8-membered mono-heterocyclic ring" in "C3-8 mono-carbocyclic ring which may have a substituent(s) or 3- to 8-membered mono-heterocyclic ring which may have a substituent(s)" is preferably 5- to 6-membered cyclic group, more preferably, tetrahydropyran, tetrahydrothiopyran, piperidine, piperazine, benzene, pyridine, pyrimidine, pyrazine, furan, oxazole, thiophene, pyrrole, thiazole, imidazole, particularly preferably, benzene.

In the present invention, $R^2$ is preferably C4-6 carbocyclic ring which may have a substituent(s) and C1-6 aliphatic hydrocarbon group which may have a substituent(s), more preferably, -(C1-6 alkyl which may have a substituent (s)), -(C2-6 alkenyl which may have a substituent(s)), -(C2-6 alkynyl which may have a substituent(s)), -(benzene which may have a substituent(s)), -(C4-6 cycloalkyl which may have a substituent(s)), -(C1-6 alkyl) -(C3-6 cycloalkyl which may have a substituent(s)), particularly preferably, -(C1-6 alkyl), -(C4-6 cycloalkyl), -(C1-6 alkyl) -(C3-6 cycloalkyl) . In particular, propyl group, cyclobutyl group, cyclopentyl group and cyclopropylmethyl group are preferable.

In the present invention, $R^3$ or $R^4$ is preferably, each hydrogen atom, C4-6 carbocyclic ring which may have a substituent(s) and C1-6 aliphatic hydrocarbon group which may have a substituent(s), more preferably hydrogen atom, -(C1-6 alkyl which may have a substituent(s)), -(benzene which may have a substituent(s)), -(C1-6 alkyl)-(C4-6 cycloalkyl which may have a substituent(s)), -(C1-6 alkyl)-(cyclic group which may have a substituent(s)), particularly preferably,

hydrogen atom, -(C1-6 alkyl), -(C1-4 alkyl) -(benzene which may have a substituent(s)), -(C1-4 alkyl) -(naphthalene which may have a substituent(s)), -(C1-4 alkyl) -(pyridine which may have a substituent(s)), -(C1-4 alkyl) -(indole which may have a substituent(s)). In particular, hydrogen atom, benzyl which may have a substituent(s), naphthylmethyl which may have a substituent(s), pyridylmethyl which may have a substituent(s) and indolylmethyl which may have a substituent (s) are preferable.

In the present invention, $R^5$ is preferably hydrogen atom, C4-6 carbocyclic ring which may have a substituent (s) and C1-6 aliphatic hydrocarbon group which may have a substituent(s), more preferably hydrogen atom, -(C1-6 alkyl which may have a substituent(s)), -(benzene which may have a substituent(s)), -(C1-6 alkyl)-(cyclic group which may have a substituent(s)), particularly preferably, hydrogen atom, -(C1-6 alkyl), -(benzyl which may have a substituent(s)), -(phenylethyl which may have a substituent(s)).

In the present invention, ring A is preferably C5-8 mono-carbocyclic ring which may have a substituent(s), and 5- to 8-membered heterocyclic ring which may have a substituent(s), more preferably 5- to 8-membered nitrogen-containing hetrocyclic ring which may have a substituent(s), particularly preferably 6-membered nitrogen-containing hetrocyclic ring which may have a substituent(s).

In the present invention, "C5-8 carbocyclic ring" represented by ring A is preferably C5-8 mono-carbocyclic aryl ring saturated with a part or all, specifically, for example,

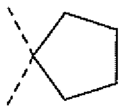 or 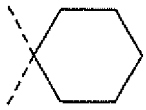

etc., are preferable.

In the present invention, "5- to 8-membered nitrogen-containing hetrocyclic ring" represented by ring A is preferably saturated a part or all among 5- to 8-membered mono-heterocyclic aryl ring including 1-2 nitrogen(s) atom, more 1-2 oxygen atom(s) and/or 1-2sulfur atom(s), specifically, for example,

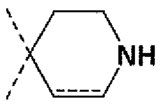 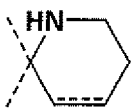, 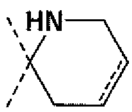, 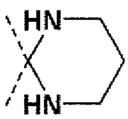, 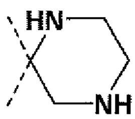 or 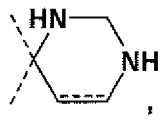,

[wherein

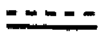

is a single bond or a double bond]
etc., are preferable.

In the present invention, "5- to 8-membered nitrogen-containing hetrocyclic ring which may have a substituent (s)" is preferably, imidazolidine, pyrazolidine, piperazine, piperidine, tetrahydropyridine, perhydropyrimidine and tetrahydropyrimidine which may have a substituent(s), and

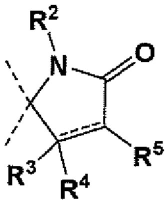, 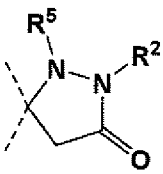, 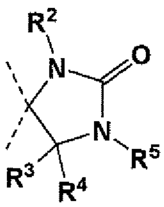, 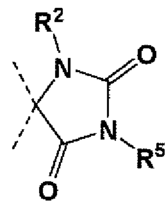, 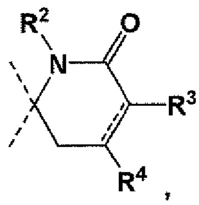,

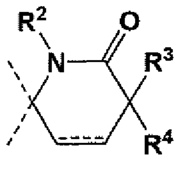,
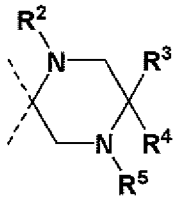,
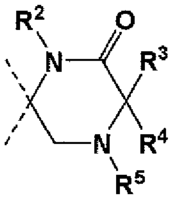,
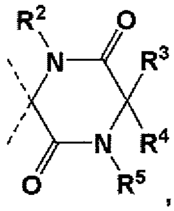,
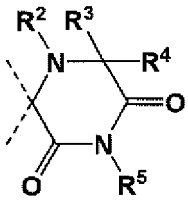,

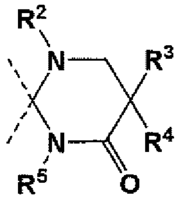,
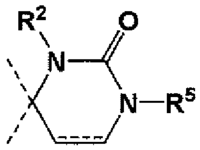
or
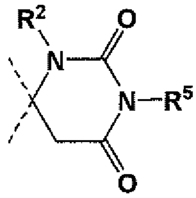

(wherein all symbols are as defined above)
is more preferably, and

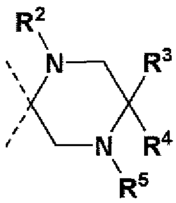,
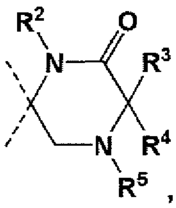,
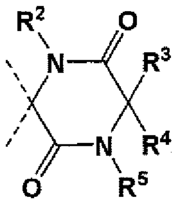,
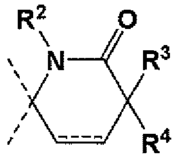

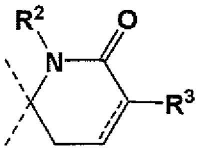
or
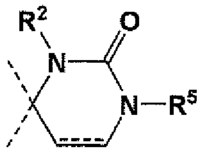

(wherein all symbols are as defined above)
is particularly preferably.

In the present invention, ring A which is not condensed to C3-8 mono-carbocyclic ring which may have a substituent(s) or 3- to 8-membered mono-heterocyclic ring which may have a substituent(s) is preferable.

In the present invention, "6-membered carbocyclic ring" represented by ring $A^{II}$ is preferably, specifically, for example,

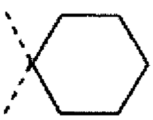

etc.

In the present invention, ring $A^{II}$ is preferably 6-membered heterocyclic ring which may have a substituent(s).

In the present invention, "6-membered nitrogen-containing hetrocyclic ring which may have a substituent(s)" represented by ring $A^{II}$ is preferably piperazine, piperidine, tetrahydropyridine, perhydropyrimidine and tetrahydropyrimidine, which may have a substituent(s), and specifically, for example,

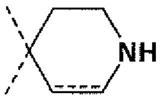 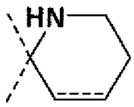, 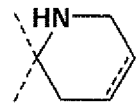, 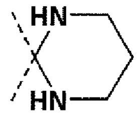, 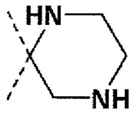 or 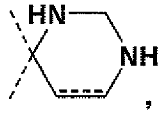,

etc., are preferable and more preferably

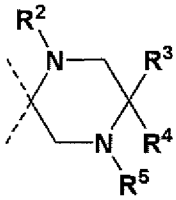, 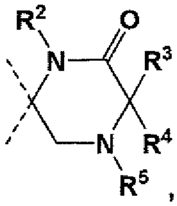, 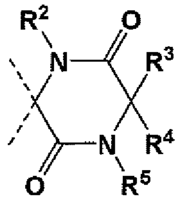, 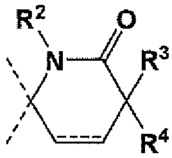

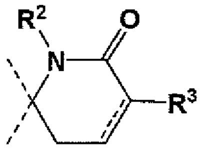 or 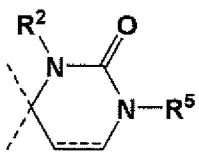

(wherein all symbols are as defined above).

In the present invention, 1 is preferable for n.

In the present invention, all compounds represented by formula(1) including a set enumerated as a preferable group and a preferable ring described herein before are preferable, the compounds represented by formula(II) including a set enumerated as, in particular, a preferable group and a preferable ring described here in before are preferable.

In the present invention, all compounds described for a example, a salt thereof, a quaternary ammonium salt thereof, an N-oxide form thereof or a solvate thereof, or a prodrug thereof are preferable. (3R)-3-benzyl-9-(4-chlorobenzyl)-1-propyl-1,4,9-triazaspiro[5.5]undecane-2,5- dione,
(3R)-3-benzyl-9-(4-chlorobenzyl)-1-(cyclopropylmethyl)-1,4,9-triazaspiro [5.5]undecane-2,5-dione,
(3R)-3-benzyl-9-(4-chlorobenzyl)-1-cyclobutyl-1,4,9-triazaspiro[5.5]undecane-2,5-dione,
(3R)-3-benzyl-9-(4-chlorobenzyl)-1-cyclopentyl-1,4,9-triazaspiro[5.5]undecane-2,5-dione,
(3S)-3-benzyl-9-(4-chlorobenzyl)-1-cyclobutyl-1,4,9-triazaspiro[5.5]undecane-2,5-dione,
(3R)-9-(4-chlorobenzyl)-1-propyl-3-(3-pyridinylmethyl)-1,4,9-triazaspiro[5.5] undecane-2,5-dione,
(3R)-9-(4-chlorobenzyl)-1-(cyclopropylmethyl)-3-(3-pyridinylmethyl)-1,4,9-triazaspiro[5.5] undecane-2,5-dione,
(3R)-9-(4-chlorobenzyl)-1-cyclobutyl-3-(3-pyridinylmethyl)-1,4,9-triazaspiro[5.5] undecane-2,5-dione,
(3R)-9-(4-chlorobenzyl)-1-cyclopentyl-3-(3-pyridinylmethyl)-1,4,9-triazaspiro[5.5]jundecane-2,5-dione,
(3R)-9-(4-chlorobenzyl)-3-(1H-indol-3-ylmethyl)-1-propyl-1,4,9-triazaspiro[5.5]undecane-2,5-dione,
(3R)-9-(4-chlorobenzyl)-1-(cyclopropylmethyl)-3-(1H-indol-3-ylmethyl)-1,4,9-triazaspiro[5.5]undecane-2,5-dione,
(3R)-9-(4-chlorobenzyl)-1-cyclobutyl-3-(1H-indol-3-ylmethyl)-1,4,9-triazaspiro [5.5]undecane-2,5-dione,
(3R)-9-(4-chiorobenzyl)-1-cyclopentyl-3-(1H-indol-3-ylmethyl)-1,4,9-triazaspiro[5.5]undecane-2,5-dione,
3-benzyl-9-(biphenyl-4-ylmethyl)-1-propyl-1,4,9-triazaspiro[5.5]undecane-2,5-dione,
9-[4-(allyloxy)benzyl]-3-benzyl-1-propyl-1,4,9-triazaspiro[5.5]undecane-2,5-dione,
3-[4-(prop-2-yn-1-yloxy)benzyl]-3-azaspira[5.5]undecane,
3-benzyl-9-(3-methylbenzyl)-1-propyl-1,4,9-triazaspiro[5.5]undecane-2,5-dione,
3-benzyl-1-(2-furylmethyl)-9-(3-methylbenzyl)-1,4,9-triazaspiro[5.5]undecane-2,5-dione,
3-benzyl-9-(4-methoxybenzyl)-1-propyl-1,4,9-triazaspiro[5.5]undecane-2,5-dione,
3-benzyl-9-(4-methoxybenzyl)-1-(3-phenylpropyl)-1,4,9-triazaspiro[5.5] undecane-2,5-dione,
3-benzyl-1-(2-furylmethyl)-9-(4-methoxylbenzyl)-1,4,9-triazaspiro[5.5]undecane-2,5-dione,
3-benzyl-9-(4-chlorobenzyl)-1-(2-furylmethyl)-1,4,9-triazaspiro[5.5]undecane-2,5-dione,
3-benzyl-9-(4-chlorobenzyl)-1-(2-methoxyethyl)-1,4,9-triazaspiro[5.5]undecane-2,5-dione,
3-benzyl-9-hexyl-1-(2-methoxyethyl)-1,4,9-triazaspiro[5.5]undecane-2,5-dione,
3-benzyl-1-(2-furylmethyl)-9-hexyl-1,4,9-triazaspiro[5.5]undecane-2,5-dione,
3-benzyl-9-hexyl-1-propyl-1,4,9-triazaspiro[5.5]undecane-2,5-dione,
(3R)-9-(4-chlorobenzyl)-3-(4-hydroxybenzyl)-1-propyl-1,4,9-triazaspiro[5.5] undecane-2,5-dione,

(3R)-9-(4-chlorobenzyl)-3-(1-naphthylmethyl)-1-propyl-1,4,9-triazaspiro[5.5] undecane-2,5-dione,
(3S)-3-(2-chlorobenzyl)-9-(4-chlorobenzyl)-1-propyl-1,4,9-triazaspiro[5.5] undecane-2,5-dione,
(3R)-9-(4-chlorobenzyl)-3-(4-methoxybenzyl)-1-propyl-1,4,9-triazaspiro[5.5] undecane-2,5-dione,
3-benzyl-9-[2-(4-chlorophenyl)ethyl]-1-propyl-1,4,9-triazaspiro[5.5]undecane-2,5-dione,
3-benzyl-9-[2-(4-chlorophenyl)ethyl]-1-(2-furylmethyl)-1,4,9-triazaspiro[5.5] undecane-2,5-dione,
3-benzyl-9-(4-chlorobenzyl)-1-cyclohexyl-1,4,9-triazaspiro[5.5]undecane-2, 5-dione,
3-benzy-9-(4-chlorobenzyl)-1-[2-(1H-imidazol-4-yl)ethyl]-1,4,9-triazaspiro[5.5] undecane-2,5-diane,
3-benzy-9-(4-chlorobenzyl)-1-(tetrahydro-2-furanylmethyl)-1,4,9-triazaspiro[5.5]undecane-2,5-dione,
3-benzy-9-(4-chlorobenzyl)-1-[2-(2-pyridinyl)ethyl]-1,4,9-triazaspiro[5.5] undecane-2,5-dione,
3-benzy-9-(4-chlorobenzyl)-1-(3-pyridinylmethyl)-1,4,9-triazaspiro[5.5]undecane-2,5-dione,
3-benzy-9-(4-chlorobenzyl)-1-(2-methoxy-1-methylethyl)-1,4,9-triazaspiro[5.5] undecane-2,5-dione,
3-benzy-1-sec-butyl-9-(4-chlorobenzyl)-1,4,9-triazaspiro[5.5]undecane-2,5-dione,
3-benzy-9-(4-chlorobenzyl)-1-(1-ethylpropyl)-1,4,9-triazaspiro[5.5]undecane-2,5-dione,
3-benzy-9-(4-chlorobenzyl)-1-(1-methylbutyl)-1,4,9-triazaspiro[5.5]undecane-2,5-dione,
3-benzy-9-(4-chlorobenzyl)-1-(2,2-dimethylpropyl)-1,4,9-triazaspiro[5.5] undecane-2,5-dione,
3-benzy-9-(4-chlorobenzyl)-1-(2-fluoroethyl)-1,4,9-triazaspiro[5.5]undecane-2,5-dione,
3-benzy-9-(4-chlorobenzyl)-1-(2-methoxyethyl)-1,4,9-triazaspiro[5.5]undecane-2,5-dione,
3-benzy-9-(4-chlorobenzyl)-1-(cyclopropylmethyl)-1,4,9-triazaspiro[5.5] undecane-2,5-dione,
3-benzy-9-(4-chlorobenzyl)-1-ethyl-1,4,9-triazaspiro[5.5]undecane-2,5-dione,
3-benzy-9-(4-chlorobenzyl)-1-[3-(methylthio)propyl]-1,4,9-triazaspiro[5.5] undecane-2,5-dione,
3-benzy-9-(4-chlorobenzyl)-1-isopropyl-1,4,9-triazaspiro[5.5]undecane-2,5-dione,
3-benzy-9-(4-chlorobenzyl)-1-(2-propyn-1-yl)-1,4, 9-triazaspiro[5.5]undecane-2,5-dione,
3-benzy-9-(4-chlorobenzyl)-1-(3-methoxypropyl)-1,4,9-triazaspiro[5.5]undecane-2,5-dione,
3-benzy-9-(4-chlorobenzyl)-1-(2-thienylmethyl)-1,4,9-triazaspiro[5.5]undecane-2,5-dione,
9-(4-chlorobenzyl)-3-(2-phenylethyl)-1-propyl-1,3,9-triazaspiro[5.5]undec-4- en-2-one,
3-benzyl-9-(4-chlorobenzyl)-1-(cyclopropylmethyl)-1,3,9-triazaspiro[5.5]undec-4-en-2-one,
3-benzyl-9-(4-chlorobenzyl)-1-propyl-1,3,9-triazaspiro[5.5]undec-4-en-2-one,
9-(4-chlorobenzyl)-1-(cyclopropylmethyl)-3-(2-phenylethyl)-1,3,9-triazaspiro[5.5]undec-4-en-2-one,
9-(4-chlorobenzyl)-1-cyclobutyl-3-(2-phenylethyl)-1,3,9-triazaspiro[5.5]undec-4-en-2-one,
3-butyl-9-(4-chlorobenzyl)-1-propyl-1,3,9-triazaspiro[5.5]undec-4-en-2-one,
9-(4-chlorobenzyl)-3-isopropyl-1-propyl-1,3,9-triazaspiro[5.5]undec-4-en-2-one,
9-(4-chlorobenzyl)-1-(cyclopropylmethyl)-3-isopropyl-1,3,9-triazaspiro[5.5] undec-4-en-2-one,
3-butyl-9-(4-chlorobenzyl)-1-(cyclopropylmethyl)-1,3,9-triazaspiro[5.5]undec-4-en-2-one,
3-butyl-9-(4-chlorobenzyl)-1-cyclopentyl-1,3,9-triazaspiro[5.5]undecan-2-one,
3-benzyl-9-(4-chlorobenzyl)-1-(cyclopropylmethyl)-1,3,9-triazaspiro[5.5] undecan-2-one,
3-benzyl-9-(4-chlorobenzyl)-1-(cyclopropylmethyl)-1,3,9-triazaspiro[5.5] undecan-2-one,
9-(4-chlorobenzyl)-3-(2-phenylethyl)-1-propyl-1,3,9-triazaspiro[5.5]undecan-2-one,
9-(4-chlorobenzyl)-1-cyclobutyl-3-(2-phenylethyl)-1,3,9-triazaspiro[5.5]undecan-2-one,
9-(4-chlorobenzyl)-1-cyclopentyl-3-(2-phenylethyl)-1,3,9-triazaspiro[5.5] undecan-2-one,
9-(4-chlorobenzyl)-3-[(1R)-1-phenylethyl]-1-propyl-1,3,9-triazaspiro[5.5] undecan-2-one,
9-(4-chlorobenzyl)-3-[(1 S)-1-phenylethyl]-1-propyl-1 ,3, 9-triazaspiro[5.5] undecan-2-one,
3-butyl-9-(4-chiorobenzyl)-1-(cyclopropylmethyl)-1,3,9-triazaspiro[5.5]undecan-2-one,
9-(4-chlorobenzyl)-1-(cyclopropylmethyl)-3-[(1S)-1-phenylethyl]-1,3,9-triazaspiro[5.5]undecan-2-one,
3-butyl-9-(4-chlorobenzyl)-1-cyclobutyl-1,3,9-triazaspiro[5.5]undecan-2-one,
9-(4-chlorobenzyl)-3-isopropyl-1-propyl-1,3,9-triazaspiro[5.5]undecan-2-one,
3-benzyl-9-(4-chlorobenzyl)-1-(cyclopropylmethyl)-1,9-diazaspiro[5.5]undecan-2-one, or
3-benzyl-9-(4-chlorobenzyl)-1-propyl-1,9-diazaspiro[5.5]undecan-2-one,
a salt thereof, a quaternary ammonium salt thereof, an N-oxide form thereof or a solvate thereof, are given, concretely, more preferably,
(3R)-3-benzyl-9-(4-chlorobenzyl)-1-propyl-1,4,9-triazaspiro[5.5]undecane-2,5-dione,
(3R)-3-benzyl-9-(4-chlorobenzyl)-1-(cyclopropylmethyl)-1,4,9-triazaspiro[5.5] undecane-2,5-dione,
(3R)-3-benzyl-9-(4-chlorobenzyl)-1-cyclobutyl-1,4,9-triazaspiro[5.5]undecane-2,5-dione,
(3R)-3-benzyl-9-(4-chlorobenzyl)-1-cyclopentyl-1,4,9-triazaspiro[5.5]undecane-2,5-dione,
(3S)-3-benzyl-9-(4-chlorobenzyl)-1-cyclobutyl-1,4,9-triazaspiro[5.5]undecane-2,5-dione,
(3R)-9-(4-chlorobenzyl)-1-propy-3-(3-pyridinylmethyl)-1,4,9-triazaspiro[5.5] undecane-2,5-dione,
(3R)-9-(4-chlorobenzyl)-1-(cyclopropylmethyl)-3-(3-pyridinylmethyl)-1,4,9-triazaspiro[5.5]undecane-2,5-dione,
(3R)-9-(4-chlorobenzyl)-1-cyclobutyl-3-(3-pyridinylmethyl)-1,4,9-triazaspiro[5.5] undecane-2,5-dione,
(3R)-9-(4-chlorobenzyl)-1-cyclopentyl-3-(3-pyridinylmethyl)-1,4,9-triazaspiro[5.5]undecane-2,5-dione,

(3R)-9-(4-chlorobenzyl)-3-(1H-indol-3-ylmethyl)-1-propyl-1,4,9-triazaspiro[5.5] undecane-2,5-dione,
(3R)-9-(4-chlorobenzyl)-1-(cyclopropylmethyl)-3-(1H-indol-3-ylmethyl)-1,4,9-triazaspiro[5.5]undecane-2,5-diane,
(3R)-9-(4-chlorobenzyl)-1-cyclobutyl-3-(1H-indol-3-ylmethyl)-1,4,9-triazaspiro [5.5]undecane-2,5-dione,
(3R)-9-(4-chlorobenzyl)-1-cyclopentyl-3-(1H-indol-3-ylmethyl)-1,4,9-triazaspiro [5.5]undecane-2,5-dione,
3-benzyl-9-(biphenyl-4-ylmethyl)-1-propyl-1,4,9-triazaspiro[5.5]undecane-2,5-dione,
9-[4-(allyloxy)benzyl]-3-benzyl-1-propyl-1,4,9-triazaspiro[5.5]undecane-2,5-dione,
3-[4-(prop-2-yn-1-yloxy)benzyl]-3-azaspiro[5.5]undecane,
9-(4-chlorobenzyl)-3-(2-phenylethyl)-1-propyl-1,3,9-triazaspiro[5.5]undec-4- en-2-one,
3-benzyl-9-(4-chlorobenzyl)-1-(cyclopropylmethyl)-1,3,9-triazaspiro[5.5]undec-4-en-2-one,
3-benzyl-9-(4-chlorobenzyl)-1-propyl-1,3,9-triazaspiro[5.5]undec-4-en-2-one,
9-(4-chlorobenzyl)-1-(cyclopropylmethyl)-3-(2-phenylethyl)-1,3,9-triazaspiro[5.5]undec-4-en-2-one,
9-(4-chlorobenzyl)-1-cyclobutyl-3-(2-phenylethyl)-1,3,9-triazaspiro[5.5]undec- 4-en-2-one,
3-butyl-9-(4-chlorobenzyl)-1-propyl-1,3,9-triazaspiro[5.5]undec-4-en-2-one,
3-butyl-9-(4-chlorobenzyl)-1-(cyclopropylmethyl)-1,3,9-triazaspiro[5.5]undec-4-en-2-one,
3-butyl-9-(4-chlorobenzyl)-1-cyclopentyl-1,3,9-triazaspiro[5.5]undec-4-en-2-one,
9-(4-chlorobenzyl)-3-(2-phenylethyl)-1-propyl-1,3,9-triazaspiro[5.5]undecan-2-one,
9-(4-chlorobenzyl)-1-cyclobutyl-3-(2-phenylethyl)-1,3,9-triazaspiro[5.5]undecan-2-one,
9-(4-chlorobenzyl)-1-cyclopentyl-3-(2-phenylethyl)-1,3,9-triazaspiro[5.5] undeca-2-one,
3-butyl-9-(4-chlorobenzyl)-1-(cyclopropylmethyl)-1,3,9-triazaspiro[5.5]undecan-2-one,
3-butyl-9-(4-chlorobenzyl)-1-cyclobutyl-1,3,9-triazaspiro[5.5]undecan-2-one,
3-benzyl-9-(4-chlorobenzyl)-1-(cyclopropylmethyl)-1,9-diazaspiro[5.5]undecan-2-one, or
3-benzyl-9-(4-chlorobenzyl)-1-propyl-1,9-diazaspiro[5.5]undecan-2-one,
a salt thereof, a quaternary ammonium salt thereof, an N-oxide form thereof, a solvate thereof, or a prodrug thereof, are given specifically preferably among their compounds.

It is shown in the following about the denomination of the compound in the present invention.

All the compounds described in the present description were named using computer program which names on the basis of IUPAC, using ACD/Name Batch (registered trademark, a product made in Advanced Chemistry Development Inc.), or according to IUPAC nomenclature system. A translation to a Japanese name was done in accordance with "kagoubutsu meimeihou" (Editing of Japanese chemical institute). For example, the compound represented by

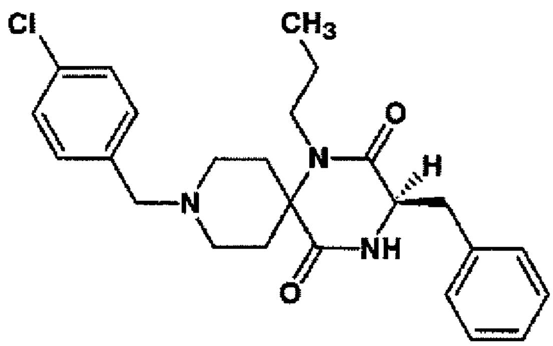

was named (3R)-3-benzyl-9-(4-chlorobenzyl)-1-propyl-1,4,9-triazaspiro[5.5]undecane-2,5-dione.

In the present invention, unless otherwise specified, as is apparent to those skilled in the art, the symbol

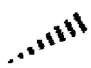

represents that it is bonded to α configuration, the symbol

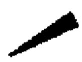

represents that it is bonded to β configuration, the symbol

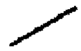

represents α configuration, β configuration or the mixture of them.

[Isomers]

In the present invention, all isomers are included unless otherwise specified. For example, alkyl group, alkenyl group, alkynyl group, alkoxy group, alkylthio group, alkylene group, alkenylene group, alkynylene group and the like include those which are linear and branched. Furthermore, all of isomers (E-, Z-, cis-, and trans-isomers) on a double bond, ring and condensed ring, isomers (R-isomer, S-isomer, α,β configuration, enantiomer, and diastereomer) due to the presence of asymmetric carbon etc., optically active substances with optical rotation (D-, L-, d-, and I-compounds), polar compounds (a more polar compound and a less polar compound) generated by a chromatographic separation, equilibrium compounds, rotational isomers, mixtures in an optional mixing ratio and racemic mixtures are included in the present invention.
Furthermore, in the present invention, all isomers by tautomerism are included.

[Salts and solvates]

In the present invention, salts of the compound represented by formula (I) include all of nontoxic salts and pharmaceutically acceptable salts and the like. The pharmaceutically acceptable salt is preferably a water soluble salt which shows low toxicity. Examples of the suitable salt of the compound represented by formula (I) include salts of alkali metal (potassium, sodium, lithium, etc.), salts of alkaline earth metal (calcium, magnesium, etc.), ammonium salts (tetramethylammonium salt, tetrabutylammonium salt, etc.), salts of organic amine (triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, etc.), acid addition salts [inorganic acid salts (hydrochloride, hydrobromide, hydroiodide, sulfate, phosphate, nitrate, etc.), and organic acid salts (acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, etc.)] and the like. Examples of suitable solvate of the compound represented by formula (I) include solvates such as hydrate, alcoholate (for example, ethanolate, etc.) and the like. The solvate is preferably low toxic and water soluble. The solvate of the compound of the present invention also includes solvates of alkali metal, alkaline earth metal salts, ammonium salts, salts of organic amine, and acid addition salts of the compound of the present invention. The compound of the present invention can be converted into the nontoxic salt and the pharmaceutically acceptable salts by a known method.
Furthermore, salts include quaternary ammonium salts. The quaternary ammonium salt represented by formula (I) is obtained by quaternizing nitrogen atom of the compound represented by formula (I) with $R^0$ group ($R^0$ group represents C1-8 alkyl group, or C1-8 alkyl group substituted with phenyl group).
Also, salts include N-oxide form. The compound of the present invention can be converted into N-oxide form by an optional method. N-oxide form of the compound represented by formula (I) is obtained by oxidizing nitrogen atom of the compound represented by formula (I).

[Prodrugs]

In the present invention, the prodrug of the compound represented by the formula (I) means the compound which is converted into the compound (I) by an enzyme and gastric acid etc., in the body. The prodrugs of the compound represented by the formula (I) are included, when the compound represented by the formula (I) possesses an amino group, the amino group is acylated, alkylated, phosphorylated (e.g., the amino group of the compound represented by the formula (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, t-butylated, etc.); when the compound represented by the formula (I) possesses a hydroxy group, the hydroxy group is acylated, alkylated, phosphorylated, borated (e.g., the hydroxy group of the compound represented by the formula (I) is acetylated, palmitoylated, propanoylated, pivaloylated, succinylated, fumarylated, alanylated, dimethylaminomethylcarbonylated, *etc.*); when the compound represented by the formula (I) possesses a carboxy group, the carboxy group is esterified, or amidated (*e.g.*, the carboxy group of the compound represented by the formula (I) is converted into ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,8-dioxolen-4-yl)methyl ester, cyclohexyloxycarbonylethyl ester, methyl amide, *etc.*), *etc.* These compounds may be prepared by known methods. The prodrug of the compound (I) may be a solvate. Also, the

prodrugs of the compound represented by the formula (I) may be converted into the compounds represented by the formula (I) under such physiological conditions as described in "Bunshisekkei" pages 163-198, in the "Iyakuhin no Kaihatsu" Vol.7, 1990, Hirokawa Shoten. Furthermore, the compound represented by the formula (I) may be labeled by isotope (*e.g.*, $^{3}H$, $^{14}C$ $^{35}S$ $^{125}I$ , *etc.*).

The compound represented by the formula (I) used for CXCR3 antagonist of the present invention, a salt thereof, a solvate thereof or a prodrug thereof is superior to solubility and oral absorption, and, besides, the inhibition of drug metabolizing enzyme is weak, and toxicity is low. It is important that a compound which is developed as agent has this character. A required property is physical, chemical, pharmacological aspect. As a result, these compounds have probability of very superior agent. [(The Merck Manual of Diagnosis and Therapy (17th Ed.), Merck & Co.) see]

[Method for Producing Compound of the Present Invention]

The compound of the present invention represented by formula (I) can be prepared by using improved methods in combination a known method, for example, methods shown below, methods described in examples, and a method described in Comprehensive Organic Transformations: A Guide to Functional Group Preparations, Second edition (written by Richard C. Larock, John Wiley & Sons Inc, 1999). In the following manufacturing methods, a starting compound may be used in the form of a salt. As the salt, for example, those described as a salt of the above described formula (1) are used.

The compound of the present invention represented by formula (1) can be prepared by same methods described in WO01/40227, WO02/74770 and WO2004/92169, a known method, or the method that was improved appropriately. In the following manufacturing methods, a starting compound may be used in the form of a salt. As the salt, those described as a salt of the above described compound of the present invention represented by formula (I) are used.

The compound of the present invention represented by formula (II) can be prepared by using improved methods in combination a known method, for example, methods shown below, methods followed these, or methods described in examples, or a method described in Comprehensive Organic Transformations: A Guide to Functional Group Preparations, Second edition (written by Richard C. Larock, John Wiley & Sons Inc, 1999) and the like.

The compound represented by formula (II)

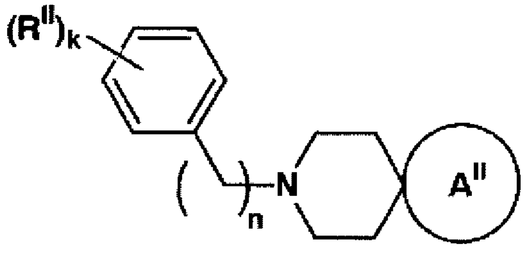

(II)

(wherein all symbols are as defined above)
can be produced, for example, by the using a compound wherein $R^1$ is hydrogen atom, ring A is represented by 6-membered mono-carbocyclic group which may have a substituent(s) or 6-membered mono-heterocyclic group which may have a substituent(s), among the compound of the present invention represented by formula (I), i.e. the compound represented by formula (1-2),

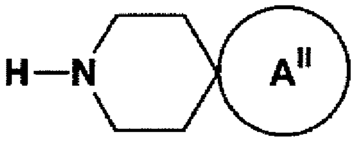

(I-2)

(wherein all symbols are as defined above)
by means of a method as described in the following reaction scheme. In addition, it can be produced by a method described in WO02/74770 and WO2004/92169, a known method or a method that was improved appropriately as well as this reaction.

## Reaction Scheme

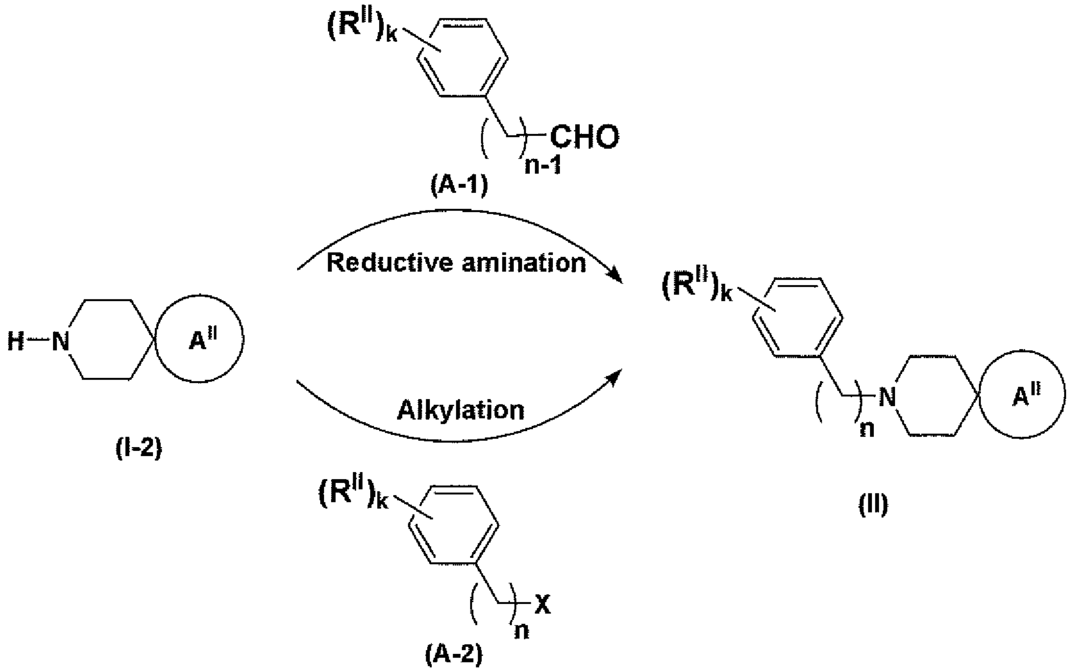


(A-1) (I-2) (II) (A-2)

In a reaction scheme, X is a leaving group (such as chlorine atom, bromine atom, iodine atom, p-toluenesulfonyloxy group, methanesulphonyloxy group, trifluoromethanesulfonyloxy group and the group represented by formula :

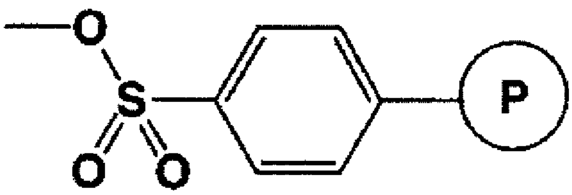

(wherein,
Ⓟ
is polystyrene resin (such as 1~10% divinylbenzene copolymer))), the other symbols are as defined above.

In a reaction scheme, the reductive amination reaction is known and is carried out, for example, in an organic solvent (*e.g.*, dichloroethane, dichloromethane, N, N-dimethyl formamide, acetic acid, a mixture thereof, *etc.*) about 0 to 40°C in the presence of a reducing agent (e.g., sodium borohydride, sodium triacetoxyborohydride, sodium cyanoborohydride, etc.).

Furthermore, in a reaction scheme, the alkylation reaction is known and is carried out, for example, by subjecting an amine to a reaction with the compound having leaving group in an organic solvent (for example, aromatic hydrocarbon such as benzene, toluene and xylene, etc., for example, halogenated hydrocarbon such as dichloromethane and chloroform, etc., for example, saturated hydrocarbon such as hexane, heptane and cyclohexane, etc., for example, ether such as diethyl ether, tetrahydrofuran and 1,4-dioxane, etc., for example, ketone such as acetone and methyl ethyl ketone, etc., for example, nitrile such as acetonitrile, etc., for example, sulfoxide such as dimethyl sulfoxide, etc., for example, acid amide such as N, N-dimethylformamide, etc., for example, ester such as ethyl acetate, etc., is used. These solvents can be used alone, and if necessary, mixed at the suitable rate, for example, at ratio of about 1:1 ~ 1: 10, of two or more, and it may be used) in the presence of a base (for example, hydride of alkali metal such as sodium hydride and potassium hydride or hydride of alkaline-earth metals, for example, alkyllithium such as butyllithium, sec-butyllithium and tert-butyllithium, for example, alkoxide of alkali metal such as sodium methoxide and sodium ethoxide, for example, inorganic base such as alkali metal of metallic sodium and metallic potassium, etc., for example, alkyl amine

such as triethylamine, tributylamine and diisopropyl ethylamine, for example, aromatic amine such as N, N- dimethylaniline, pyridine, lutidine, collidine and 4-(dimethylamino)pyridine, organic base such as DBU (1,8-diazabicyclo[5,4,0] undecene-7), for example, metallic amide such as lithium diisopropylamide, lithium bis(trimethylsilyl)amide, potassium bis(trimethylsilyl)amide and sodium bis(trimethylsilyl)amide) at a temperature of about -78 to 100°C.

Furthermore, the compound of the present invention represented by formula (1-2) can be prepared by same methods described in WO01/40227, WO02/74770 and WO2004/92169, a known method, or a method that was improved appropriately.

Among the compound represented by formula (II-B), the compound wherein $R^5$ is hydrogen atom or aliphatic hydrocarbon group which may have a substituent(s), i.e. the compound of the present invention represented by formula (II-B-1):

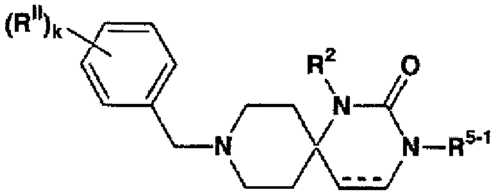

**(II-B-1)**

(wherein $R^{5-1}$ is hydrogen atom or aliphatic hydrocarbon group which may have a substituent(s), the other symbols are as defined above)
can be produced by the reaction of a cyclization with the compound represented by formula (II-B-2):

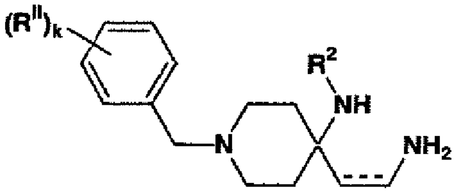

**(II-B-2)**

(wherein all symbols are as defined above),
further by the reaction with the compound represented by formula (II-B-3)

**R5-2-X1** **(II-B-3)**

(wherein $R^{5-2}$ is aliphatic hydrocarbon group which may have a substituent(s), and $X^1$ is a leaving group (*e.g.*, chlorine atom, bromine atom, iodine atom, p-toluenesulfonyloxy group, methanesulphonyloxy group, trifluoromethanesulfonyloxy group, *etc.*)),
or by the reaction of a cyclization with the compound represented by formula (II-B-4)

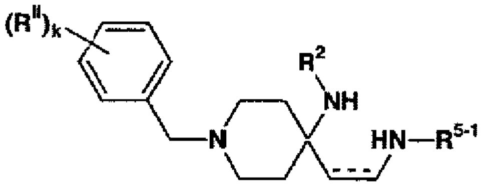

**(II-B-4)**

(wherein all symbols are as defined above), if necessary.

This cyclization reaction is known and the cyclic compound can be produced by the reaction with, for example, phosgene compound (phosgene and triphosgene(bis(trichloromethyl)carbonate), etc.) and imidazole compound (for example, CDI (carbonyldiimidazole), etc.), etc., for example, in an organic solvent (for example, ethyl acetate, chloroform, dichloromethane, diethyl ether, tetrahydrofuran, benzene, toluene, etc.) or without a solvent, at a temperature of about -20°C to a refluxing temperature, in the presence of a base (for example, pyridine, triethylamine, dimethylaniline, dimethylaminopyridine, diisopropyl ethylamine, etc.).

The reaction with the cyclization product of the compound represented by formula (II-B-2) and the compound represented by formula (II-B-3) is well known, it is for a person of ordinary skill in the art to be able to understand the reaction easily. By the using different reaction by means of each $R^{5-2}$ group, the directed compound of the present invention can be produced easily. For example, it can be produced by,

1) An alkylation reaction when the carbonyl group does not represent the carbon atom which is next to $X^1$ of $R^{5-2}$,
2) An amidation reaction when the carbonyl group represents by the carbon atom which is next to $X^1$ of $R^{5-2}$ , etc.

In addition, as well as described above, for example, by means of a method described in Comprehensive Organic Transformations: A Guide to Functional Group Preparations, Second edition (written by Richard C. Larock, John Wiley & Sons Inc, 1999), by the bond-formation reaction of nitrogen atom and $R^{5-2}$ group, the compound of the present invention represented by formula (II-B-1) can be produced.

1) The alkylation reaction is known and is carried out, for example, by subjecting an amine to a reaction with the compound having leaving group in an organic solvent (for example, aromatic hydrocarbon such as benzene, toluene and xylene, etc., for example, halogenated hydrocarbon such as dichloromethane and chloroform, etc., for example, saturated hydrocarbon such as hexane, heptane and cyclohexane, etc., for example, ether such as diethyl ether, tetrahydrofuran and 1,4-dioxane, etc., for example, ketone such as acetone and methyl ethyl ketone, etc., for example, nitrile such as acetonitrile, etc., for example, sulfoxide such as dimethyl sulfoxide, etc., for example, acid amide such as N, N- dimethylformamide, etc., for example, ester such as ethyl acetate, etc., is used. These solvents can be used alone, and if necessary, mixed at the suitable rate, for example, at ratio of about 1:1 ~ 1:10, of two or more, and it may be used) in the presence of a base (for example, hydride of alkali metal such as sodium hydride and potassium hydride or hydride of alkaline-earth metals, for example, alkyllithium such as butyllithium, sec-butyllithium and tert- butyllithium, for example, alkoxide of alkali metal such as sodium methoxide and sodium ethoxide, for example, inorganic base such as alkali metal of metallic sodium and metallic potassium, for example, alkyl amine such as triethylamine, tributylamine and diisopropyl ethylamine, for example, aromatic amine such as N,N-dimethylaniline, pyridine, lutidine, collidine and 4-(dimethylamino)pyridine, organic base such as DBU (1,8-diazabicyclo [5,4,0]undecene-7), for example, metallic amide such as lithium diisopropylamide, lithium bis(trimethylsilyl)amide, potassium bis(trimethylsilyl)amide and sodium bis(trimethylsilyl)amide) at a temperature of about -78 to 100°C.
2) The amidation reaction is known and examples thereof include:

(1) a method using an acyl halide,
(2) a method using a mixed acid anhydride, and
(3) a method using a condensing agent.

These methods are described in detail below.

(1) The method using an acyl halide is carried out, for example, by reacting carboxylic acid with an acid halogenating agent (for example, oxalyl chloride, thionyl chloride, phosphorus pentachloride and phosphorus trichloride, etc.) in an organic solvent (for example, halogenated hydrocarbon such as chloroform and dichloromethane, etc., for example, ether such as diethyl ether, tetrahydrofuran and dioxane, etc., for example, acid amide such as dimethylformamide, etc., is used. These solvents can be used alone, and if necessary, mixed at the suitable rate, for example, at ratio of about 1:1 ~ 1:10, of two or more, and it may be used) or in the absence of the solvent at a temperature of about -20°C to a refluxing temperature. Then the obtained acyl halide derivative may be reacted with amine in the presence of a base (for example, alkyl amine such as triethylamine, tributylamine and diisopropyl ethylamine, for example, aromatic amine such as N, N- dimethylaniline, pyridine and 4-(dimethylamino)pyridine) about 0 to 40°C. Alternatively, the obtained acyl halide can be reacted with amine in an organic solvent (for example, diethyl ether, dioxane, tetrahydrofuran, etc., is used. These solvents can be used alone, and if necessary, mixed at the suitable rate, for example, at ratio of about 1:1 ~ 1:10, of two or more, and it may be used) about 0 to 40°C using an aqueous alkali solution (sodium bicarbonate solution or sodium hydroxide solution, etc.).
(2) The method using a mixed acid anhydride is carried out, for example, by reacting carboxylic acid with an acyl halide (for example, pivaloyl chloride, tosyl chloride, mesyl chloride, etc.) or an acid derivative (for example, ethyl chloroformate, isobutyl chloroformate, etc.) in the presence of a base (for example, pyridine, triethylamine, dimethylaniline, dimethylaminopyridine, diisopropylethylamine, etc.) in an organic solvent (forexample, halogenated hydrocarbon such as chloroform and dichloromethane, etc., for example, ether such as diethyl ether, tetrahydrofuran and dioxane, etc., for example, acid amide such as dimethylformamide, etc., is used. These solvents can be used alone, and if necessary, mixed at the suitable rate, for example, at ratio of about 1:1 ~ 1:10, of two or more, and it may be used) or in the absence of the solvent about 0 to 40°C, and reacting the resulting mixedacid anhydride with

amine in an organic solvent (for example, halogenatedhydrocarbon such as chloroform and dichloromethane, etc., for example, ether such as diethyl ether, tetrahydrofuran and dioxane, etc., for example,acid amide such as dimethylformamide, etc., is used. These solvents can be used alone, and if necessary, mixed at the suitable rate, for example, at ratio of about 1:1 ~ 1:10, of two or more, and it may be used) about 0 to 40°C.

(3) The method using a condensing agent is carried out, for example, by reacting carboxylic acid with amine in an organic solvent (for example, halogenated hydrocarbon such as chloroform and dichloromethane, etc., for example, ether such as diethyl ether, tetrahydrofuran and dioxane, etc., for example, acid amide such as dimethylformamide, etc., is used. These solvents can be used alone, and if necessary, mixed at the suitable rate, for example, at ratio of about 1:1 ~ 1:10, of two or more, and it may be used) or in the absence of the solvent about 0 to 40°C in the presence or absence of a base (for example, alkyl amine such as triethylamine, tributylamine and diisopropyl ethylamine, for example, aromatic amine such as N, N- dimethylaniline, pyridine and 4-(dimethylamino)pyridine), using a condensing agent (for example, 1,3-dicyclohexylcarbodiimide (DCC), 1-ethyl-3-[3-(dimethylamino)propyl]carbodiimide (EDC), 1,1'-carbonyldiimidazole (CDI), 2-chloro-1-methylpyridiniumiodine, 1-propylphosphonic acid cyclic anhydride (PPA), etc.) and using, or not using, 1-hydroxybenztriazole (HOBt).

These reactions described in (1), (2) and (3) are preferably carried out under an inert gas (argon, nitrogen, etc.) atmosphere on anhydrous condition.

Futhermore, among the compound represented by formula (II-B), the compound wherein

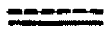

represents a double bond, i.e. the compound of the present invention represented by formula (II-B-5):

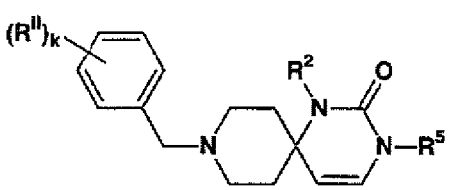

(II-B-5)

(wherein all symbols are as defined above)
can be produced by the reaction of a cyclization with the compound represented by formula (II-B-6):

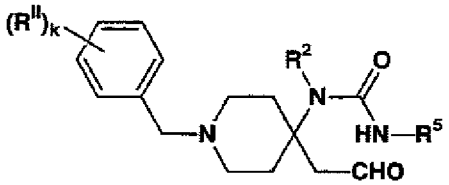

(II-B-6)

(wherein all symbols are as defined above).

This cyclization reaction is known and the cyclic compound can be produced by the reacting, for example, in an organic solvent (for example, ethyl acetate, chloroform, dichloromethane, diethyl ether, tetrahydrofuran, benzene and toluene, etc.) or without a solvent, at a temperature of about -20°C to a refluxing temperature, in the presence of an acid (for example, trifluoroacetic acid, boron trifluoride-diethyl etherate and sulfuric acid, etc.).

Futhermore, among the compound represented by formula (II-B), the compound wherein

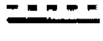

represents a single bond, i.e. the compound of the present invention represented by formula (II-B-7):

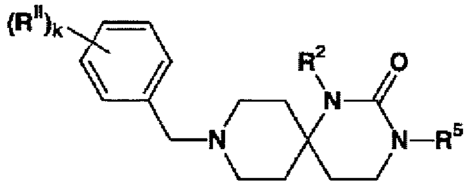

(II-B-7)

[wherein all symbols are as defined above]
can be produced by the reaction of a cyclization with the compound represented by formula (II-B-6) described above.

This cyclization reaction is known and the cyclic compound can be produced by the reacting, for example, in an organic solvent (for example, ethyl acetate, chloroform, dichloromethane, diethyl ether, tetrahydrofuran, benzene and toluene, etc.) or without a solvent, at a temperature of about -20°C to a refluxing temperature, in the presence of an acid (for example, trifluoroacetic acid, boron trifluoride-diethyl etherate and sulfuric acid, etc.) and a silicon compound (for example, triethylsilane and triisopropyl silane, etc.).

Among the compound of the present invention represented by formula (II-C), the compound wherein

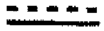

represents a double bond, i.e. the compound represented by formula (II-C-1):

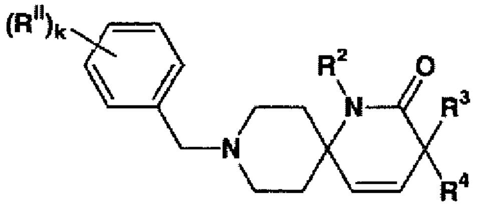

(II-C-1)

(wherein all symbols are as defined above)

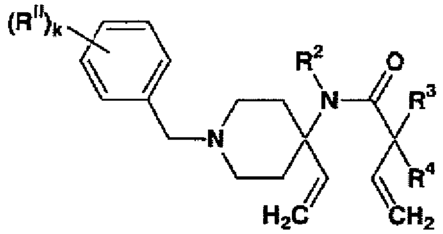

(II-C-2)

(wherein all symbols are as defined above)
can be produced by the reaction of a cyclization (metathesis) of the compound represented by formula (II-C-2).

This cyclization reaction is known and the cyclic compound can be produced by the reacting, for example, in an organic solvent (for example, dichloromethane, toluene and a mixture thereof, etc.), at room temperature to 100°C, in the presence of Grubbs' catalyst (for example,
benzylidene[1,3-bis(mesitylene)-2-imidazolidinylidene]tricyclohexylphosphine ruthenium (IV) dichloride, etc.).

Among the compound of the present invention represented by formula (II-C), the compound wherein

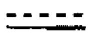

represents a single bond, i.e. the compound represented by formula (II-C-3)

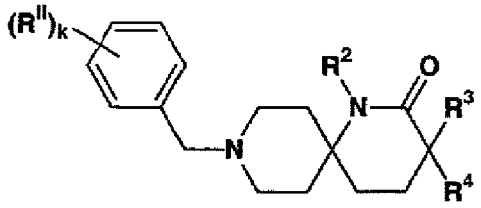

(II-C-3)

(wherein all symbols are as defined above)
can be produced by means of that the compound represented by formula (II-C-1) described above is carried out by a reductive reaction of double bond.

This reductive reaction is known and the reduced compound can be produced by the reacting, for example, in a solvent (tetrahydrofuran, dioxane, dimethoxyethane, diethyl ether, methanol, ethanol, benzene, toluene, acetone, methyl ethyl ketone, acetonitrile, dimethylformamide, water, ethyl acetate, acetic acid or a mixture of two or more thereof, etc.) in the presence of a metal catalyst (palladium-carbon, palladium black, palladium, palladium hydroxide, platinum oxide, platinum-carbon, nickel, Raney nickel, ruthenium chloride, etc.), in the presence or absence of an acid (hydrochloric acid, sulfuric acid, hypochlorite, boric acid, tetrafluoroboric acid, acetic acid, p- toluenesulfonic acid, oxalic acid, trifluoroacetic acid, formic acid, etc.), under the atmosphere of hydrogen of normal or suppressed pressure, in the presence of ammonium formate or hydrazine at a temperature of about 0 to 200°C.

Among the compound of the present invention represented by formula (II-D), the compound wherein

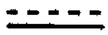

represents a double bond, i.e. the compound represented by formula (II-D-1):

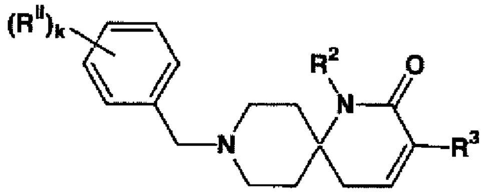

(II-D-1)

(wherein all symbols are as defined above)
can be produced by the reaction of a cyclization (metathesis) of the compound represented by formula (II-D-2):

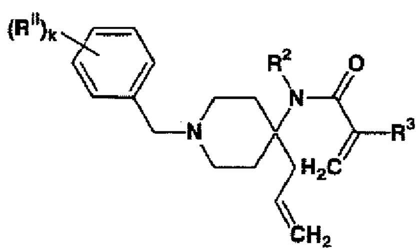

(II-D-2)

(wherein all symbols are as defined above).

This reductive reaction is known and is carried out, for example, by a same method as a using method when the compound represented by formula (II-C-1) is produced from the compound represented by formula (II-C-2) described above.

Among the compound of the present invention represented by formula (II-D), the compound wherein

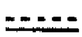

represents a single bond, i.e. the compound represented by formula (II-D-3)

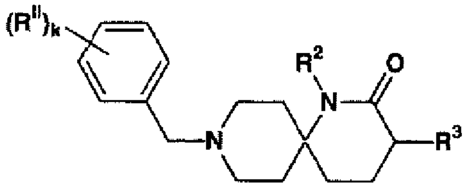

(II-D-3)

(wherein all symbols are as defined above)
can be produced by means of that the compound represented by formula (II-D-1) described above is carried out by a reductive reaction of double bond.

This reductive reaction is known and is carried out, for example, in a solvent (tetrahydrofuran, dioxane, diethyl ether, etc.), in the presence of a base (L-Selectride, etc.) at a temperature of about -78 to 100°C.

Among the compound of represented by formula (II-D), the compound wherein $R^3$ is aliphatic hydrocarbon group which may have a substituent(s), and

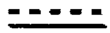

represents a single bond, i.e. the compound represented by formula (II-D-4):

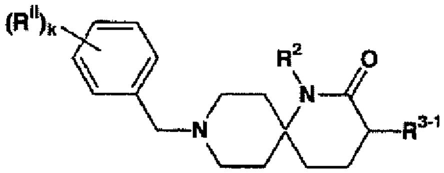

(II-D-4)

(wherein $R^{3-1}$ is aliphatic hydrocarbon group which may have a substituent(s), the other symbols are as defined above) can be produced by C-alkylation reaction of the compound represented by formula (II-D-6):

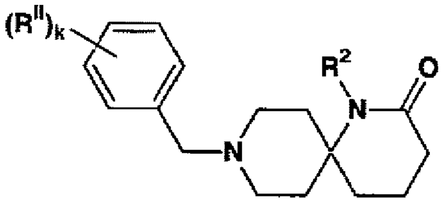

(II-D-6)

(wherein all symbols are as defined above)
which can be produced by means of that the compound represented by formula (II-D-5):

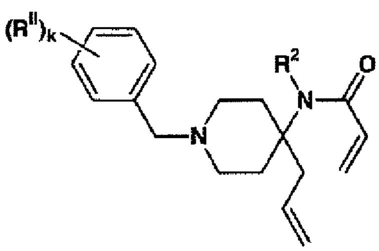

(II-D-5)

(wherein all symbols are as defined above)
is carried out by the same cyclization reaction described above and furthermore the same reductive reaction of double bond described above, with the compound represented by formula (II-D-7):

**R3-1-X1 (II-D-7)**

(wherein all symbols are as defined above).

This C-alkylation reaction is known and is carried out, for example, by subjecting a reaction with the compound having leaving group in an organic solvent (for example, diethyl ether, tetrahydrofuran, etc.) in the presence of a base (for example, lithium diisopropylamide, lithium bis(trimethylsilyl)amide, etc.) at a temperature of about -78 to 100°C.

In the reaction scheme, a starting material or the compound represented by formula (A-1), formula (A-2), formula (II-B-2), formula (II-B-3), formula (II-B-4), formula (II-B-6), formula (II-C-2)), formula (II-D-2), formula (II-D-5) and formula (II-D-7) used as the reagent can be produced by a known method, for example, a mixed method of a method described in "Comprehensive Organic Transformations: A Guide to Functional Group Preparations, Second edition (written by Richard C. Larock, John Wiley & Sons Inc, 1999)".

In each reaction in the present description, a reaction with heating can be carried out, as is apparent to those skilled in the art, by using a water bath, an oil bath, a sand bath or a microwave.

In each reaction in the present description, a solid phase supported reagent obtained by supporting on a polymer (for example, polystyrene, polyacrylamide, polypropylene, polyethylene glycol, etc.) may be used appropriately.

In each reaction in the present description, a reaction product can be purified by conventional purification means, for example, a distillation under normal pressure or reduced pressure, a high performance liquid chromatography using a silica gel or magnesium silicate, a thin layer chromatography, an ion-exchange resin, a scavenger resin or a chromatography or a washing, or a recrystallization, etc. The purification may be carried out for every reaction, or may be carried out after the completion of some reactions.

[Toxicity]

A toxicity of the compound represented by the formula (I), a salt thereof, a quaternary ammonium salt thereof, an N-oxide form thereof, a solvate thereof or a prodrug thereof (abbreviated "the compound used for CXCR3 antagonist of the present invention" hereinafter), is very low, and therefore it is considered to be sufficiently safe when used as a drug.

[Application to pharmaceuticals]

The compound used for CXCR3 antagonist of the present invention has CXCR3 antagonistic activity in an animal including human, particularly human, is useful as a preventive, therapeutic and/or progression-suppressing agent for a disease such as an immune or an allergic disease [e.g, an atopic disease, anaphylaxis or anaphylactoid reaction, an autoimmune disease (e.g, multiple sclerosis, rheumatoid arthritis, systemic lupus erythematosus, type I diabetes, glomerulonephritis, Sjogren's syndrome and the like), systemic inflammatory response syndrome (SIRS), a rejection response to transplanted organ, tissue and/or cell, an allergia angiitis, rhinitis, arthritis, an inflammatory oculopathy (e.g, conjunctivitis) and the like], a gastrointestinal disease [e.g, inflammatory bowel disease (e.g, colitis ulcerosa, Crohn's disease, eosinophil stomach and intestines symptoms and the like), hepatitis, nephritis, nephropathy, pancreatitis and the like], a respiratory disease [e.g, asthma, a chronic obstructive pulmonary disease, respiratory insufficiency, lungs irritation, acute respiratory distress syndrome (ARDS), allergic bronchopulmonary aspergillosis], a brain / neurologic disease [e.g, a cerebrovascular disease (e.g, arterial sclerosis, thrombosis, an ischemia / reperfusion disorder, re-constriction, infarct and the like) and the like], a dermatosis [e.g, dermatitis (e.g, atopic dermatitis, psoriasis, contact dermatitis, eczema, urticaria, itch and the like) and the like], a metabolism/an endocrine disease (e.g, diabetes and the like), a carcinoma disease [e.g, malignant neoplasm (e.g, leucaemia, solid cancer and cancer metastasis and the like) and the like], an infection or a disease with infection [e.g, viral disease (e.g, acquired immunodeficiency syndrome (AIDS), SARS and the like), affiliation symptom (dementia) by AIDS and the like].

In the present description, with "therapy", that disease state is led to direction of cure is meant, and with "progression-suppression", that symptomatic progress / exacerbation is restrained, and left progression of disease state is meant.

The compound used for CXCR3 antagonist of the present invention is safe and low toxic, and, therefore for example, it can be administered to human and mammalian (such as rat, mouse, rabbit, sheep, pig, cattle, cat, dog, monkey).

The compound used for CXCR3 antagonist of the present invention may be administered as a concomitant drug by using in combination with other drugs for the purpose of

(1) complementation and/or enhancement of the preventive, therapeutic and/or progression-suppressing effects of the compound used for CXCR3 antagonist of the present invention,
(2) improvement of pharmacokinetics and absorption of the compound used for CXCR3 antagonist of the present invention and reduction of the dosage, and/or

(3) reduction of side effects of the compound used for CXCR3 antagonist of the present invention.

Also, the compound used for CXCR3 antagonist of the present invention may be administered as a concomitant drug by using in combination with other drugs the purpose of

(1) complementation and/or enhancement of preventive, therapeutic and/or progression-suppressing effects of other drugs,
(2) improvement of pharmacokinetics and absorption of the compound and reduction of the dosage of other drugs, and/or
(3) reduction of side effects of other drugs.

Concomitant agents of the compound used for CXCR3 antagonist of the present invention with other drugs may be administered in a mode of an agent in which both components are comprised in a single preparation or in a mode of separate preparations. When administration is conducted using separate preparations, a simultaneous administration and administrations with time difference are included. In the case of administrations with time difference, the compound used for CXCR3 antagonist of the present invention may be firstly administered and then the other drug may be administered, and *vice versa.* Each of the methods for the administration may be same or different.

Other agents as described above may be low molecular compounds, and high molecular proteins, polypeptides, polynucleotides (DNA, RNA, genes),anti-sense, decoys, antibodies, vaccines, etc. The dose of other agents may be determined taking the clinically used dose as a reference appropriately. A ratio of the compound used for CXCR3 antagonist of the present invention and the other agents may be determined according to a patients' age, weight, route of administration, time of administration, the target disease, symptom or combination, etc. For example, approximately 0.01 to 100 of the other agents in weight ratio may be used versus the compound used for CXCR3 antagonist of the present invention. One or more of the other agent(s) may be selected from the same group or different groups described hereafter, and may be administered alone or in combination thereof in optional ratios.

The disease, on which the preventive, therapeutic and/or progression-suppressing effects are exerted by the concomitant drug, is not specifically limited, and may be any disease which complements and/or enhances the preventive, therapeutic and/or progression-suppressing effects of the compound used for CXCR3 antagonist of the present invention.

For the compound used for CXCR3 antagonist of the present invention and the other drugs put together thereof and used, such as a drug to use in the preventive, therapeutic and/or progression-suppressing agents against an autoimmune disease, such as a nonsteroidal anti-inflammatory drug, a disease modifying antirheumatic drug (DMARDs, slow-acting antirheumatic drug), steroids, an immunosuppressant agent, antiinflammatory enzyme preparations, chondroprotective agents, T-cell activator inhibitors, a TNF$\alpha$ inhibitor (include protein preparation such as anti-TNF$\alpha$ antibody), a prostaglandin synthase inhibitor, an IL-1 inhibitor, an IL-6 inhibitor (include protein preparation such as anti-IL-6 receptor antibody), interferon gamma agonists, prostaglandins, a phosphodiesterase inhibitor, a metalloproteinase inhibitor and a chemokine receptor antagonist are given.

For a drug to use in the preventive, therapeutic and/or progression-suppressing agents against psoriasis, such as a steroid drug, a vitamin $D_3$ pharmaceutical and an etretinate are given.

For a drug to use in the preventive, therapeutic and/or progression-suppressing agents against rejection response of transplantation, such as an immunosuppressant and a chemokine receptor antagonist are given.

For a drug to use in the preventive, therapeutic and/or progression-suppressing agents against ischemic diseases, such as a radical scavenger, an astrocyte modulator, N-methyl D-aspartate (NMDA) antagonist, $\alpha$-amino-3-hydroxy-5- methylisoxazole-4-propionate (AMPA) antagonist, an antithrombotic drug, a thrombolytic agent, an immunosuppressant, an intercellular adhesion factor inhibitor, a nitric monoxide synthase (NOS) inhibitor, a neurotrophic factor and an interleukin-8 antagonist are given.

For a drug to use in the preventive, therapeutic and/or progression-suppressing agents against allergic diseases, for example, for a drug to use in asthma, such as a steroid, a $\beta_2$ adrenergic receptor irritant, a leukotriene receptor antagonist, a thromboxane synthase inhibitor, a thromboxane $A_2$ receptor antagonist, a mediator release inhibitor, an antihistaminic, a xanthine derivative, an anticholinergic agent, a cytokine inhibitor, prostaglandins, a forskolin pharmaceutical, a phosphodiesterase inhibitor, an elastase inhibitor, a metalloproteinase inhibitor, a chemokine receptor antagonist, expectorans and antibiotics are given.

As chemokine receptor antagonists, such as internal ligand of chemokine receptor, its derivatives, non-peptide low molecular compound or antibody of chemokine receptor are included.

Examples of internal ligand of chemokine receptor are, for example, MIP-1$\alpha$, MIP-1$\beta$, RANTES, SDF-1$\alpha$, SDF-1$\beta$, MCP-1, MCP-2, MCP-4, Eotaxin and MDC, etc.

Examples of derivatives of internal ligand of chemokine receptor are, for example, AOP-RANTES, Met-SDF-1$\alpha$, Met- SDF-1$\beta$, etc.

Non-peptide low molecular compounds as chemokine receptor antagonist are, for example, CCR1, CCR2,

CCR3, CCR4, CCR5, CXCR1, CXCR2, CXCR3, CXCR4 receptor antagonist or agonit, etc.

CCR2 antagonists are concretely the compounds described in WO99/07351, WO99/40913, WO00/46195, WO00/46196, WO00/46197, WO00/46198, WO00/46199, WO00/69432 or WO00/69815 or in Bioorg. Med. Chem. Lett., 10, 1803 (2000), etc.

CCR3 antagonists are the compounds described in, for example, DE 19837386, WO99/55324, WO99/55330, WO00/04003, WO00/27800, WO00/27835, WO00/27843, WO00/29377, WO00/31032, WO00/31033, WO00/34278, WO00/35449, WO00/35451, WO00/35452, WO00/35453, WO00/35454, WO00/35876, WO00/35877, WO00/41685, WO00/51607, WO00/51608, WO00/51609, WO00/51610, WO00/53172, WO00/53600, WO00/58305, WO00/59497, WO00/59498, WO00/59502, WO00/59503, WO00/62814, WO00/73327 orWO01/09088, etc.

CCR5 antagonists are, for example, TAK-779, SCH-351125 (SCH-C), SCH-417690 (SCH-D), UK-427857, GW873140A (ONO-4128), TAK-220, etc. Moreover, there include the compounds described in, for example, WO99/17773 WO99/32100, WO00/06085, WO00/06146, WO00/10965, WO00/06153, WO00/21916, WO00/37455, EP1013276, WO00/38680, WO00/39125, WO00/40239, WO00/42045, WO00/53175, WO00/42852, WO00/66551, WO00/66558, WO00/66559, WO00/66141, WO00/68203, JP2000309598, WO00/51607, WO00/51608, WO00/51609, WO00/51610, WO00/56729, WO00/59497, WO00/59498, WO00/59502, WO00/59503, WO00/76933, WO98/25605, WO99/04794, WO99/38514, Bioorg. Med. Chem. Lett., 10, 1803 (2000), or Bioorg. Med. Chem. Lett., 11, 2663 (2003), etc.

CXCR3 antagonists are the compounds described in, for example, WO01/16114, WO02/083143, WO02/085862, US6469002, WO03/101970, WO04/094381, or WO05/003127, etc.

CXCR4 antagonists are, for example, AMD-31 00, AMD-070, T-22, KRH-1120, KRH-1636, KRH-2731, CS-3955 or the compounds described in WO00/66112, WO2004/24697, etc.

Antibodies of chemokine receptor are Pro-140, etc.

Examples of the steroids for external application include clobetasol propionate, diflorasone acetate, fluocinonide, monometasone furancarboxylate, betamesone dipropionate, betamesone butyropropionate, betamesone valerate, difluprednate, budesonide, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyropropionate, deprodone propionate, prednisolone valeroacetate, fluocinolone acetonide, beclometasone dipropionate, triamcinonide acetonide, flumethasone pivalate, alclometasone dipropionate, clobetasone butyrate, prednisolone, beclometasone propionate, and fludroxycortide, etc. Examples of the steroids for internal use or injection include cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredon acetate, methyl prednisolone, methyl prednisolone acetate, methyl prednisolone sodium succinate, triamicinolon, triamicinolon acetate, triamicinonolon acetonide, dexamethasone, dexamethasone acetate, dexamethasone sodium phosphate, dexamethasone palmitate, paramethasone acetate, and betamethasone, etc. Examples of the steroids as an inhalant include beclomethasone propionate, fluticasone propionate, budesonide, flunisolide, triamicinolon, ST-126P, ciclesonide, dexamethasone palomitionate, mometasone furancarboxylate, prasterone sulfonate, deflazacort, methylprednisolone suleptanate, and methylprednisolone sodium succinate, etc.

Examples of the immunosuppressant include tacrolimus (FK506), cyclosporin, sirolimus (rapamycin), corticosteroids, azathioprine, mycophenolate mofetil, and cyclophosphamide, etc.

Examples of Vitamin $D_3$ pharmaceutical include calcitriol, tacalcitol, maxacalcitol, etc.

Examples of the $\beta_2$ adrenoreceptor stimulant include fenoterol hydrobromide, salbutamol sulfate, terbutaline sulfate, formoterol fumarate, salmeterol xinafoate, isoprotenol sulfate, orciprenalin sulfate, chloroprenalin sulfate, epinephrine, trimetoquinol hydrochloride, hexoprenalinmesyl sulfate, procaterol hydrochloride, tulobuterol hydrochloride, tulobuterol, pirbuterol hydrochloride, clenbuterol hydrochloride, mabuterol hydrochloride, ritodrine hydrochloride, bambuterol, dopexamine hydrochloride, meradrin tartrate, AR-C68397, levosalbutamol, R,R-formoterol, KUR-1246, KUL-7211, AR-C89855, and S-1319, etc.

Examples of the leukotriene receptor antagonist include pranlukast hydrate, montelukast, zafirlukast, seratrodast, MCC-847, KCA-757, CD-615,YM-158, L-740515, CP-195494, LM-1484, RS-635, A-93178, S-36496, BIIL-284, and ONO-4057, etc.

Examples of the thromboxane synthetase inhibitor include ozagrel hydrochloride, and imitrodast sodium, etc.

Examples of the thromboxane $A_2$ receptor antagonist include seratrodast, ramatroban, domitroban calcium dihydrate, and KT-2-962, etc.

Examples of the mediator releasing inhibitor include tranilast, sodium cromoglicate, anlexanox, repirinast, ibudilast, tazanolast, and pemilolast potassium, etc.

Examples of the antihistamines include ketotifen fumarate, mequitazine, azelastine hydrochloride, oxatomide, terfenadine, emedastine fumarate, epinastine hydrochloride, astemizole, ebastin, cetirizine hydrochloride, bepotastine, fexofenadine, lolatadine, deslolatadine, olopatadine hydrochloride, TAK-427, ZCR-2060, NIP-530, mometasone furoate, mizolastine, BP-294, andolast, auranofin, and acribastin, etc.

Examples of the xanthine derivatives include aminophylline, theophylline, doxophylline, cipamphylline, and

diprophilline, etc.

Examples of the anticholinergic agent include ipratropium bromide, oxitropium bromide, flutropium bromide, cimetropium bromide, temiverine, tiotropium bromide, and revatropate (UK-112166), etc.

Examples of the cytokine inhibitor include suplatast tosilate (trade name: IPD), etc.

Examples of the prostaglandins (hereinafter abbreviated as "PG") include PG receptor agonist, and PG receptor antagonist, etc. Examples of the PG receptor include PGE receptor (EP1, EP2, EP3, EP4), PGD receptor (DP, CRTH2), PGF receptor (FP), PGI receptor (IP), and TX receptor (TP), etc.

Examples of the phosphodiesterase inhibitor include, for example, rolipram, cilomilast (trade name: Ariflo), Bay 19-8004, NIK-616, roflumilast (BY-217), cipamfylline (BGL-61063), atizolam (CP-80633), SCH-351591, YM-976, V-11294A, PD-168787, D-4386, IC-485, and ONO-6126 as PDE-4 inhibitor, etc.

Examples of the elastase inhibitors include ONO-5046, ONO-6818, MR-889, PBI-1101, EPI-HNE-4, R-665, ZD-0892, ZD-8321, GW-311616, andAE-3763, etc.

Examples of the expectorant include foeniculated ammonia spirit, sodium hydrogencarbonate, bromhexine hydrochloride, carbocisteine, ambroxol hydrochloride, sustained release ambroxol hydrochloride, methylcysteine hydrochloride, acetyl cysteine, L-ethylcysteine hydrochloride, and tyloxapol, etc.

Examples of the nonsteroidal antiinflammatory drug include sasapyrine, sodium salicylate, aspirin, aspirin dialuminate formulation, diflunisal, indomethacin, suprofen, ufenamate, dimethylisopropyl azulen, bufexamac, felbinac, diclofenac, tolmetin sodium, Clinoril, fenbufen, napmetone, proglumetacin, indomethacin farnesil, acemetacin, proglumetacin maleate, amfenac sodium, mofezolac, etodolac, ibuprofen, ibuprofen piconol, naproxen, flurbiprofen, flurbiprofen axethyl, ketoprofen, fenoprofen calcium, tiaprofenen, oxaprozin, pranoprofen, loxoprofen sodium, aluminoprofen, zaltoprofen, mefenamic acid, aluminum mefenamate, tolfenamic acid, floctafenine, ketophenylbutazone, oxyfenbutazone, piroxicam, tenoxicam, anpiroxicam, napageln cream, epirizole, tiaramide hydrochloride, tinoridine hydrochloride, emorfazone, sulpyrine, Migrenin, Saridon, Sedes G, Amipylo N, Sorbon, pyrine system antipyretics, acetaminophen, phenacetin, dimethothiazine mesylate, simetride formulation, and antipyrine system antipyretics, etc.

Examples of the disease modifying anti-rheumatic drug (DMARDs, slow-acting anti-rheumatic drug) include gold thioglucose, aurothiomalate sodium, auranofin, actarit, D-penicillamine preparations, lobenzarit disodium, bucillamine, hydroxychloroquine, salazosulfapyridine, methotrexate, and leflunomide, etc.

Examples of the chondroprotective agents include sodium hyaluronate, glucosamine, chondroitin sulfate, and glucosaminoglycan polysulfate, etc.

Examples of the prostaglandin synthase inhibitor include salazosulfapyridine, mesalazine, olsalazine, 4-aminosalicylic acid, JTE-522, auranofin, carprofen, diphenpyramid, flunoxaprofen, flurbiprofen, indomethacin, ketoprofen, lornoxicam, loxoprofen, Meloxicam, oxaprozin, parsalmide, piproxen, piroxicam, piroxicam betadex, piroxicam cinnamate, tropine indomethacinate, zaltoprofen, and pranoprofen, etc.

Examples of the radical scavenger include radicut, etc.

Examples of the astrocyte modulator include ONO-2506, etc.

Examples of the antithrombotic drug include cataclot, argatroban, and aspirin, etc.

Examples of the thrombolytic agent include human tissue plasminogen activator (t-PA), urokinase, and heparin, etc.

Examples of the antiinflammatory enzyme preparations include lysozyme chloride, bromelain, pronase, serrapeptase, and streptokinase-streptodornase, etc.

Examples of TNFα inhibitor (include protein preparation such as anti-TNFα antibody) include infliximab, adalimumab, and etanercept, etc.

Examples of IL-6 inhibitor (include protein preparation such as anti-IL-6 receptor antibody) include and MRA, etc.

Examples of IL-1 inhibitor (include protein preparation such as human IL-1 receptor antagonist) include and anakinra, etc.

Examples of antibiotics include sodium cefuroxime, meropenem trihydrate, netilmicin sulfate, sisomicin sulfate, ceftibuten, PA-1806, IB-367, tobramycin, PA-1420, doxorubicin, astromicin sulfate, cefetamet pivoxil hydrochloride, etc. Examples of antibiotics as an inhalant include PA-1806, IB-367, tobramycin, PA-1420, doxorubicin, astromicin sulfate, and cefetamet pivoxil hydrochloride, etc.

In addition, the drugs found in future as well as the drugs found by the present is included in the other drugs supplementing and/or reinforcing the preventive, therapeutic and/or progression-suppressing effect of the compound used for a CXCR3 antagonist of the present invention based on the mechanism described above.

For the purpose above described, a compound used for CXCR3 antagonist of the present invention, or a physic composition containing a compound used for a CXCR3 antagonist of the present invention compound and other pharmaceutical combination agent may be normally administered systemically or locally, usually by oral or parenteral administration.

The doses to be administered are determined depending upon, for example, age, body weight, symptom, the desired therapeutic effect, the route of administration, and the duration of the treatment. In the human adult, the doses

per person are generally from 0.1 mg to 1000 mg, by oral administration, up to several times per day, and from 50 μg to 500 mg, by parenteral administration (preferably intravenous administration), up to several times per day, or continuous administration from 1 to 24 hours per day from vein.

As described above, the doses to be used depend upon various conditions. Therefore, there are cases in which doses lower than or greater than the ranges specified above may be used.

The compound used for CXCR3 antagonist of the present invention, or physic composition containing a compound used for CXCR3 antagonist of the present invention compound and other pharmaceutical combination agent may be administered, for example, in the form of solid for oral administration, liquid forms for oral administration, injections, liniments or suppositories for parenteral administration, ophthalmic solution, inhalant.

Solid forms for oral administration include such as compressed tablets, pills, capsules, dispersible powders, and granules. Capsules include such as hard capsules and soft capsules.

In such solid forms such as one or more of the active compound(s) may be admixed with vehicles (such as lactose, mannitol, glucose, microcrystalline cellulose or starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include containment within capsules of absorbable materials such as gelatin.

Liquid forms for oral administration include such as pharmaceutically acceptable solutions, suspensions, emulsions, syrups and elixirs. In such forms, one or more of the active compound(s) may be dissolved, suspended or emulsified into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

The dosage form of the external preparation for parenteral administration includes such as liquid for external preparation, ointment, gel, cream, fomentation, patch, liniment, propellant, inhalant, spray, aerosol, ophthalmic solution, nasal drop, suppositories for intrarectal injection and pessaries for vaginal administration. These products contain one or more active substances and are prepared according to the formulation which is known or commonly used.

An ointment is prepared according to the formulation which is known or commonly used. For example, it is prepared by triturating or dissolving one or more active substance(s) in a base. An ointment base is selected from well known ones or those commonly employed. For example, those selected from higher fatty acids or higher fatty acid esters (such as adipic acid, myristic acid, palmitic acid, stearic acid, oleic acid, adipate ester, myristate ester, palmitate ester, stearate ester, oleate ester), waxes (such as beeswax, whale wax, ceresin), surfactants (such as polyoxyethylene alkyl ether phosphate ester), higher alcohols (such as cetanol, stearyl alcohol, cetostearyl alcohol), silicone oils (such as dimethylpolysiloxane), hydrocarbons (such as hydrophilic petrolatum, white petrolatum, purified lanolin, liquid paraffin), glycols (such as ethylene glycol, diethylene glycol, propylene glycol, polyethylene glycol, macrogol), vegetable oils (such as castor oil, olive oil, sesame oil, turpentine oil), animal oils (such as mink oil, egg yolk oil, squalane, squalene), water, absorption accelerators, agents for preventing contact dermatitis are used alone or in combination. Furthermore, it may contain humectants, preservatives, stabilizers, antioxidizing agents, flavors, and the like.

A gel is prepared according to the formulation which is known or commonly used. For example, it is prepared by dissolving one or more active substances in a base. A gel base is selected from a base which is known or commonly used. For example, those selected from lower alcohols (such as ethanol, isopropyl alcohol), gelling agents (such as carboxymethyl cellulose, hydroxyethyl cellulose, hydroxypropyl cellulose, ethyl cellulose), neutralizers (such as triethanolamine, diisopropanolamine), surfactants (such as monostearic acid polyethylene glycol), gums, water, absorption accelerator, and agent for preventing contact dermatitis are used alone or in combination. Furthermore, it may contain preservatives, antioxidizing agents, and flavoring agents.

A cream is prepared according to the formulation which is known or commonly used. For example, it is prepared by dissolving or emulsifying one or more active substances in a base. A cream base is selected from a base which is known or commonly used. For example, those selected from higher fatty acid esters, lower alcohols, hydrocarbons, polyhydric alcohols (such as propylene glycol, 1,3-butylene glycol), higher alcohols (such as 2-hexyl decanol, cetanol), emulsifiers (such as polyoxyethylene alkyl ethers, fatty acid esters), water, absorption accelerators, and agents for preventing contact dermatitis are used alone or in combination. Furthermore, it may contain preservatives, antioxidizing agents, and flavoring agents.

A fomentation is prepared according to the formulation which is known or commonly used. For example, it is prepared by dissolving one or more active substance(s) in a base to obtain a kneaded mixture and spreading the kneaded mixture over a substrate. A fomentation base is selected from a base which is known or commonly used. For example, those selected from thickeners (such as polyacrylic acid, polyvinylpyrrolidone, gum arabic, starch, gelatin, methyl cellulose), humectants (such as urea, glycerin, propylene glycol), fillers (such as kaolin, zinc oxide, talc, calcium, magnesium), water, solubilizing agents, tackifiers, and agents for preventing contact dermatitis are used alone or in combination. Furthermore, it may contain preservatives, antioxidizing agents, and flavoring agents.

A patch is prepared according to the formulation which is known or commonly used. For example, it is prepared by dissolving one or more active substance(s) in a base, and spreading the solution over a substrate. A patch base is selected from a base which is known or commonly used. For example, those selected from polymer bases, fats and oils, higher fatty acids, tackifiers, and agents for preventing contact dermatitis are used alone or in combination. Furthermore, it may contain preservatives, antioxidizing agents, and flavoring agents.

A liniment is prepared according to the formulation which is known or commonly used. For example, it is prepared by dissolving, suspending or emulsifying one or more active substance(s) in one or more kind(s) selected from water, alcohol (such as ethanol, polyethylene glycol), higher fatty acids, glycerin, soap, emulsifiers, and suspending agents. Furthermore, it may contain preservatives, antioxidizing agents, and flavoring agents.

A propellant, an inhalant, and a spray may contain, in addition to a diluent used commonly, a stabilizer such as sodium hydrogen sulfite and a buffer capable of imparting isotonicity, for example, an isotonicity such as sodium chloride, sodium citrate or citric acid.

An injection for parenteral administration includes all injections and also includes a drop. For example, it includes intramuscular injection, subcutaneous injection, endodermic injection, intraarterial injection, intravenous injection, intraperitoneal injection, intraspinal injection, and intravenous drop.

The injection for parenteral administration includes solutions, suspensions, emulsions, and solid injections used by dissolving or suspending in a solvent before use. The injection is used after dissolving, suspending, or emulsifying one or more active substance(s) in a solvent. As the solvent, for example, distilled water for injection, physiological saline, vegetable oil, and alcohols such as propylene glycol, polyethylene glycol or ethanol are used alone or in combination. Furthermore, the injection may contain stabilizers, solubilizing agents (such as glutamic acid, aspartic acid, polysolvate 80®), suspending agents, emulsifiers, soothing agents, buffers, and preservatives. These injections are prepared by sterilizing in the final process, or prepared by an aseptic treatment. Also, a sterile solid, for example, a freeze-dried product is prepared and can be used after dissolving in sterilized distilled water or distilled water for sterile injection, or the other solvent before use.

An ophthalmic solution for parenteral administration includes ophthalmic solution, suspension type ophthalmic solution, emulsion type ophthalmic solution, ophthalmic solution soluble when used, and eye ointment.

These ophthalmic solutions are prepared according to a known method. For example, one or more active substance(s) are dissolved, suspended or emulsified in a solvent before use. As the solvent for ophthalmic solution, for example, sterilized purified water, physiological saline, and other aqueous solvent or non-aqueous agent for injection (such as vegetable oil) are used alone or in combination. If necessary, the ophthalmic solution may contain appropriately selected isotonizing agents (such as sodium chloride, concentrated glycerin), buffering agents (such as sodium phosphoate, sodium acetate), surfactants (such as polysolvate 80 (trade name), polyoxyl 40 stearate, polyoxyethylene hardened castor oil), stabilizers (such as sodium citrate, sodium edetate), and antiseptics (such as benzalkonium chloride, paraben). These ophthalmic solutions are prepared by sterilizing in the final process, or prepared by an aseptic treatment. Also, a sterile solid, for example, a freeze-dried product is prepared and can be used after dissolving in sterilized distilled water or distilled water for sterile injection, or the other solvent before use.

An inhalants for parenteral administration includes aerozol, inhalation powder, and inhalation solution, and the inhalation solution may be such a configuration that it is used after dissolving in water or other suitable medium at the point of use.

These inhalants are prepared according to a known method.

For example, an inhalation solution is prepared by appropriately selecting antiseptics (such as benzalkonium chloride, paraben), colorants, buffering agents (such as sodium phosphate, sodium acetate), isotonizing agents (such as sodium chloride, concentrated glycerin), thickeners (such as carboxyvinyl polymer), and absorption accelerator, if necessary.

An inhalation powder is prepared by appropriately selecting lubricants (such as stearic acid and a salt thereof), binders (such as starch, dextrin), excipients (such as lactose, cellulose), colorants, antiseptics (such as benzalkonium chloride, paraben), and absorption accelerator if necessary.

In case of administering the inhalation solution, a spraying apparatus (such as atomizer, nebulizer) is commonly used. In case of administering the inhalation powder, an inhalation administration apparatus for powder is commonly used.

## EFFECT OF THE INVENTION

A compound used for CXCR3 antagonist of the present invention is useful as a preventive, therapeutic and/or progression-suppressing agent for a CXCR3-mediated disease because it has CXCR3 antagonist activity.

BEST MODE FOR CARRYING OUT THE INVENTION

The present invention is explained in detail by means of example as follows, but the present invention is not limited to these examples.

[PREPARATION EXAMPLES]

In chromatographic separations and TLC, the solvents in parenthesis show the eluting and developing solvents and the ratios of the solvents used are by volume. The number value that was shown in a part of NMR is the measured value of $^1$H-NMR when deuterochloroform was used as solvent for measurement except the case which mentioned specially.

Reversed-phase high-speed liquid chromatography assay condition to measure HPLC retention time is as follows.

<Condition A>

HPLC equipment: HP1100 HPLC made in Hewlett Packard company
Mass spectrometry equipment : Sciex API150 spectrometer made in Perkin-Elmer corporation
Column : Gemini 5 μm C18 column (50 x 4.6 mm) made in Phenomenex Inc. Temperature of Column : 40°C
Flow rate : 2mL/min
Mobile phase A : 0.1% aqueous solution of trifluoroacetic acid
Mobile phase B : 0.1 % trifluoroacetic acid - acetonitrile solution
LC-MS/ELS Gradient:

| Time(min) | %A | %B |
|---|---|---|
| 0.00 | 99 | 1 |
| 4.50 | 20 | 80 |
| 5.50 | 20 | 80 |

<Condition B>

HPLC equipment : LC-10ADvp series HPCL pump made in Shimazu Corporation+Dual wavelength UV detector+215 autosampler made in Gilson Inc.
ELSD detector : 75 ELS detector made in Sedex
Mass spectrometry equipment : API 150EX mass spectrometer made in PE/Sciex
Column : Gemini 5 μm C18 column (50 x 4.6 mm) made in Phenomenex Inc. Temperature of Column : 40 °C
Flow rate : 2mL/min
Mobile phase A : 10mM aqueous solution of ammonium carbonate
Mobile phase B : acetonitrile
Injection volume : 5 μL
LC-MS/ELS Gradient :

| Time (min) | %A | %B |
|---|---|---|
| 0.00 | 95 | 5 |
| 3.00 | 5 | 95 |
| 3.80 | 5 | 95 |
| 4.00 | 95 | 5 |
| 5.00 | 95 | 5 |

<Condition C>

Column : Gemini 5 μm C 18 column (50 x 4.6 mm) made in Phenomenex Inc. Temperature of Column : Room temperature
Flow rate : 2mL/min

Mobile phase A : 0.05% aqueous solution of formic acid
Mobile phase B : 0.05% formic acid - acetonitrile solution
LC-MS/ELS Gradient :

| Time (min) | %A | %B |
|---|---|---|
| 0.00 | 95 | 5 |
| 3.00 | 0 | 100 |

<Condition D>

Column : Xterra (registered trademark) MS $C_{18}$ 5 μm, 4.6 × 50mm I.D.
Flow rate : 3mL/min
Mobile phase A : 0.1 % aqueous solution of trifluoroacetic acid
Mobile phase B : 0.1 % trifluoroacetic acid - acetonitrile solution
LC-MS/ELS Gradient :

A mixing ratio of A and B was fixed to 95/5 between 0.5 min interval after assay initiation. A mixing ratio of A and B was changed into 0/100 between 2.5 min linearly afterwards. A mixing ratio of A and B was fixed to 0/100 between 0.5 min interval afterwards. A mixing ratio of A and B was changed into 95/5 between 0.01 min linearly afterwards.

Example 1 (1) : (3R)-3-benzyl-9-(4-chlorobenzyl)-1-propyl-1,4,9-triazaspiro[5.5]undecane-2,5- dione

Isonitrile resin (400mg, a preparation method are described below) prepared separately was swelled with a mixed solution of tetrahydrofuran - methanol (1:1) (4.5mL), and to the solution was added N-(4-chlorobenzyl)-4-piperidone (233mg, 1.04mmol), N-(tert-butoxycarbonyl)-D-phenylalanine (276mg, 1.04mmol) and propylamine (61 mg, 1.04mmol), and the mixture was stirred for 24hours at 60°C. The reaction mixture was filtrated, and the obtained resin was washed with a mixed solution of tetrahydrofuran-methanol (1:1), methanol and dichloromethane sequentially. To the obtained resin was added a mixed solution of trifluoroacetic acid-dichloromethane (1:1), and was stirred for 30minutes at room temperature. The reaction mixture was filtrated, and the obtained resin was washed with dichloromethane, 5% diisopropyl ethylamine/dichloromethane solution and toluene sequentially. To the obtained resin was added toluene, and was stirred for 16hours at 60°C. After the reaction mixture was cooled in room temperature, it was filtered. The obtained resin was washed with a mixed solution of tetrahydrofuran-methanol (1:1) and methanol sequentially. The filtrate and washing were gathered up, and concentrated. The obtained residue was purified by column chromatography on silica gel (chloroform: methanol = 20 : 1) to give the compound of the present invention (22mg) having the following physical data.
TLC : Rf 0.45 (chloroform : methanol = 20 : 1);
NMR : δ 7.60-7.74 (m, 1H), 7.40-7.60 (m, 2H), 7.13-7.40 (m, 6H), 5.51-5.63 (m, 1H), 4.13-4.26 (m, 1H), 3.18-3.57 (m, 5H), 2.60-3.00 (m, 4H), 2.41-2.60 (m, 1H), 1.73-2.08 (m, 3H), 1.37-1.66 (m, 3H), 0.94 (t, J=7.40Hz, 3H).
A preparation method of isonitrile resin used for the reaction : After the commercial aminomethyl resin hydrochloride (20g, quantity of induction : 0.52mmol/g) was washed with 5% diisopropyl ethylamine/N, N-dimethylformamide solution and N, N-dimethylformamide sequentially, was swelled with N, N-dimethylformamide (80mL), was added ethyl formate (120mL) hereto. The reaction mixture was stirred for 24hours at 115°C. The reaction mixture was filtrated, and the obtained resin was washed with N, N-dimethylformamide, dichloromethane, methanol and dichloromethane sequentially. To the obtained resin was suspension with dichloromethane (200mL), added triphenylphosphine (13.64g), triethylamine (7.24mL) and carbon tetrachloride (5.02mL), and stirred for 90minutes at 60°C. After the reaction mixture was filtrated, and the obtained resin was washed with dichloromethane, methanol and dichloromethane sequentially, dried over, and was gived an aimed isonitrile resin (20.7g).

Example 1 (2) ~ Example 1 (16)

By the same procedure as described in Example 1 (1) using the corresponding piperidone compounds instead of N-(4-chlorobenzyl)-4-piperidone, the corresponding amino acid compounds instead of N- (tert - butoxycarbonyl) -D-phenylalanine and the corresponding amine compounds instead of propylamine, the compounds of the present invention having the following physical data were obtained.
Example 1 (2) : (3R)-3-benzyl-9-(4-chlorobenzyl)-1-(cyclopropylmethyl)-1,4,9-triazaspiro[5.5]undecane-2,5-dione

TLC : Rf 0.45 (chloroform : methanol = 20 : 1);
NMR : δ 7.60-7.76 (m, 1H), 7.41-7.61 (m, 2H), 7.10-7.41 (m, 6H), 5.53-5.59 (m, 1H), 4.15-4.27 (m, 1H), 3.44-3.55 (m, 3H), 3.42 (d, J=6.80Hz, 2H), 2.76-2.94 (m, 2H), 2.63-2.76 (m, 2H), 2.38-2.52 (m, 1H), 1.96-2.14 (m, 2H), 1.82-1.94 (m, 1H), 1.54-1.73(m, 1 H), 0.87-1.06 (m, 1H), 0.35-0.61 (m, 4H).
Example 1 (3) : (3R)-3-benzyl-9-(4-chlorobenzyl)-1-cyclobutyl-1,4,9-triazaspiro[5.5]undecane- 2,5-dione
TLC : Rf 0.45 (chloroform : methanol = 20 : 1);
NMR : δ 7.61-7.74 (m, 1H), 7.39-7.61 (m, 2H), 7.09-7.38 (m, 6H), 5.57-5.70 (m, 1H), 4.13-4.25 (m, 1H), 3.85-4.05 (m, 1H), 3.48 (d, J=13.40Hz, 1H), 3.43 (d, J=13.40Hz, 1H), 3.05-3.29 (m, 4H), 2.40-2.80 (m, 4H), 1.82-2.15 (m, 4H), 1.54-1.83 (m, 3H), 0.68-0.80 (m, 1H).
Example 1 (4) : (3R)-3-benzyl-9-(4-chlorobenzyl)-1-cyclopentyl-1,4,9-triazaspiro[5.5]undecane- 2,5-dione
TLC : Rf 0.45 (chloroform : methanol = 20 : 1);
NMR : δ 7.60-7.76 (m, 1H), 7.37-7.61 (m, 2H), 7.07-7.39 (m, 6H), 5.57-5.72 (m, 1H), 4.16(td, J=5.60, 1.90Hz, 1H), 3.58-3.75 (m, 1H), 3.49 (d, J=13.20Hz, 1 H), 3.44 (d, J=13.20Hz, 1H), 3.11 (d, J=5.30Hz, 2H), 2.40-2.84 (m, 5H), 1.88-2.35 (m, 5H), 1.44-1.85 (m, 5H), 0.74-0.86 (m, 1H).
Example 1 (5) : (3S)-3-benzyl-9-(4-chlorobenzyl)-1-cyclobutyl-1,4,9-triazaspiro[5.5]undecane- 2,5-dione
TLC : Rf 0.45 (chloroform: methanol = 20 : 1);
NMR : δ 7.60-7.72 (m, 1H), 7.39-7.58 (m, 2H), 7.08-7.35 (m, 6H), 5.56-5.68 (m, 1H), 4.13-4.25 (m, 1H), 3.84-4.04 (m, 1H), 3.48 (d, J=13.40Hz, 1H), 3.42 (d, J=13.40Hz, 1H), 3.03-3.30 (m, 4H), 2.39-2.80 (m, 4H), 1.86-2.14 (m, 4H), 1.54-1.81 (m, 3H), 0.69-0.81 (m, 1H).
Example 1 (6) : (3R)-9-(4-chlorobenzyl)-1-propyl-3-(3-pyridinylmethyl)-1,4,9 triazaspiro[5.5] undecane-2,5-dione
TLC : Rf 0.20 (chloroform : methanol = 20 : 1);
NMR : δ 8.53 (dd, J=4.80, 1.70Hz, 1H), 8.49 (d, J=1.70Hz, 1H), 7.51-7.58 (m, 1 H), 7.18-7.32 (m, 5H), 5.73-5.83 (m, 1H), 4.22-4.32 (m, 1H), 3.47 (s, 2H), 3.20-3.44 (m, 3H), 3.09 (dd, J=14.00, 7.40Hz, 1H), 2.49-2.81 (m, 4H), 1.75-2.08 (m, 3H), 1.44-1.62 (m, 2H), 1.25-1.37 (m, 1H), 0.92 (t, J=7.30Hz, 3H).
Example 1 (7) : (3R)-9-(4-chlorobenzyl)-1-(cyclopropylmethyl)-3-(3-pyridinylmethyl)-1,4,9- triazaspiro[5.5]undecane-2,5-dione
TLC : Rf 0.20 (chloroform: methanol = 20 : 1);
NMR : δ 8.52 (dd, J=4.80, 1.50Hz, 1H), 8.50 (d, J=1.50Hz, 1H), 7.52-7.59 (m, 1H), 7.17-7.33 (m, 5H), 5.68-5.77 (m, 1H), 4.23-4.33 (m, 1H), 3.47 (s, 2H), 3.29-3.44 (m, 3H), 3.07(dd, J=14.40, 8.00Hz, 1H), 2.63-2.86 (m, 3H), 2.42-2.55 (m, 1H), 1.97-2.13 (m,2H), 1.82-1.93 (m, 1H), 1.49-1.59 (m, 1H), 0.85-1.01 (m, 1H), 0.48-0.57 (m, 2H), 0.36-0.45 (m, 2H).
Example 1 (8) : (3R)-9-(4-chlorobenzyl)-1-cyclobutyl-3-(3-pyridinylmethyl)-1,4,9-triazaspiro[5.5] undecane-2,5-dione
TLC : Rf 0.20 (chloroform : methanol = 20 : 1);
NMR: δ 8.49 (d, J=4.80Hz, 1H), 8.45 (s,1H), 7.52 (d, J=7.90Hz, 1H), 7.15-7.34 (m, 5H), 6.01-6.18 (m, 1H), 4.22-4.32 (m, 1H), 3.83-4.02 (m, 1H), 3.48(d, J=13.50Hz, 1H), 3.42 (d, J=13.50Hz, 1H), 3.29 (dd, J=13.90, 5.50Hz, 1H), 3.09-3.23 (m, 2H), 3.00 (dd, J=13.90, 3.80Hz, 1 H), 2.43-2.76 (m, 4H), 1.86-2.14 (m, 4H), 1.55-1.82 (m, 3H), 0.56-0.71 (m, 1H).
Example 1 (9): (3R)-9-(4-chlorobenzyl)-1-cyclopentyl-3-(3-pyridinylmethyl)-1,4,9-triazaspiro[5.5]undecane-2,5-dione
TLC : Rf 0.20 (chloroform : methanol = 20 : 1);
NMR : δ 8.50 (dd, J=4.80, 1.70Hz, 1H), 8.45 (d, J=1.70Hz, 1H), 7.48-7.55 (m, 1H), 7.16-7.30 (m, 5H), 6.05-6.13 (m, 1H), 4.20-4.28 (m, 1H), 3.54-3.72 (m, 1H), 3.49(d, J=13.40Hz, 1H), 3.44 (d, J=13.40Hz, 1H), 3.26 (dd, J=14.10, 5.30Hz, 1H), 2.98 (dd, J=14.10, 4.30Hz, 1H), 2.44-2.81 (m, 4H), 1.87-2.29 (m, 5H), 1.45-1.81 (m, 6H), 0.62-0.74 (m, 1H).
Example 1 (10) : (3R)-9-(4-chlorobenzyl)-3-(1H-indol-3-ylmethyl)-1-propyl-1,4,9-triazaspiro[5.5] undecane-2,5-dione
TLC : Rf 0.20 (chloroform: methanol = 20 : 1);
NMR : δ 8.17 (s, 1H), 6.98-7.76 (m, 9H), 5.57-5.74 (m, 1H), 4.24 (dd, J=9.8, 3.0Hz, 1H), 3.69 (dd, J=14.8, 3.0Hz, 1H), 3.37-3.57 (m, 3H), 3.19-3.37 (m, 1H), 2.98 (dd, J=14.8, 9.8Hz, 1H), 2.76-2.91 (m, 1H), 2.58-2.76 (m, 2H), 2.29-2.52 (m, 1H), 1.87-2.08 (m, 2H), 1.76-1.88 (m, 1H), 1.65-1.76 (m, 1H), 1.45-1.65 (m, 2H), 0.94 (t, J=7.4Hz, 3H).
Example 1 (11): (3R)-9-(4-chlorobenzyl)-1-(cyclopropylmethyl)-3-(1H-indol-3-ylmethyl)-1,4,9- triazaspiro[5.5]undecane-2,5-dione
TLC : Rf 0.20 (chloroform : methanol = 20 : 1);
NMR: δ 8.12-8.25 (m, 1H), 7.02-7.73 (m, 9H), 5.62-5.73 (m, 1H), 4.26 (dd, J=10.00, 3.60Hz, 1 H), 3.73 (dd, J=14.60, 3.60Hz, 1H), 3.32-3.53 (m, 4H), 2.81-3.04 (m, 2H), 2.59-2.80 (m, 2H), 2.25-2.40 (m, 1H), 1.94-2.20 (m, 2H), 1.80-1.93 (m, 2H), 0.90-1.05 (m, 1H), 0.37-0.59 (m, 4H).
Example 1 (12) : (3R)-9-(4-chlorobenzyl)-1-cyclobutyl-3-(1H-indol-3-ylmethyl)-1,4,9-triazaspiro [5.5]undecane-2,5-dione
TLC : Rf 0.20 (chloroform : methanol = 20 : 1);
NMR : δ 8.06-8.20 (m, 1H), 6.99-7.75 (m, 9H), 5.54-5.69 (m, 1H), 4.14-4.27 (m, 1H), 3.86-4.08 (m, 1H), 3.35-3.56 (m, 3H), 3.01-3.26 (m, 3H), 2.42-2.80 (m, 4H), 1.84-2.16 (m, 4H), 1.48-1.85 (m, 3H), 1.11-1.31 (m, 1H).
Example 1 (13) : (3R)-9-(4-chlorobenzyl)-1-cyclopentyl-3-(1H-indol-3-ylmethyl)-1,4,9-triazaspiro [5.5]undecane-2,5-dione

TLC : Rf 0.20 (chloroform : methanol = 20 : 1);
NMR : δ 8.08-8.29 (m, 1H), 6.99-7.76 (m, 9H), 5.55-5.78 (m, 1H), 4.06-4.28 (m, 1H), 3.60-3.83 (m, 1H), 3.34-3.62 (m, 3H), 3.05 (dd, J=14.40, 8.90Hz, 1H), 2.50-2.83 (m, 4H), 1.44-2.34 (m, 11H), 1.22-1.37(m, 1H).
Example 1 (14): 3-benzyl-9-(biphenyl-4-ylmethyl)-1-propyl-1,4,9-triazaspiro[5.5]undecane-2,5- dione
TLC : Rf 0.49 (chloroform : methanol = 10 : 1);
NMR ($CD_3OD$): δ 7.54-7.69 (m, 4H), 7.38-7.48 (m, 4H), 7.29-7.37 (m, 1H), 7.19-7.29 (m, 3H), 7.10-7.17 (m, 2H), 4.30-4.35 (m, 1H), 3.84 (s, 2H), 3.19-3.35 (m, 3H), 2.99-3.18 (m, 2H), 2.83-2.99 (m, 2H), 2.67-2.80 (m, 1H), 2.07-2.26 (m, 1H), 1.76-1.91 (m, 1H), 1.55-1.72 (m, 1H), 1.38-1.56 (m, 2H), 0.87(t, J=7.41Hz, 3H), 0.03-0.15 (m, 1H).
Example 1 (15) : 9-[4-(allyloxy)benzyl]-3-benzyl-1-propyl-1,4,9-triazaspiro[5.5]undecane-2,5- dione
TLC : Rf 0.54 (chloroform : methanol = 10 : 1);
NMR ($CD_3OD$) : δ 7.05-7.34 (m, 7H), 6.81-6.97 (m, 2H), 5.92-6.15 (m, 1H), 5.30-5.47 (m, 1H), 5.15-5.29 (m, 1H), 4.44-4.64 (m, 2H), 4.31 (t, J=4.12Hz, 1H), 3.52-3.70 (m, 2H), 3.19-3.35 (m, 2H), 2.99-3.19 (m, 2H), 2.82-2.98 (m, 2H), 2.65-2.78 (m, 1H), 2.53-2.64 (m, 1H), 1.98-2.17 (m, 1H), 1.73-1.85 (m, 1H), 1.40-1.65 (m, 3H), 0.88 (t, J=7.32Hz, 3H), 0.01-0.16 (m, 1H).
Example 1 (16) : 9-[4-cyanobenzyl]-3-benzyl-1-propyl-1,4,9-triazaspiro[5.5]undecane-2,5-dione
TLC : Rf 0.22 (chloroform : methanol = 30 : 1);
NMR ($CD_3OD$): δ 7.68 (d, J=8.42Hz, 2H), 7.48 (d, J=8.42Hz, 2H), 7.08-7.30 (m, 5H), 4.31 (t, J=4.21 Hz, 1H), 3.54-3.65 (m, 2H), 3.20-3.28 (m, 1H), 3.06-3.19 (m, 1H), 2.82-3.00 (m, 2H), 2.65-2.78 (m, 1H), 2.51-2.64 (m, 1 H), 2.38-2.47 (m, 1 H), 1.87-2.12(m, 2H), 1.70-1.80 (m, 1H), 1.39-1.60 (m, 3H), 0.89 (t, J=7.41 Hz, 3H), 0.07-0.16 (m, 1H).

Example 2 : 1,1- cyclohexane diethanol

To a solution of 1,1-cyclohexane diacetic acid (CAS No. 4355-11-7, 1.00g) in N, N-dimethylformamide (15mL) was added potassium carbonate (2.07g) and iodomethane (0.78mL), and the reaction mixture was stirred for 2days at room temperature. The reaction mixture was added by water, and extracted with a mixed solvent of ethyl acetate-hexane (1:3). The organic layer was washed with brine, dried over, and concentrated. To a solution of the obtained ester (1.06g) in tetrahydrofuran (20mL) was added lithium aluminium hydride (569mg), and the reaction mixture was stirred for 3hours at 0°C. To the reaction mixture was added sodium sulfate, after the insoluble substance was filtrated through Celite (trade name), the filtrate was concentrated to give the title compound (821 mg) having the following physical data.
TLC : Rf 0.26 (chloroform : methanol = 10:1).

Example 3 : 3-benzyl-3-azaspiro[5.5]undecane

To a solution of the compound prepared in Example 2 (790mg) in dichloromethane (15ml) was added triethylamine (224mL) and mesyl chloride (0.85mL), and the reaction mixture was stirred for 2hours at 0°C. To the reaction mixture was added a saturated aqueous solution of sodium hydrogen carbonate, and extracted with dichloromethane. The organic layer was washed with brine, dried over, and concentrated. Quantity of 700mg among the obtained dimesyl compound (1.41g) was mixed with benzylamine (0.70mL), and the reaction mixture was stirred for 4hours at 40°C. The reaction mixture was purified by column chromatography on silica gel (dichloromethane : methanol = 100 : 3) to give the compound of the present invention (220mg) having the following physical data.
TLC : Rf 0.25 (chloroform : methanol = 10 : 1);
NMR: δ 7.20-7.35 (m, 5H), 3.50 (s, 2H), 2.37 (t, J=5.5Hz, 4H), 1.46 (t, J=5.5Hz, 4H), 1.35-1.43 (m, 6H), 1.27-1.35 (m, 4H).

Example 4 : 3-azaspiro[5.5]undecane

To a solution of the compound prepared in Example 3 (100mg) in methanol (2ml) was added palladium hydroxide (20% wet, 20mg), and the reaction mixture was stirred for 4hours at room temperature under hydrogen ambient atmosphere. After the reaction mixture was filtrated through Celite (trade name), the filtrate was concentrated to give the title compound (53mg) having the following physical data.
TLC : Rf 0.32 (chloroform: methanol : 28 % aqueous solution of ammonia = 80 : 20 : 2).

Example 5 : 3-[4-(prop-2-yn-1-yloxy)benzyl]-3-azaspiro[5.5]undecane

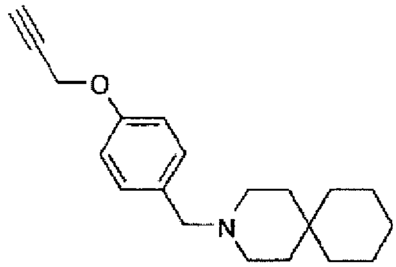

To a solution of the compound prepared in Example 4 (30mg) in dichloromethane (1ml) was added acetic acid (0.01 mL), 4-(prop-2-ynyloxy) benzaldehyde (41 mg) and sodium triacetoxyborohydride (62mg), and the reaction mixture was stirred for 3hours at room temperature. To a solution of the reaction mixture was added 1 N aqueous solution of sodium hydoxide, and extracted with dichloromethane. The organic layer was washed with brine, dried over, and concentrated. The obtained residue was purified by column chromatography on silica gel (dichloromethane : methanol = 50 : 2) to give the compound of the present invention (35mg) having the following physical data.
TLC : Rf 0.45 (chloroform : methanol = 10 : 1);
NMR : $\delta$ 7.25 (d, J=8.6Hz, 2H), 6.92 (d, J=8.6Hz, 2H), 4.68 (d, J=2.4Hz, 2H), 3.46 (s, 2H), 2.52(t, J=2.4Hz, 1 H), 2.37 (t, J=5.5Hz, 4H), 1.46 (t, J=5.5Hz, 4H), 1.35-1.43 (m, 6H), 1.25-1.35 (m, 4H).

Example 6 (1) ~ Example 6 (35)

By the same procedure as described in Example 1 (1) using the corresponding piperidone compounds instead of N-(4-chlorobenzyl)-4-piperidone, the corresponding amino acid compounds instead of N-(tert-butoxycarbonyl)-D-phenylalanine and the corresponding amine compounds instead of propylamine, the compounds of the present invention having the following physical data were obtained.
Example 6 (1):
3-benzyl-9-(3-methylbenzyl)-1-propyl-1,4,9-triazaspiro[5.5]undecane-2,5-dione HPLC retention time (min) : 3.27 (condition D);
MS (LC-MS, ESI, Pos., 20V): 420 $(M+H)^+$.
Example 6 (2) :
3-benzyl-1-(2-furylmethyl)-9-(3-methylbenzyl)-1,4,9-triazaspiro[5.5]undecane-2,5-dione
HPLC retention time (min): 3.31 (condition D);
MS (LC-MS, ESI, Pos., 20V) : 458 $(M+H)^+$.
Example 6 (3) :
3-benzyl-9-(4-methoxybenzyl)-1-propyl-1,4,9-triazaspiro[5.5]undecane-2,5-dione
HPLC retention time (min): 3.20 (condition D);
MS (LC-MS, ESI, Pos., 20V) : 436 $(M+H)^+$.
Example 6 (4) :
3-benzyl-9-(4-methoxybenzyl)-1-(3-phenylpropyl)-1,4,9-triazaspiro[5.5] undecane-2,5-dione
HPLC retention time (min) : 3.44 (condition D);
MS (LC-MS, ESI, Pos., 20V) : 512 $(M+H)^+$.
Example 6 (5):
3-benzyl-1-(2-furylmethyl)-9-(4-methoxybenzyl)-1,4,9-triazaspiro[5.5]undecane-2,5-dione
HPLC retention time (min): 3.24 (condition D);
MS (LC-MS, ESI, Pos., 20V) : 474 $(M+H)^+$.
Example 6 (6) :
3-benzyl-9-(4-chlorobenzyl)-1-(2-furylmethyl)-1,4,9-triazaspiro[5.5]undecane-2,5-dione
HPLC retention time (min): 3.31 (condition D);
MS (LC-MS, ESI, Pos., 20V) : 480, 478 $(M+H)^+$.
Example 6 (7) :
3-benzyl-9-(4-chlorobenzyl)-1-(2-methoxyethy)-1,4,9-triazaspiro[5.5]undecane-2,5-dione
HPLC retention time (min) : 3.21 (condition D);
MS (LC-MS, ESI, Pos., 20V) : 458, 456 $(M+H)^+$.
Example 6 (8) :
3-benzyl-9-hexyl-1-(2-methoxyethyl)-1 ,4, 9-triazaspiro[5.5]undecane-2,5-dione
HPLC retention time (min) : 3.23 (condition D);

MS (LC-MS, ESI, Pos., 20V) : 416 $(M+H)^+$.

Example 6 (9):

3-benzyl-1-(2-furylmethyl)-9-hexyl-1,4,9-triazaspiro[5.5]undecane-2,5-dione

HPLC retention time (min) : 3.35 (condition D);

MS (LC-MS, ESI, Pos., 20V) : 438 $(M+H)^+$.

Example 6 (10) :

3-benzyl-9-hexyl-1-propyl-1,4,9-triazaspiro[5.5]undecane-2,5-dione

HPLC retention time (min) : 3.30 (condition D);

MS (LC-MS, ESI, Pos., 20V) : 400 $(M+H)^+$.

Example 6 (11):

(3R)-9-(4-chlorobenzyl)-3-(4-hydroxybenzyl)-1-propyl-1,4,9-triazaspiro[5.5] undecane-2,5-dione

HPLC retention time (min) : 3.09 (condition D);

MS (LC-MS, ESI, Pos., 20V) : 458, 456 $(M+H)^+$.

Example 6 (12) :

(3R)-9-(4-chlorobenzyl)-3-(1-naphthylmethyl)-1-propyl-1,4,9-triazaspiro[5.5] undecane-2,5-dione

HPLC retention time (min) : 3.41 (condition D);

MS (LC-MS, ESI, Pos., 20V) : 492, 490 $(M+H)^+$.

Example 6 (13):

(3S)-3-(2-chlorobenzyl)-9-(4-chlorobenzyl)-1-propyl-1,4,9-triazaspiro[5.5] undecane-2,5-dione

HPLC retention time (min): 3.32 (condition D);

MS (LC-MS, ESI, Pos., 20V): 478, 476, 474 $(M+H)^+$.

Example 6 (14) :

(3R)-9-(4-chlorobenzyl)-3-(4-methoxybenzyl)-1-propyl-1,4,9-triazaspiro[5.5] undecane-2,5-dione

HPLC retention time (min) : 3.24 (condition D);

MS (LC-MS, ESI, Pos., 20V) : 472, 470 $(M+H)^+$.

Example 6 (15) :

3-benzyl-9-[2-(4-chlorophenyl)ethyl]-1-propyl-3 ,4, 9-triazaspiro[5. 5]undecane-2,5-dione

HPLC retention time (min) : 3.32 (condition D);

MS (LC-MS, ESI, Pos., 20V): 456, 454 $(M+H)^+$.

Example 6 (16) :

3-benzyl-9-[2-(4-chlorophenyl)ethyl]-1-(2-furylmethyl)-1,4,9-triazaspiro[5.5] undecane-2,5-dione

HPLC retention time (min): 3.37 (condition D);

MS (LC-MS, ESI, Pos., 20V): 494, 492 $(M+H)^+$.

Example 6 (17) :

3-benzyl-9-(4-chlorobenzyl)-1-cyclohexyl-1,4,9-triazaspiro[5.5]undecane-2,5-dione

HPLC retention time (min) : 3.40 (condition D);

MS (LC-MS, ESI, Pos., 20V) : 482, 480 $(M+H)^+$.

Example 6 (18) :

3-benzyl-9-(4-chlorobenzyl)-1-[2-(1H-imidazole-4-yl)ethyl]-1,4,9-triazaspiro[5.5]undecane-2,5-dione

HPLC retention time (min) : 3.01 (condition D);

MS (LC-MS, ESI, Pos., 20V) : 494, 492 $(M+H)^+$.

Example 6 (19) :

3-benzyl-9-(4-chlorobenzyl)-1-(tetrahydro-2-furanylmethyl)-1,4,9-triazaspiro [5.5]undecane-2,5-dione

HPLC retention time (min) : 3.24 (condition D);

MS (LC-MS, ESI, Pos., 20V): 484, 482 $(M+H)^+$.

Example 6 (20):

3-benzyl-9-(4-chlorobenzyl)-1-[2-(2-pyridinyl)ethyl]-1,4,9-triazaspiro[5.5] undecane-2,5-dione

HPLC retention time (min) : 3.03 (condition D);

MS (LC-MS, ESI, Pos., 20V) : 505, 503 $(M+H)^+$.

Example 6 (21);

3-benzyl-9-(4-chlorobenzyl)-1-(3-pyridinylmethyl)-1,4,9-triazaspiro[5.5] undecane-2,5-dione

HPLC retention time (min) : 3.00 (condition D);

MS (LC-MS, ESI, Pos., 20V) : 491, 489 $(M+H)^+$.

Example 6 (22) :

3-benzyl-9-(4-chlorobenzyl)-1-(2-methoxy-1-methylethyl)-1,4,9-triazaspiro[5.5]undecane-2,5-dione

HPLC retention time (min) : 3.23 (condition D);

MS (LC-MS, ESI, Pos., 20V) : 472, 470 $(M+H)^+$.

Example 6 (23) :

3-benzyl-1-sec-butyl-9-(4-chlorobenzyl)-1,4,9-triazaspiro[5.5]undecane-2,5-dione
HPLC retention time (min) : 3.31 (condition D);
MS (LC-MS, ESI, Pos., 20V) : 456, 454 $(M+H)^+$.
Example 6 (24):
3-benzyl-9-(4-chlorobenzyl)-1-(1-ethylpropyl)-1,4,9-triazaspiro[5.5]undecane-2,5-dione
HPLC retention time (min) : 3.38 (condition D);
MS (LC-MS, ESI, Pos., 20V) : 470, 468 $(M+H)^+$.
Example 6 (25) :
3-benzyl-9-(4-chlorobenzyl)-1-(1-methylbutyl)-1,4,9-triazaspiro[5.5]undecane-2,5-dione
HPLC retention time (min) : 3.40 (condition D);
MS (LC-MS, ESI, Pos., 20V) : 470, 468 $(M+H)^+$.
Example 6 (26) :
3-benzyl-9-(4-chlorobenzyl)-1-(2,2-dimethylpropyl)-1,4,9-triazaspiro[5.5] undecane-2,5-dione
HPLC retention time (min) : 3.43 (condition D);
MS (LC-MS, ESI, Pos., 20V) : 470, 468 $(M+H)^+$.
Example 6 (27) :
3-benzyl-9-(4-chlorobenzyl)-1-(2-fluoroethyl)-1,4,9-triazaspiro[5.5]undecane-2,5-dione
HPLC retention time (min): 3.21 (condition D);
MS (LC-MS, ESI, Pos., 20V): 446, 444 $(M+H)^+$.
Example 6 (28) :
3-benzyl-9-(4-chlorobenzyl)-1-(2-methoxyethyl)-1,4, 9-triazaspiro[5.5]undecane-2,5-dione
HPLC retention time (min) : 3.18 (condition D);
MS (LC-MS, ESI, Pos., 20V) : 458, 456 $(M+H)^+$
Example 6 (29) :
3-benzyl-9-(4-chlorobenzyl)-1-(cyclopropylmethyl)-1,4,9-triazaspiro[5.5] undecane-2,5-dione
HPLC retention time (min) : 3.30 (condition D);
MS (LC-MS, ESI, Pos., 20V) : 454, 452 $(M+H)^+$.
Example 6 (30) :
3-benzyl-9-(4-chlorobenzyl)-1-ethyl-1,4,9-triazaspiro[5.5]undecane-2,5-dione
HPLC retention time (min) : 3.17 (condition D);
MS (LC-MS, ESI, Pos., 20V): 428, 426 $(M+H)^+$.
Example 6 (31) :
3-benzyl-9-(4-chlorobenzyl)-1-[3-(methylthio)propyl]-1,4,9-triazaspiro[5.5] undecane-2,5-dione
HPLC retention time (min) : 3.30 (condition D);
MS (LC-MS, ESI, Pos., 20V) : 488, 486 $(M+H)^+$.
Example 6 (32) :
3-benzyl-9-(4-chlorobenzyl)-1-isopropyl-1,4,9-triazaspiro[5.5]undecane-2,5-dione
HPLC retention time (min) : 3.25 (condition D);
MS (LC-MS, ESI, Pos., 20V) : 442, 440 $(M+H)^+$.
Example 6 (33) :
3-benzyl-9-(4-chlorobenzyl)-1-(2-propyn-1-yl)-1,4,9-triazaspiro[5.5]undecane-2,5-dione
HPLC retention time (min) : 3.19 (condition D);
MS (LC-MS, ESI, Pos., 20V) : 438, 436 $(M+H)^+$.
Example 6 (34) :
3-benzyl-9-(4-chlorobenzyl)-1-(3-methoxypropyl)-1,4,9-triazaspiro[5.5] undecane-2,5-dione
HPLC retention time (min) : 3.19 (condition D);
MS (LC-MS, ESI, Pos., 20V): 472, 470 $(M+H)^+$.
Example 6 (35):
3-benzyl-9-(4-chlorobenzyl)-1-(2-thienylmethyl)-1,4,9-triazaspiro[5.5]undecane-2,5-dione
HPLC retention time (min) : 3.36 (condition D);
MS (LC-MS, ESI, Pos., 20V) : 496, 494 $(M+H)^+$.

Example 7 : 1-(4-chlorobenzyl) piperidin-4-one

A solution of 4-chlorobenzyl chloride (40.3g), 4-piperidone hydrochloride monohydrate (42.2g) and potassium carbonate (138g) in acetonitrile (600mL) was stirred at 60°C overnight. After the reaction mixture was cooled to room temperature, and filtered. The filtered solid was washed with acetonitrile. The filtrate and washing were gathered up, and concentrated in vacuum condition. The obtained residue was dissolved in ethyl acetate, washed with water and

brine. After the organic layer was dried over sodium sulfate, and filtered. The filtrate was concentrated to give the title compound (55.7g) having the following physical data.
NMR : δ 7.31 (s, 4H), 3.58 (s, 2H), 2.73 (d, J=6.1Hz, 4H), 2.49 (d, J=6.1 Hz, 4H);
MS (LC-MS, ESI, Pos., 20V): 226, 224 $(M+H)^+$.

Example 8 : 4-allyl-1-(4-chlorobenzyl)-4-propylaminopiperidine

A solution of the activated molecular sieves 4A (8g), 1-(4-chlorobenzyl) piperidin-4-one (8.95g) and n-propylamine (2.96mL) in toluene (80mL) was stirred for 22hours at 80°C. Furthermore the reaction mixture was added n-propylamine (1.64mL) and the molecular sieves 4A (2g). After 2hours, it was cooled to room temperature. The reaction mixture was filtrated through Celite, the filtered solid was washed with dry toluene. The filtrate and washing were gathered up, and concentrated in vacuum condition. The obtained residue was dissolved in dry toluene (80mL). To the obtained solution was dropped into the ether solution of allyl magnesium bromide (1.0M, 48mL) for 20minutes on ice bath. After the reaction mixture was raised to room temperature for 30minutes, cooled by the ice bath again. After the reaction mixture was added a saturated aqueous solution of ammonium chloride, diluted with ethyl acetate. The organic layer was washed with an aqueous solution of ammonium chloride, extracted with 1 M aqueous solution of sodium dihydrogenphosphate. After the obtained acidic extracts was adjusted more than pH10 with 4M aqueous solution of sodium hydroxide, extracted with methylene chloride. After the organic layer was dried over sodium sulfate, and filtered. The filtrate was concentrated in vacuum condition to give the title compound (8.63g) having the following physical data.
HPLC retention time (min): 1.96 (condition A);
NMR : δ 7.28-7.23 (m, 4H), 5.79 (ddt, J=9.9, 17.1, 7.4Hz, 1 H), 5.09 (d, J=9.9Hz, 1H), 5.04 (d, J=17.1Hz, 1H), 3.45 (s, 2H), 2.47-2.33 (m, 4H), 2.42 (t, J=7.0Hz, 2H), 2.16 (d, J=7.4Hz, 2H), 1.59-1.50 (m, 4H), 1.44 (m, 2H), 1.40-1.00 (m, 1 H), 0.93 (t, J=7.4Hz, 3H);
MS (LC-MS, ESI, Pos., 20V): 309, 307 $(M+H)^+$.

Example 9 :

9-(4-chlorobenzyl)-3-(2-phenylethyl)-1-propyl-1,3,9-triazaspiro[5.5]undec-4-en-2-one

To a solution of the compound prepared in Example 8 in dichloroethane (0.62M, 0.80mL) was added a solution of 2-phenylethyl isocyanate in dichloroethane (1.0M, 0.55mL). The mixture was stirred at room temperature overnight, and furthermore stirred for 6-8days at 50°C. The reaction mixture was cooled to room temperature, and concentrated in vacuum condition. After the obtained residue was dissolved in acetonitrile (4.3mL), added and mixed acetic acid (0.143mL), N-methylmorpholine N-oxide (0.25M acetonitrile solution, 3.0mL) and osmium tetroxide (0.080M aqueous solution, 0.090mL). The mixture was left for 16hours at room temperature, and added 0.5M sodium periodate (1.5mL). The mixture was stirred for 40minutes, and added a mixed solution of a saturated solution of sodium bicarbonate-chloroform. The organic layer was dried over sodium sulfate, and filtered. The obtained filtrate was added trifluoroacetic acid (0.19mL). The reaction mixture was left at room temperature overnight, and concentrated in vacuum condition. After the obtained residue was dissolved in chloroform, and concentrated in vacuum condition. The obtained residue was purified by reversed-phase HPLC (Gemini C18 column 20X50mm made in Phenomenex Inc., by the gradient of acetonitrile-10mM aqueous solution of ammonium carbonate) to give the compound of the present invention having the following physical data.
HPLC retention time (min): 3.35 (condition D);
MS (LC-MS, ESI, Pos., 20V) : 875 $(2M+H)^+$, 440, 438 $(M+H)^+$.

Example 9 (1) ~ Example 9 (8)

By the same procedure as described in Example 8→Example 9 using the corresponding amine compounds instead of propylamine and the corresponding isocyanate compounds instead of 2-phenylethyl isocyanate, the compounds of the present invention having the following physical data were obtained.
Example 9 (1):
3-benzyl-9-(4-chlorobenzyl)-1-(cyclopropylmethyl)-1,3,9-triazaspiro[5.5]undec-4-en-2-one
HPLC retention time (min) : 3.34 (condition B);
MS (LC-MS, ESI, Pos., 20V) : 871 $(2M+H)^+$, 438, 436 $(M+H)^+$.
Example 9 (2) :
3-benzyl-9-(4-chlorobenzyl)-1-propyl-1,3,9-triazaspiro[5.5]undec-4-en-2-one
HPLC retention time (min) : 3.30 (condition B);
MS (LC-MS, ESI, Pos., 20V) : 847 $(2M+H)^+$, 426, 424 $(M+H)^+$.

Example 9 (3) :
9-(4-chlorobenzyl )-1-(cyclopropylmethyl)-3-(2-phenylethyl)-1,3,9-triazaspiro [5.5]undec-4-en-2-one
HPLC retention time (min) : 3.34 (condition B);
MS (LC-MS, ESI, Pos., 20V) : 871 $(2M+H)^+$, 438, 436 $(M+H)^+$.
Example 9 (4);
9-(4-chlorobenzyl)-1-cyclobutyl-3-(2-phenylethyl)-1,3,9-triazaspiro[5.5]undec-4-en-2-one
HPLC retention time (min) : 3.41 (condition B);
MS (LC-MS, ESI, Pos., 20V) : 452, 450 $(M+H)^+$.
Example 9 (5) :
3-butyl-9-(4-chlorobenzyl)-1-propyl-1,3,9-triazaspiro[5.5]undec-4-en-2-one
HPLC retention time (min) : 3.27 (condition B);
MS (LC-MS, ESI, Pos., 20V) : 781, 779 $(2M+H)^+$, 392, 390 $(M+H)^+$.
Example 9 (6):
9-(4-chlorobenzyl)-3-isopropyl-1-propyl-1,3,9-triazaspiro[5.5]undec-4-en-2-one
HPLC retention time (min) : 3.16 (condition B);
MS (LC-MS, ESI, Pos., 20V) : 378, 376 $(M+H)^+$.
Example 9 (7) :
9-(4-chlorobenzyl)-1-(cyclopropylmethyl)-3-isopropyl-1,3,9-triazaspiro[5.5] undec-4-en-2-one
HPLC retention time (min) : 3.18 (condition B);
MS (LC-MS, ESI, Pos., 20V) : 777, 775 $(2M+H)^+$, 390, 388 $(M+H)^+$.
Example 9 (8) :
3-butyl-9-(4-chlorobenzyl)-1-(cyclopropylmethyl)-1,3,9-triazaspiro[5.5]undec-4-en-2-one
HPLC retention time (min) : 3.29 (condition B);
MS (LC-MS, ESI, Pos., 20V) : 805, 803 $(2M+H)^+$, 404, 402 $(M+H)^+$.

Example 10: 4-allyl-1-(4-chlorobenzyl)-4-cyclopentylaminopiperidine

A solution of the activated molecular sieves 4A (8g), 1-(4-chlorobenzyl) piperidin-4-one (8.95g) and cyclopentylamine (4.26g) in toluene (80mL) was stirred for 24hours at 80°C, and furthermore stirred for 30minutes at 100°C. After the reaction mixture was cooled to room temperature, filtered through Celite, and the filtered solid was washed with dry toluene. The filtrate and washing were gathered up, and concentrated in vacuum condition. The obtained residue was dissolved in dry toluene (80mL). To the obtained solution was dropped into the ether solution of allyl magnesium bromide (1.0M, 48mL) for 20minutes on ice bath. After the reaction mixture was raised to room temperature for an hour, cooled by the ice bath again. After the reaction mixture was added a saturated aqueous solution of ammonium chloride, diluted with ethyl acetate. The organic layer was washed with an aqueous solution of ammonium chloride, extracted with 1M aqueous solution of sodium dihydrogenphosphate. After the obtained acidic extracts was adjusted more than pH10 with 4M aqueous solution of sodium hydroxide, extracted with methylene chloride. After the organic layer was dried over sodium sulfate, and filtered. The filtrate was concentrated in vacuum condition to give the title compound (8.92g) having the following physical data.
HPLC retention time (min): 2.11 (condition A);
NMR : $\delta$ 7.31-7.22 (m, 4H), 5.81 (ddt, J=10.0, 17.1, 7.3Hz, 1H), 5.09 (d, J=10.0Hz, 1H), 5.04 (d, J = 17, 1Hz, 1H), 3.44 (s, 2H), 3.08 (m, 1 H), 2.48-2.33 (m, 4H), 2.21 (d, J=7.3Hz, 2H), 1.88-1.78 (m, 2H), 1.72-1.61 (m, 2H), 1.54 (m, 2H), 1.53-1.43 (m, 2H), 1.29-1.19 (m, 2H), 1.40-0.95 (m, 1 H);
MS (LC-MS, ESI, Pos., 20V): 335, 333 $(M+H)^+$.

Example 11 3-butyl-9-(4-chlorobenzyl)-1-cyclopentyl-1,3,9-triazaspiro[5.5]undecan-2-one

To a solution of the compound prepared in Example 10 in dichloroethane (0.62M, 0.80mL) was added a solution of n-butyl isocyanate in dichloroethane (1.0M, 0.55mL). The mixture was stirred at room temperature overnight, and furthermore stirred for 6-8days at 50°C. The reaction mixture was cooled to room temperature, and concentrated in vacuum condition. After the obtained residue was dissolved in acetonitrile (4.3mL), added and mixed acetic acid (0.143mL), N-methylmorpholine N-oxide (0.25M acetonitrile solution, 3.0mL) and osmium tetroxide (0.080M aqueous solution, 0.090mL). The mixture was left for 16hours at room temperature, and added 0.5M sodium periodate (1.5mL). The mixture was stirred for 40minutes, and added a mixed solution of a saturated solution of sodium bicarbonate-chloroform. The organic layer was dried over sodium sulfate, and filtered. The obtained filtrate was added trifluoroacetic acid (0.19mL). The reaction mixture was left at room temperature overnight, and concentrated in vacuum condition. The obtained residue was dissolved in chloroform (4mL). To the obtained solution was added trifluoroacetic acid (0.19mL) and triethylsilane (0.40mL), and stirred at room temperature overnight. The reaction mixture was concentrated in vacuum

condition. The obtained residue was purified by reversed-phase HPLC (Gemini C18 column 20X50mm made in Phenomenex Inc., by the gradient of acetonitrile-10mM aqueous solution of ammonium carbonate) to give the compound of the present invention having the following physical data.
HPLC retention time (min) : 3.28 (condition D);
MS (LC-MS, ESI, Pos., 20V) : 418 $(M+H)^+$.

Example 11 (1) ~ Example 11 (11)

By the same procedure as described in Example 10→Example 11 using the corresponding amine compounds instead of cyclopentylamine and the corresponding isocyanate compounds instead of n-butyl isocyanate, the compounds of the present invention having the following physical data were obtained.
Example 11 (1):
3-benzyl-9-(4-chlorobenzyl)-1-(cyclopropylmethyl)-1,3,9-triazaspiro[5.5] undecan-2-one
HPLC retention time (min) : 3.24 (condition B);
MS (LC-MS, ESI, Pos., 20V) : 875 $(2M+H)^+$ 438 $(M+H)^+$.
Example 11 (2) :
3-benzyl-9-(4-chlorobenzyl)-1-(cyclopropylmethyl)-1,3,9-triazaspiro[5.5] undecan-2-one
HPLC retention time (min) : 3.25 (condition B);
MS (LC-MS, ESI, Pos., 20V) : 875 $(2M+H)^+$, 440, 438 $(M+H)^+$.
Example 11 (3) :
9-(4-chlorobenzyl)-3-(2-phenylethyl)-1-propyl-1,3,9-triazaspiro[5.5]undecan-2-one
HPLC retention time (min) : 3.25 (condition B);
MS (LC-MS, ESI, Pos., 20V): 879 $(2M+H)^+$, 442, 440 $(M+H)^+$.
Example 11 (4):
9-(4-chlorobenzyl)-1-cyclobutyl-3-(2-phenylethyl)-1,3,9-triazaspiro[5.5] undecan-2-one
HPLC retention time (min) : 3.32 (condition B);
MS (LC-MS, ESI, Pos., 20V) : 454, 452 $(M+H)^+$.
Example 11 (5) :
9-(4-chlorobenzyl)-1-cyclopentyl-3-(2-phenylethyl)-1,3,9-triazaspiro[5.5] undecan-2-one
HPLC retention time (min) : 3.37 (condition B);
MS (LC-MS, ESI, Pos., 20V) : 468, 466 $(M+H)^+$.
Example 11 (6) :
9-(4-chlorobenzyl)-3-[(1R)-1-phenylethyl]-1-propyl-1,3,9-triazaspiro[5.5] undecan-2-one
HPLC retention time (min) : 3.24 (condition B);
MS (LC-MS, ESI, Pos., 20V): 879 $(2M+H)^+$, 442, 440 $(M+H)^+$.
Example 11 (7) :
9-(4-chlorobenzyl)-3-[(1S)-1-phenylethyl]-1-propyl-1,3,9-triazaspiro[5.5] undecan-2-one
HPLC retention time (min): 3.26 (condition B);
MS (LC-MS, ESI, Pos., 20V) : 881, 879 $(2M+H)^+$, 442, 440 $(M+H)^+$.
Example 11 (8):
3-butyl-9-(4-chlorobenzyl)-1-(cyclopropylmethyl)-1,3,9-triazaspiro[5.5] undecan-2-one
HPLC retention time (min) : 3.20 (condition B);
MS (LC-MS, ESI, Pos., 20V): 406, 404 $(M+H)^+$.
Example 11 (9) :
9-(4-chlorobenzyl)-1-(cyclopropylmethyl)-3-[(1S)-1-phenylethyl]-1,3,9-triazaspiro[5.5]undecan-2-one
HPLC retention time (min) : 3.29 (condition B);
MS (LC-MS, ESI, Pos., 20V) : 905, 903 $(2M+H)^+$, 454, 452 $(M+H)^+$.
Example 11 (10) :
3-butyl-9-(4-chlorobenzyl)-1-cyclobutyl-1,3,9-triazaspiro[5.5]undecan-2-one
HPLC retention time (min) : 3.24 (condition B);
MS (LC-MS, ESI, Pos., 20V) : 406, 404 $(M+H)^+$.
Example 11 (11) :
9-(4-chlorobenzyl)-3-isopropyl-1-propyl-1,3,9-triazaspiro[5.5]undecan-2-one
HPLC retention time (min) : 3.06 (condition B);
MS (LC-MS, ESI, Pos., 20V) : 380, 378 $(M+H)^+$.

Example 12 : 4-allyl-1-(4-chlorobenzyl)-4-(cyclopropylmethylamino) piperidine

A solution of the activated molecular sieves 4A (8g), 1-(4-chlorobenzyl) piperidin-4-one (8.95g) and cyclopropylmethylamine (3.56g) in toluene (80mL) was stirred for 22hours at 80°C, and the reaction mixture was cooled to room temperature. The reaction mixture was filtered through Celite, and the filtered solid was washed with dry toluene. The filtrate and washing were gathered up, and concentrated in vacuum condition. The obtained residue was dissolved in dry toluene (80mL). To the obtained solution was dropped into the ether solution of allyl magnesium bromide (1.0M, 48mL) for 20minutes under the ice bath. After the reaction mixture was raised to room temperature for 30minutes, cooled by the ice bath again. After the reaction mixture was added a saturated aqueous solution of ammonium chloride, diluted with ethyl acetate. The organic layer was washed with an aqueous solution of ammonium chloride, extracted with 1 M aqueous solution of sodium dihydrogenphosphate. After the obtained acidic extracts was adjusted more than pH10 with 4M aqueous solution of sodium hydroxide, extracted with methylene chloride. After the organic layer was dried over sodium sulfate, and filtered. The filtrate was concentrated in vacuum condition to give the title compound (8.82g) having the following physical data.
NMR : δ 7.31-7.23 (m, 4H), 5.80 (ddt, J=10.1, 17.1, 7.4Hz, 1H), 5.09 (d, J=10.1Hz, 1H), 5.03 (d, J=17.1 Hz, 1H), 3.45 (s, 2H), 2.48-2.32 (m, 4H), 2.32 (d, J=6.8Hz, 2H), 2.14 (d, J=7.4Hz, 2H), 1.59-1.48 (m, 4H), 1.50-0.95 (m, 1H), 0.97-0.85 (m, 1H), 0.50-0.41 (m, 2H), 0.08 (m, 2H);
MS (LC-MS, ESI, Pos., 20V) : 321, 319 $(M+H)^+$.

Example 13 : N-(4-allyl-1-(4-chlorobenzyl)piperidin-4-yl)-N-(cyclopropylmethyl)acrylamide

To a solution of the compound prepared in Example 12 (290mg) in methylene chloride (10mL) was added triethylamine (1.0mL) and acryloyl chloride (0.22mL) on ice bath. After the reaction mixture was stirred for an hour at room temperature, and added 10% aqueous solution of sodium bicarbonate and methylene chloride. The organic layer was dried over sodium sulfate, and filtered. The filtrate was concentrated in vacuum condition. The obtained residue was purified by column chromatography on silica gel (methylene chloride→0.5% methanol-methylene chloride mixture) to give the title compound (231 mg) having the following physical data.
HPLC retention time (min) : 1.44 (condition A);
MS (LC-MS, ESI, Pos., 20V): 374, 372 $(M+H)^+$.

Example 14 :

9-(4-chlorobenzyl)-1-(cyclopropylmethyl)-1,9-diazaspiro[5.5]undec-3-en-2-one

To a solution of the compound prepared in Example 13 (105mg) in methylene chloride (20mL) was added titanium(IV) tetraisopropoxide (0.168mL) under argon ambient atmosphere. After the reaction mixture was refluxed for an hour, cooled to room temperature. After the reaction mixture was added benzylidene-bis(tricyclohexylphosphine) dichlororuthenium (Grubbs ruthenium catalyst, 23mg), the mixture was refluxed overnight under argon ambient atmosphere. The reaction mixture was cooled to room temperature, and concentrated in vacuum condition. The obtained residue was purified by column chromatography on silica gel (1% methanol-methylene chloride mixture →2% methanol-methylene chloride mixture) to give the compound of the present invention (75mg) having the following physical data.
HPLC retention time (min): 1.51 (condition A);
NMR : δ 7.29-7.20 (m, 4H), 6.40-6.34 (m, 1H), 5.95 (d, J=7.8Hz, 1 H), 3.47 (s, 2H), 3.38 (d, J=6.6Hz, 2H), 2.76 (d, J=9.0Hz, 2H), 2.49 (m, 2H), 2.06 (m, 4H), 1.79 (d, J=10.0Hz, 2H), 0.98 (m, 1H), 0.51-0.37 (m, 4H);
MS (LC-MS, ESI, Pos., 20V) : 347, 345 $(M+H)^+$.

Example 15 : 3-benzyl-9-(4-chlorobenzyl)-1-(cyclopropylmethyl)-1,9-diazaspiro[5.5]undecan-2-one

To a solution of the compound prepared in Example 14 (80mg) in tetrahydrofuran (5mL) was added L- selectride (1.0M tetrahydrofuran solution, 0.255mL) on ice bath. The mixture was stirred for 15minutes at room temperature. The mixture was added benzyl bromide (0.030mL) at room temperature, and furthermore added lithium bis(trimethylsilyl) amide (1.0M tetrahydrofuran solution, 0.24mL) at 0 °C. After the mixture was stirred for 10minutes at room temperature, added 1.0M aqueous solution of sodium bicarbonate, and concentrated in vacuum condition. The obtained residue was purified by column chromatography on silica gel (methylene chloride→1% methanol-methylene chloride mixture) to give the compound of the present invention (17mg) having the following physical data.
HPLC retention time (min) : 3.32 (condition D);
MS (LC-MS, ESI, Pos., 20V): 875, 873 $(2M+H)^+$, 439, 437 $(M+H)^+$.
Example 15 (1) :

3-benzyl-9-(4-chlorobenzyl)-1-propyl-1,9-diazaspiro[5.5]undecan-2-one

By the same procedure as described in Example 12→Example 13→ Example 14→Example 15 using n-propylamine instead of cyclopropylmethylamine, the compound of the present invention having the following physical data was obtained.
HPLC retention time (min) : 3.31 (condition C);
MS (LC-MS, ESI, Pos., 20V) : 851, 849 $(2M+H)^+$, 427, 425 $(M+H)^+$.

BIOLOGICAL EXAMPLES

It was proved by, for example, the following experiments that the compound of the present invention has the antagonistic activity against CXCR3 and the functional inhibitory activity against effector cell.

Total operation was based on basic biological procedure, was utilized a conventional method. In addition, the measuring method of the present invention is added the enhancement of the measuring accuracy and/or the improvement of the determination sensibility to evaluate the compound of the present invention as follows. A detailed experiment method was shown in the following.

Biological Example 1 : An inhibitory experiment against the binding of IP-10 and CXCR3 (the activity of that Ca ion ($Ca^{2+}$) is raised in transient and induced by IP-10)

The CHO cell (CXCR3/CHO cell) which overexpresses human CXCR3 in stable was suspended with Ham's F-12 culture medium and FBS (10%), was rolled up in 96well plate to become $3.0\times10^4$cell/well. After the plate was incubated for one day at 37°C, removed the cultural supernatant, added Ham's F-12 culture media (Fura-2AM (5 μM), Probenecid (2.5mM) and HEPES (20mM; pH7.4) were included) to 80 μl/well, incubated for one hour at 37°C in light exclusion state. After it was washed with a solution of 1×Hanks /HEPES (20mM ; pH7.4) twice, and added the same solution to 100 μl/well. Against the CXCR3/CHO cell which took in this Fura-2AM, at the time of course for 3minutes after the addition of a test compound, the recombination human IP -10 (PeproTech) was diluted by a solution of 1 ×Hanks /HEPES (20mM ; pH7.4) to become 30nM final concentration. The transient rise of the intracellular $Ca^{2+}$ concentration induced by human IP-10 was measured by means of $Ca^{2+}$ detector for 96well (made in Hamamatsu Photonics), and the inhibitory rate (%) of the test compound was calculated by the following calculation formula.

$$\text{The rate of inhibition} = \frac{(Ec - Ea)}{Ec} \times 100$$

Ec : The measured value of the $Ca^{2+}$ transient rise induced by IP -10
Ea : The measured value of the $Ca^{2+}$ transient rise induced by IP -10 when a test compound was added

As a result, the compound of the present invention showed the inhibition of more than 50% in 10 μM with the activity of that $Ca^{2+}$ was raised in transient and induced by IP-10. For example, the $IC_{50}$ value of the compound prepared in Example 1 (1), Example 5, Example 9, Example 11 and Example 15 was 0.14, 3.2, 3.4, 2.1 and 1.1 μM respectively.

Biological Example 2 : The effect as opposed to the action of the chemotaxis acceleration by IP-10 or I-TAC with the use of the human Th1 differentiated cell 2-1 : The Cellular preparation

Blood samples were collected from a human normal volunteer, using a heparinized syringe. The peripheral blood mononucleosis (PBMC) was isolated by means of the centrifuge separation method of the density that the gradient depended on with the use of the centrifugation vessel (Lympho prep tube(made in Nycomed Pharma)). The human CD4-positive T cell was isolated by the negative selection method with the use of the CD4 subset column kit of the human T Cell from PBMC. The following experiment was done by the use of this cell.

To the 24 well plate precoated by anti CD3 antibody (2 μg/mL) was disseminated the human CD4-positive T cell with the cellular density of $3\times10^6$cells/2mL/well, and incubated for 3days at 37°C by the $CO_2$ incubator in the existence of anti CD28 antibody (1 μg/mL), IL-2 (4ng/mL), IL-12 (5ng/mL) and anti IL-4 antibody (1 μg/mL). The 24 well plate coating by anti human CD3 antibody used here was made in OKT-3 (2 μg/mL) which was anti human CD3 antibody by the coating for 2hours at 37°C. After the cell was collected and washed, was suspended with RPMI1640 cultural medium (10mmol/L HEPES/1vol%, Antibiotic-Antimycotic) included 10 vol % fetal bovine serum. The well plate uncoated by anti CD3 antibody was transfered the cell with the cellular density of $3\times10^6$ cells/2 mL/well, incubated for 5days at 37°C by the $CO_2$ incubator in the existence of IL-2 (1 ng/mL), and the human Th1 differentiated cell was prepared. These serial irritation may repeat themselves several times.

2-2 : The chemotaxis in vitro

To the lower chamber of trance well was added 600 μL of 300 μL culture medium stated above including IP-10 or I-TAC inclusion solution (30nmol/L), and 300 μL culture medium stated above including the subject medicinal solution of the double concentration of the final concentration (dimethyl sulfoxide final concentration 0.3%) in total, attached the filter. 300 μL culture medium stated above including the chemokine non-inclusion solution (dimethyl sulfoxide final concentration 0.3%) was added to the group of control. To the higher was added 100 μL of 50 μL solution of the human Th1 differentiated cell ($1\times10^6$cells/well) prepared and 50 μL culture medium stated above including the subject medicinal solution of the double concentration of the final concentration in total, left for 90minutes at 37°C by the $CO_2$ incubator. After the reaction, the cellular fluid in the higher chamber was aspirated, added 20 μmol/L solution of ethylenediaminetetraacetic acid tetrasodium salt /phosphate buffered saline to 100 μL, and reacted for 30minutes 4°C. Subsequently, after the reaction solution was centrifuged for 5minutes in 1000rpm, dropped the cell which was adhered to underside of the filter in the lower chamber, the filter was removed, 600 μL of the lower chamber liquid was moved the another vessel, the analysis was done by means of FACS Calibur (trade name, made in Becton Dickinson company), and the cell population was counted. The value that deducted the number of the counts of the group of control (chemokine non-inclusion solution) from those of the group that the compound was additive-free was assumed 100%, and the activity that the compound inhibited the chemotaxis was evaluated.

As a result, the compound of the present invention showed the inhibition activity more than 50% in 30 μM.

FORMULATION EXAMPLES

Formulation Example 1:

We admixed (3R)-3-benzyl-9-(4-chlorobenzyl)-1-propyl-1,4,9-triazaspiro[5.5]undecane-2,5-dione (100g), carcium carboxymethyl cellulose (20.0g), magnesium stearate (10.0g) and microcrystalline cellulose (870g) in a conventional manner, punched them out to give 10000 tablets each containing 10 mg of active ingredient.

Formulation Example 2:

We admixed (3R)-3-benzyl-9-(4-chlorobenzyl)-1-propyl-1,4,9-triazaspiro[5.5]undecane-2,5-dione (200g), mannitol (2kg) and distilled water (50L) in a conventional manner. Then the solution was filtered through a dustproofing filter, and then 5 ml aliquots were charged into ampoules, which were autoclaved to give 10000 ampoules each containing 20mg of active ingredient.

INDUSTRIAL APPLICABILITY

The compound used for a CXCR3 antagonist in the present invention is useful as a preventive, therapeutic and/or progression-suppressing agent for a CXCR3-related disease, because it has CXCR3 antagonism. Therefore, the present invention is useful as medicament.

**Claims**

**1.** A CXCR3 antagonist comprising a compound represented by formula (I):

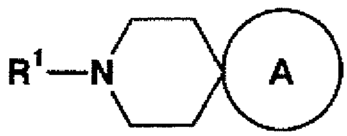

(I)

wherein ring A is 5- to 8-membered cyclic group which may be condensed with C3-8 mono-carbocyclic group which may have a substituent(s) or 3- to 8-membered mono-heterocyclic group which may have a substituent(s), which may have a further substituent(s);
$R^1$ is a hydrogen atom, aliphatic hydrocarbon which may have a substituent(s) or a cyclic group which may have a substituent(s)),
a salt thereof, a quaternary ammonium salt thereof, an N-oxide form thereof or a solvate thereof, or a prodrug thereof.

**2.** The CXCR3 antagonist according to claim 1, wherein the compound represented by formula (I):

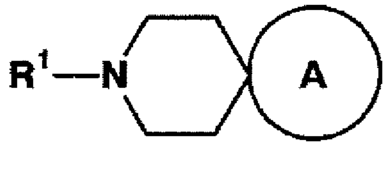

(I)

wherein all symbols have the same meanings as those described in the claim 1 is a compound represented by formula (II):

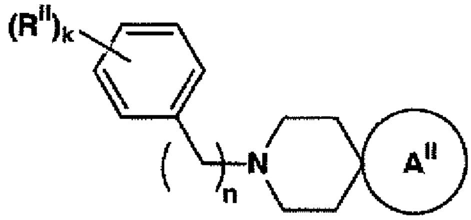

(II)

wherein ring $A^{II}$ is 6-membered mono-carbocyclic group which may have a substituent(s) or 6-membered mono-heterocyclic group which may have a substituent(s), and n is an integer of 1 to 4, and $R^{II}$ is substituent, and k is 0 or an integer of 1 to 5, and plural of $R^{II}$ may be same or different when k is 2 or more).

**3.** The CXCR3 antagonist according to claim 2, wherein n is 1.

**4.** The CXCR3 antagonist according to claim 2, wherein ring $A^{II}$ is cyclohexane ring which may have a substituent(s), piperazine ring which may have a substituent(s), tetrahydropyrimidine ring which may have a substituent(s), perhydropyrimidine ring which may have a substituent(s), tetrahydropyridine ring which may have a substituent(s) or piperidine ring which may have a substituent(s).

**5.** The CXCR3 antagonist according to claim 2, wherein $R^{II}$ is a chlorine atom, benzene ring which may have a substituent(s), methyl, methoxy, allyloxy, propargyloxy or cyano.

**6.** The CXCR3 antagonist according to claim 2, wherein the compound represented by formula (II):

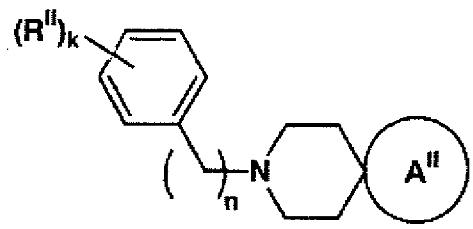

(II)

wherein all symbols have the same meanings as those described in the claim 2 is a compound represented by formula (II-A):

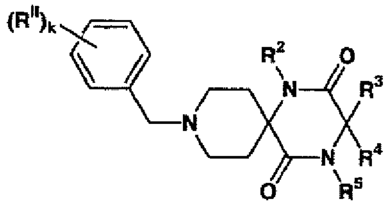

(II-A)

wherein, $R^2$, $R^3$, $R^4$, and $R^5$ are each independently a hydrogen atom, aliphatic hydrocarbon which may have a substituent(s), hydroxy which may be protected, carboxy which may be protected, carbamoyl which may have a substituent(s) or cyclic group which may have a substituent(s), and the other symbols have the same meanings as those described in the claim 2.

7. The CXCR3 antagonist according to claim 6,
wherein $R^{II}$ is a chlorine atom, benzene ring which may have a substituent(s), methyl, methoxy, allyloxy, propargyloxy, or cyano;
$R^2$ is propyl, cyclopropylmethyl, cyclobutyl or cyclopentyl;
$R^3$ is benzyl which may have a substituent(s), pyridylmethyl which may have a substituent(s) or indolylmethyl which may have a substituent(s);
$R^4$ is a hydrogen atom and
$R^5$ is a hydrogen atom.

8. The CXCR3 antagonist according to claim 2, wherein the compound represented by formula (II):

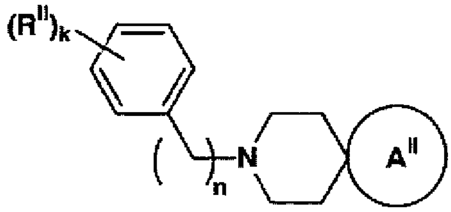

(II)

wherein all symbols have the same meanings as those described in the claim 2 is a compound represented by formula (II-B):

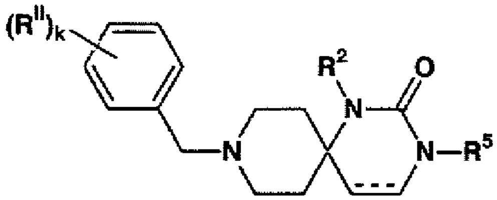

(II-B)

wherein

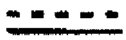

is a single bond or a double bond, the other symbols have the same meanings as those described in the claims 2 and 6.

9. The CXCR3 antagonist according to claim 8,
wherein $R^{II}$ is a chlorine atom, methyl, methoxy, allyloxy, propargyloxy or cyano;
$R^2$ is propyl, cyclopropylmethyl, cyclobutyl or cyclopentyl;
$R^5$ is benzyl which may have a substituent(s), phenylethyl which may have a substituent(s) or butyl.

10. The CXCR3 antagonist according to claim 2, wherein the compound represented by formula (II):

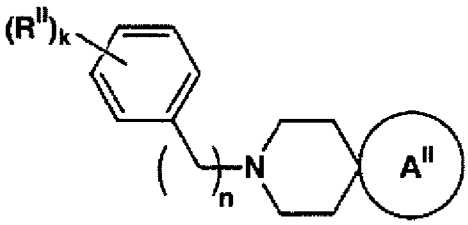

(II)

wherein all symbols have the same meanings as those described in the claim 2 is a compound represented by formula (II-C):

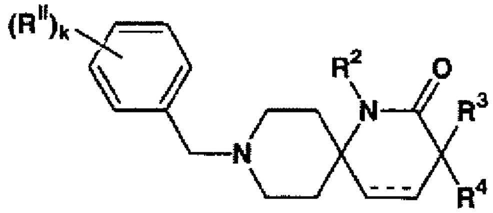

(II-C)

wherein all symbols have the same meanings as those described in the claims 2, 6 and 8, or a compound represented by formula (II-D):

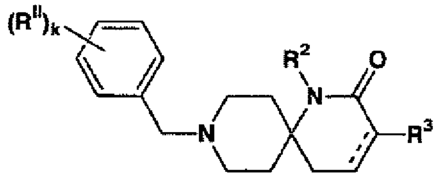

(II-D)

wherein all symbols have the same meanings as those described in the claims 2, 6 and 8.

**11.** The CXCR3 antagonist according to claim 10,
wherein $R^{II}$ is a chlorine atom, methyl, methoxy, allyloxy, propargyloxy or cyano;
$R^2$ is propyl, cyclopropylmethyl, cyclobutyl or cyclopentyl;
$R^3$ is benzyl which may have a substituent(s), pyridylmethyl which may have a substituent(s) or indolylmethyl which may have a substituent(s); and
$R^4$ is a hydrogen atom.

**12.** The CXCR3 antagonist according to claim 1, which is a preventive, therapeutic and/or progression-suppressing agent of CXCR3-related disease.

**13.** The CXCR3 antagonist according to claim 12, wherein the CXCR3-related disease is a rejection response to transplanted organ, tissue and/or cell, autoimmune disease, allergic disease, inflammatory bowel disease and/or respiratory disease.

**14.** The CXCR3 antagonist according to claim 13, wherein the autoimmune disease is systemic lupus erythematosus, rheumatoid arthritis, multiple sclerosis, type I diabetes and/or psoriasis, and the allergic disease is atopic dermatitis, and the respiratory disease is a chronic obstructive pulmonary disease.

**15.** A compound represented by formula (II-B):

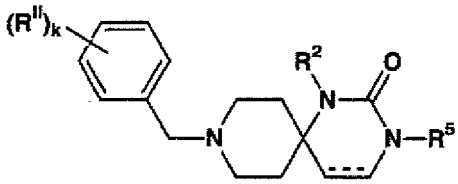

(II-B)

wherein all symbols have the same meanings as those described in the claims 2, 6 and 8,
a salt thereof, a quaternary ammonium salt thereof, an N-oxide form thereof, the solvate thereof, or a prod rug thereof.

**16.** The compound according to claim 15,
wherein $R^{II}$ is a chlorine atom, methyl, methoxy, allyloxy, propargyloxy or cyano;
$R^2$ is propyl, cyclopropylmethyl, cyclobutyl or cyclopentyl;
$R^5$ is benzyl which may have a substituent(s), phenylethyl which may have a substituent(s) or butyl;
k is an integer of 1 to 5.

**17.** A compound represented by formula (II-C):

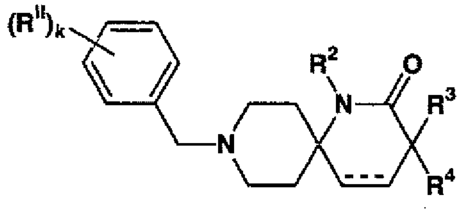

(II-C)

wherein all symbols have the same meanings as those described in the claims 2, 6 and 8 or a compound represented by formula (II-D):

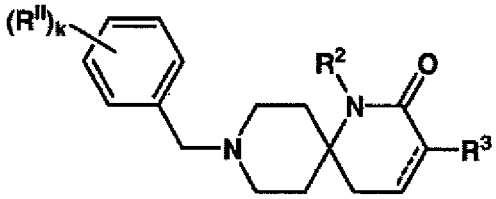

(II-D)

wherein all symbols have the same meanings as those described in the claims 2, 6 and 8,
a salt thereof, a quaternary ammonium salt thereof, an N-oxide form thereof, a solvate thereof, or a prodrug thereof.

**18.** The compound according to claim 17,
wherein $R^{II}$ is a chlorine atom, methyl, methoxy, allyloxy, propargyloxy or cyano;
$R^2$ is propyl, cyclopropylmethyl, cyclobutyl or cyclopentyl;
$R^3$ is benzyl which may have a substituent(s), pyridylmethyl which may have a substituent(s), or indolylmethyl which may have a substituent(s);
$R^4$ is a hydrogen atom; and
k is an integer of 1 to 5.

**19.** (3R)-3-benzyl-9-(4-chlorobenzyl)-1-propyl-1,4,9-triazaspiro[5.5]undecane-2,5-dione,
(3R)-3-benzyl-9-(4-chlorobenzyl)-1-(cyclopropylmethyl)-1 ,4,9-triazaspiro[5.5]undecane-2,5-dione,
(3R)-3-benzyl-9-(4-chlorobenzyl)-1-cyclabutyl-1,4,9-triazaspira[5.5]undecane-2,5-dione,
(3R)-3-benzyl-9-(4-chlorobenzyl)-1-cyclopentyl-1,4,9-triazaspiro[5.5]undecane-2,5-dione,
(3S)-3-benzyl-9-(4-chlorobenzyl)-1-cyclobutyl-1,4,9-triazaspiro[5.5]undecane-2,5-dione,
(3R)-9-(4-chlorobenzyl)-1-propyl-3-(3-pyridinylmethyl)-1,4,9-triazaspiro[5.5]undecane-2,5-dione,
(3R)-9-(4-chlorobenzyl)-1-(cyclopropylmethyl)-3-(3-pyridinylmethyl)-1,4,9-triazaspiro[5.5]undecane-2,5-dione,
(3R)-9-(4-chlorobenzyl)-1-cyclobutyl-3-(3-pyridinylmethyl)-1,4,9-triazaspiro[5.5]undecane-2,5-dione,
(3R)-9-(4-chlarobenzyl)-1-cyclopentyl-3-(3-pyridinylmethyl)-1,4,9-triazaspiro[5.5]undecane-2,5-dione,
(3R)-9-(4-chlorobenzyl)-3-(1H-indol-3-ylmethyl)-1-propyl-1,4,9-triazaspiro[5.5]undecane-2,5-dione,
(3R)-9-(4-chlorobenzyl)-1-(cyclopropylmethyl)-3-(1H-indol-3-ylmethyl)-1,4,9-triazaspiro[5.5]undecane-2,5-dione,
(3R)-9-(4-chlorobenzyl)-1-cyclobutyl-3-(1H-indol-3-ylmethyl)-1,4,9-triazaspiro[5.5]undecane-2,5-dione,
(3R)-9-(4-chlorobenzyl)-1-cyclopentyl-3-(1H-indol-3-ylmethyl)-1,4,9-triazaspiro[5.5]undecane-2, 5-dione,
3-benzyl-9-(biphenyl-4-ylmethyl)-1-propyl-1,4,9-triazaspiro[5.5]undecane-2,5-dione,
9-[4-(allyloxy)benzyl]-3-benzyl-1-propyl-1,4,9-triazaspiro[5.5]undecane-2,5-dione,
3-[4-(prop-2-yn-1-yloxy)benzyl]-3-azaspiro[5.5]undecane,
9-(4-chlorobenzyl)-3-(2-phenylethyl)-1-propyl-1,3,9-triazaspiro[5.5]undec-4-en-2-one,
3-benzyl-9-(4-chlorobenzyl)-1-(cyclopropylmethyl)-1,3,9-triazaspiro[5.5]undec-4-en-2-one,
3-benzyl-9-(4-chlorobenzyl)-1-propyl-1,3,9-triazaspiro[5.5]undec-4-en-2-one,
9-(4-chlorobenzyl)-1-(cyclopropylmethyl)-3-(2-phenylethyl)-1,3,9-triazaspiro[5.5]undec-4-en-2-one,
9-(4-chlorobenzyl)-1-cyclobutyl-3-(2-phenylethyl)-1,3,9-triazaspiro[5.5]undec-4-en-2-one,
3-butyl-9-(4-chlorobenzyl)-1-propyl-1,3,9-triazaspiro[5.5]undec-4-en-2-one,
3-butyl-9-(4-chlorobenzyl)-1-(cyclopropylmethyl)-1,3,9-triazaspiro[5.5]undec-4-en-2-one, 3-butyl-9-(4-chlorobenzyl)-1-cyclopentyl-1,3,9-triazaspiro[5.5]undecan-2-one,
9-(4-chlorobenzyl)-3-(2-phenylethyl)-1-propyl-1,3,9-triazaspiro[5.5]undecan-2-one,

9-(4-chlorobenzyl)-1-cyclobutyl-3-(2-phenylethyl)-1,3,9-triazaspiro[5.5]undecan-2-one,
9-(4-chlorobenzyl)-1-cyclopentyl-3-(2-phenylethyl)-1,3,9-triazaspiro[5.5]undecan-2-one,
3-butyl-9-(4-chlorobenzyl)-1-(cyclopropylmethyl)-1,3,9-triazaspiro[5.5]undecan-2-one,
3-butyl-9-(4-chlorobenzyl)-1-cyclobutyl-1,3,9-triazaspiro[5.5]undecan-2-one,
3-benzyl-9-(4-chlorobenzyl)-1-(cyclopropylmethyl)-1,9-diazaspiro[5.5]undecan-2-one, or
3-benzyl-9-(4-chlorobenzyl)-1-propyl-1,9-diazaspiro[5.5]undecan-2-one,
a salt thereof, a quaternary ammonium salt thereof, an N-oxide form thereof, or a solvate thereof, or a prodrug thereof.

**20.** A pharmaceutical composition which comprises the compound represented by formula (II-B) described in claim 15, formula (II-C) described in claim 17, formula (II-D) described in claim 17, or the compound according to claim 19, a salt thereof, a quaternary ammonium salt thereof, an N-oxide form thereof, a solvate thereof, or a prodrug thereof.

**21.** A medicament comprising the compound represented by formula (I) described in claim 1, a salt thereof, a quaternary ammonium salt thereof, an N-oxide form thereof, a solvate thereof, or a prodrug thereof, and one or more agent(s) selected from a nonsteroidal antiinflammatory drug, a disease modifying anti-rheumatic drug, steroids, an immunosuppressant agent, an antiinflammatory enzyme preparations, a chondroprotective agents, a T-cell inhibitor, a TNF$\alpha$ inhibitor, a prostaglandin synthase inhibitor, an IL-1 inhibitor, an IL-6 inhibitor, an interferon gamma agonist, prostaglandins, a phosphodiesterase inhibitor, a metalloproteinase inhibitor, and a chemokine receptor antagonist in combination.

**22.** A method for antagonizing against CXCR3 in a mammal, which comprises administering to a mammal an effective amount of a compound represented by formula (I) described in claim 1, a salt thereof, a quaternary ammonium salt thereof, an N-oxide form thereof, a solvate thereof, or a prodrug thereof.

**23.** A method for preventing, treating or progression-suppressing for CXCR3-related disease in a mammal, which comprises administering to a mammal an effective amount of the compound represented by formula (I) described in claim 1, a salt thereof, a quaternary ammonium salt thereof, an N-oxide form thereof, a solvate thereof, or a prodrug thereof.

**24.** A use of the compound represented by formula (I) described in claim 1, a salt thereof, a quaternary ammonium salt thereof, an N-oxide form thereof, a solvate thereof, or a prodrug thereof, for the manufacture of a CXCR3 antagonist.

**INTERNATIONAL SEARCH REPORT**

International application No.
PCT/JP2006/310814

A. CLASSIFICATION OF SUBJECT MATTER
***A61K31/499***(2006.01), ***A61K31/527***(2006.01), ***A61P1/04***(2006.01), ***A61P1/16***(2006.01), ***A61P3/10***(2006.01), ***A61P9/10***(2006.01), ***A61P11/00***(2006.01), ***A61P11/06***(2006.01), ***A61P13/12***(2006.01), ***A61P17/00***(2006.01), ***A61P17/06***(2006.01),

According to International Patent Classification (IPC) or to both national classification and IPC

B. FIELDS SEARCHED

Minimum documentation searched (classification system followed by classification symbols)
A61K31/499, A61K31/527, A61P1/04, A61P1/16, A61P3/10, A61P9/10, A61P11/00, A61P11/06, A61P13/12, A61P17/00, A61P17/06, A61P19/02, A61P25/00, A61P27/02, A61P27/16, A61P29/00, A61P31/18, A61P35/00, A61P37/06, A61P37/08, A61P43/00,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 2005/035534 A1 (ONO PHARM CO., LTD., JP), 21 April, 2005 (21.04.05), Pages 2, 36, 48, 118 (Family: none) | 1,12-14,21, 24<br>2-11,15-20 |
| X<br><br>Y | US 5962462 A (MERCK & CO., INC.), 05 October, 1999 (05.10.99), Full text & WO 98/25605 A1 & AU 9858033 A | 1,12-14,21, 24<br>2-11,15-20 |
| X<br>Y | WO 2004/092169 A1 (ONO PHARM CO., LTD., JP), 28 October, 2004 (28.10.04), Pages 7, 74, 75, 120 to 126 & EP 1619193 A1 | 15,20,21<br>1-14,16-19, 24 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 June, 2006 (14.06.06) | 27 June, 2006 (27.06.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/310814

C (Continuation). DOCUMENTS CONSIDERED TO BE RELEVANT

| Category* | Citation of document, with indication, where appropriate, of the relevant passages | Relevant to claim No. |
|---|---|---|
| X | WO 2003/028732 A1 (KYOWA HAKKO KOGYO CO., LTD.), 10 April, 2003 (10.04.03), Pages 121, 122 & US 2004/110826 A1 & EP 1430898 A1 & KR 2004038899 A & AU 2002338040 A1 & JP 2003-532064 A | 15 |
| Y | GAO, P., The unique target specificity of a nonpeptide chemokine receptor antagonist: selective blockade of two Thl chemokine receptors CCR5 and CXCR3., J.Leukoc.Biol., (2003), Vol.73, No.2, pages 273 to 280 | 1-21,24 |
| Y | MAHAD, D.J., Longitudinal study of chemokine receptor expression on peripheral lymphocytes in multiple sclerosis: CXCR3 upregulation is associated with relapse. Mult.Scler., (2003), Vol.9, No.2, pages 189 to 198 | 1-21,24 |
| P,A | WO 2006/004925 A1 (AMGEN SF LLC, US), 12 January, 2006 (12.01.06), & US 2006/069106 A1 | 1-21,24 |

Form PCT/ISA/210 (continuation of second sheet) (April 2005)

**INTERNATIONAL SEARCH REPORT**

International application No.
PCT/JP2006/310814

Continuation of A. CLASSIFICATION OF SUBJECT MATTER
(International Patent Classification (IPC))

***A61P19/02***(2006.01), ***A61P25/00***(2006.01), ***A61P27/02***(2006.01), ***A61P27/16***(2006.01), ***A61P29/00***(2006.01), ***A61P31/18***(2006.01), ***A61P35/00***(2006.01), ***A61P37/06***(2006.01), ***A61P37/08***(2006.01), ***A61P43/00***(2006.01), ***C07D221/20***(2006.01), ***C07D471/10***(2006.01)

(According to International Patent Classification (IPC) or to both national classification and IPC)

Continuation of B. FIELDS SEARCHED
Minimum documentation searched (International Patent Classification (IPC))

C07D221/20, C07D471/10

Minimum documentation searched (classification system followed by classification symbols)

Form PCT/ISA/210 (extra sheet) (April 2005)

**INTERNATIONAL SEARCH REPORT**

International application No.
PCT/JP2006/310814

**Box No. II Observations where certain claims were found unsearchable (Continuation of item 2 of first sheet)**

This international search report has not been established in respect of certain claims under Article 17(2)(a) for the following reasons:

1. [x] Claims Nos.: 22, 23
because they relate to subject matter not required to be searched by this Authority, namely:
Claims 22 and 23 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

**INTERNATIONAL SEARCH REPORT**

International application No.
PCT/JP2006/310814

(Concerning the subject to be searched)

The general formula (I) described in claim 1 includes extremely wide variety of compounds, however, what is disclosed in the description within the meaning of PCT Article 5 is only a small portion of the general formula (I), therefore, it is recognized that the support by the disclosure of the description within the meaning of PCT Article 6 is lacking.

Further, in the claims, there is a description of such as “may be substituted”, “prodrug” or the like, which makes what compounds with what structure are included unclear, therefore, it is practically impossible to perform presentation upon close inspection of all the documents describing these compounds.

Further, the invention according to claims 1-14, 20, 21 and 24 is an invention of a medicinal use or the like, however, what is recognized to be supported and disclosed in the description is only compounds described as compounds 5, 9, 11 and 15.

Accordingly, in this International Search, the search was made within the range of rational effort based on the compounds specifically described in the description.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0285862 A
- WO 0283143 A
- WO 0116114 A
- WO 0370242 A
- WO 200494381 A
- WO 20053127 A
- WO 9711940 A
- WO 9825605 A
- WO 0274770 A
- WO 200492169 A
- WO 200328 A
- WO 0140227 A
- WO 9907351 A
- WO 9940913 A
- WO 0046195 A
- WO 0046196 A
- WO 0046197 A
- WO 0046198 A
- WO 0046199 A
- WO 0069432 A
- WO 0069815 A
- DE 19837386
- WO 9955324 A
- WO 9955330 A
- WO 0004003 A
- WO 0027800 A
- WO 0027835 A
- WO 0027843 A
- WO 0029377 A
- WO 0031032 A
- WO 0031033 A
- WO 0034278 A
- WO 0035449 A
- WO 0035451 A
- WO 0035452 A
- WO 0035453 A
- WO 0035454 A
- WO 0035876 A
- WO 0035877 A
- WO 0041685 A
- WO 0051607 A
- WO 0051608 A
- WO 0051609 A
- WO 0051610 A
- WO 0053172 A
- WO 0053600 A
- WO 0058305 A
- WO 0059497 A
- WO 0059498 A
- WO 0059502 A
- WO 0059503 A
- WO 0062814 A
- WO 0073327 A
- WO 9917773 A
- WO 9932100 A
- WO 0006085 A
- WO 0006146 A
- WO 0010965 A
- WO 0006153 A
- WO 0021916 A
- WO 0037455 A
- EP 1013276 A
- WO 0038680 A
- WO 0039125 A
- WO 0040239 A
- WO 0042045 A
- WO 0053175 A
- WO 0042852 A
- WO 0066551 A
- WO 0066558 A
- WO 0066559 A
- WO 0066141 A
- WO 0068203 A
- JP 2000309598 B
- WO 0056729 A
- WO 0076933 A
- WO 9904794 A
- WO 9938514 A
- WO 02083143 A
- WO 02085862 A
- US 6469002 B
- WO 03101970 A
- WO 04094381 A
- WO 05003127 A
- WO 0066112 A
- WO 200424697 A


### Non-patent literature cited in the description


- Bunshisekkei. Iyakuhin no Kaihatsu. Hirokawa Shoten, 1990, vol. 7, 163-198
- The Merck Manual of Diagnosis and Therapy. Merck & Co

- Comprehensive Organic Transformations: A Guide to Functional Group Preparations. John Wiley & Sons Inc, 1999
- *Bioorg. Med. Chem. Lett.,* 2000, vol. 10, 1803
- *Bioorg. Med. Chem. Lett,* 2000, vol. 10, 1803
- *Bioorg. Med. Chem. Lett,* 2003, vol. 11, 2663